(19)

Europäisches Patentamt
European Patent Office
Office européen des brevets

(11)  **EP 2 976 045 B1**

(12)  **EUROPEAN PATENT SPECIFICATION**

(45) Date of publication and mention
of the grant of the patent:
**23.06.2021 Bulletin 2021/25**

(51) Int Cl.:
***A61F 2/34*** (2006.01)

(21) Application number: **14769055.6**

(22) Date of filing: **21.03.2014**

(86) International application number:
**PCT/US2014/031487**

(87) International publication number:
**WO 2014/153530 (25.09.2014 Gazette 2014/39)**

(54) **SYSTEMS, METHODS OF MAKING, AND DEVICES RELATED TO PATIENT-ADAPTED HIP JOINT IMPLANTS**

HERSTELLUNGSVERFAHREN UND VORRICHTUNGEN IN VERBINDUNG MIT PATIENTENADAPTIERTEN HÜFTGELENKIMPLANTATEN

SYSTÈMES, PROCÉDÉS DE FABRICATION ET DISPOSITIFS LIÉS À DES PROTHÈSES DE LA HANCHE ADAPTÉES À DES PATIENTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.03.2013 US 201361803803 P**

(43) Date of publication of application:
**27.01.2016 Bulletin 2016/04**

(73) Proprietor: **ConforMIS, Inc.
Billerica, MA 01821 (US)**

(72) Inventors:
• **KHALILI, Farid
Briarcliff Manor, New York 10510 (US)**

• **LANG, Philipp
Lexington, Massachusetts 02421 (US)**

(74) Representative: **Grund, Martin
Grund Intellectual Property Group
Patentanwalt und Solicitor PartG mbB
Postfach 44 05 16
80754 München (DE)**

(56) References cited:
**FR-A1- 2 847 458      FR-A1- 2 847 459
US-A1- 2004 199 258   US-A1- 2007 106 389
US-A1- 2008 140 215   US-A1- 2009 088 865
US-A1- 2009 326 670   US-A1- 2011 213 430
US-A1- 2011 295 378   US-A1- 2012 123 423**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**TECHNICAL FIELD**

This disclosure relates to patient-adapted (e.g., patient-specific and/or patient-engineered) hip joint implant, implant systems, as well as related surgical instrumentation.

**BACKGROUND**

Historically, a diseased or damaged joint, e.g., a joint exhibiting osteoarthritis, has been repaired using standard off-the-shelf implants and other surgical devices. Hip arthroplasty has become a routine procedure in surgically repairing a diseased or damaged hip joint. Total hip replacement (THR) procedures typically involve the implantation of two main components: a femoral component and an acetabular component. The femoral component is anchored within the existing femur, usually through a rigid stem secured within a canal in the natural femur bone tissue, and includes a head that replaces the natural hip joint femoral head. The acetabular component is secured within the acetabulum of the patient and serves as a bearing surface for the femoral component.

**BRIEF DESCRIPTION OF THE DRAWINGS**

FIG. 2 shows a resected hip femur (panel A) and a cross-sectional view of the cut bone surface (B). Areas generally corresponding to cancellous or trabecular bone 22, endosteal bone 23 and cortical bone 24 are indicated.
FIG. 3 shows a femoral implant with a short stem as implanted on a resected hip femur of a patient.
FIG. 4 shows a femoral implant with a long stem as implanted on a resected hip femur of a patient.
FIG. 9 shows a femoral implant as implanted on a resected hip femur of a patient.
FIG. 10 shows a portion of the femoral implant as implanted on a resected surface of a hip femur of a patient.
FIG. 11 shows a portion of the femoral implant with an outer sleeve as implanted on a resected surface of a hip femur of a patient (panel A) and an amplified view of a portion of the bone-contact surface of the outer sleeve (panel B). Panel B shows a step ladder design of the bone-contacting surface, which converts shear force to compressive force.
FIG. 12 shows a portion of a resected hip femur (panel A) and the portion of a resected hip femur after further burring or milling on or about the resection surface to facilitate engagement (or improve the fit) with a flanged outer sleeve. As shown in panel B, different portions of the outer sleeve are configured differently to match (or conform with) the shapes of the corresponding outer bone surface portions of the resected femoral neck.
FIG. 18 shows a native hip joint including the acetabulum engaged with the femoral head.
FIG. 19 shows the acetabulum 191 with the dashed line indicating a planned ream depth.
FIG. 20 shows a hip replacement system including an acetabular cup and a femoral head.
FIG. 21 shows a native hip joint being prepared for hip replacement or resurfacing.
FIG. 22 shows a hip implant as implanted in a patient's hip joint.
FIG. 23 shows a hip implant as implanted in a patient's hip joint.
FIG. 24 shows a hip implant as implanted in a patient's hip joint.
FIG. 25A shows an acetabulum of a hip joint.
FIG. 25B shows an acetabular implant component with an insert with a thickness AI and a metal backing with a thickness AML (acetabular metal liner). The diameter of the acetabular insert is shown as DAI.
FIG. 25C shows a femoral head and neck of a hip joint of a patient.
FIG. 25D shows a femoral head component for hip resurfacing.
FIG. 26 shows a flow chart illustrating the process of designing or selecting a patient-adapted implant or implant component according to certain embodiments herein.
FIG. 28 shows a flow chart illustrating a process of designing, selecting or adapting one or more patient-adapted hip implants or implant components according to certain embodiments herein.
FIG. 29 shows a flow chart illustrating a process of designing, selecting or adapting one or more patient-adapted hip implants or implant components according to certain embodiments herein.
FIG. 30 shows a flow chart illustrating a process of designing, selecting or adapting one or more patient-adapted hip implants or implant components according to certain embodiments herein.
FIG. 31 shows a flow chart illustrating a process of designing, selecting or adapting one or more patient-adapted hip implants or implant components according to certain embodiments herein.
FIG. 32A illustrates a hip joint, and FIG. 32B shows the hip joint where the femoral head has been separated the femoral neck. FIG. 32C illustrates a femoral neck cut guide that fits over the remaining femoral neck after being separated from the femoral head as shown in FIG. 32B. FIG. 32D shows the resected femoral neck.

FIG. 34 is a perspective view of an acetabular cavity and an embodiment of an acetabular cup component.

FIG. 35a and 35b are perspective views of an acetabular cup component having alignment markings and the same acetabular cup component aligned with and matching the rim of an acetabular cavity.

FIG. 36 is a perspective view of an acetabular cup component with an attachable rim component.

FIG. 37 is a perspective view of the acetabular components of FIG. 36, depicted in an attached configuration.

FIG. 38a is a side view of an embodiment of an acetabular cup component with an attachable rim component, in which the rim component includes male attachment features.

FIG. 38b is a front view of the acetabular cup component of FIG. 38a, in which female attachment features are shown.

FIG. 38c is a front view of the rim component of FIG. 38a, in which marking features are shown.

FIG. 39 is a perspective view of an exemplary acetabular jig embodiment relative to a patient's pelvis.

FIG. 40 is a perspective view of an additional exemplary acetabular jig embodiments relative to a patient's pelvis.

## DETAILED DESCRIPTION

When a surgeon uses a traditional off-the-shelf implant to replace a patient's joint, certain features of the implant typically do not match the particular patient's biological features. These mismatches can cause various complications during and after surgery. For example, surgeons may need to extend the surgery time and apply estimates and rules of thumb during surgery to address the mismatches. For the patient, complications associated with these mismatches can include pain, discomfort, soft tissue impingement, and an unnatural feeling of the joint during motion as well as an altered range of movement and an increased likelihood of implant failure. In order to fit a traditional implant component to a patient's articular bone, surgeons typically remove substantially more of the patient's bone than is necessary to merely clear diseased bone from the site. This removal of substantial portions of the patient's bone frequently diminishes the patient's bone stock to the point that only one subsequent revision implant is possible. Examples of multicomponent acetabular implants (FR 2 847 458 A1) and multicomponent patient-adapted acetabular implants (US 2011/295378 A1) and method of making such implants are known from the prior art. In view of the above, there is a need for an improved anatomically adapted implant and method of manufacture thereof which would be able to overcome or at least minimize some of the above-discussed prior art concerns.

The present invention describes a method of making a patient-adapted acetabular implant component for treatment of a hip joint of a patient, the hip joint including an acetabular cavity, the implant component comprising an acetabular cup component; the acetabular cup component comprises a cup portion having a substantially hemispherical shape and an attachment mechanism and a rim portion having a complementary attachment mechanism configured to attach the rim portion to the cup portion and said rim side matches at least a portion of the rim of the acetabular cavity; furthermore the method comprises choosing a cup portion, which is a standard, non patient-specific size and making at least one feature of the acetabular cup component patient-adapted, wherein the at least one patient-adapted feature is derived from image data of at least a portion of the patient's hip joint, wherein the at least one patient-adapted feature of the acetabular cup component is a feature selected from the group consisting of: a locking mechanism; one or more screw holes; a radius of the acetabular cup component; and combinations thereof.

Certain embodiments of the implants, surgical tools, and related methods (e.g. methods of designing, selecting or optimizing), and methods of using the implants (not claimed) and surgical tools (e.g. guide tools) described herein can be applied to any joint including hip joint. Furthermore, various embodiments described herein can apply to methods and procedures, and the design of methods and procedures, for preparing, resectioning or otherwise revising the patient's anatomy in order to implant the implant components described herein or to using the surgical tools described herein.

In certain embodiments, an implant or implant components or related methods described herein can include a combination of patient-specific and patient-engineered features. In certain embodiments, an implant or implant components or related methods described herein can include a combination of patient adapted (patent specific or patient-engineered) features with standard features (i.e., designed or selected without reference to an individual patient or the patient intended to receive the implant or implant components). An implant, or one or more components of the implant, may include a joint-facing surface, at least a portion of which provides an articular surface upon implantation. Similarly, such a joint-facing surface can include a combination of patient-specific and patient-engineered features, which may be obtained or derived from patient-specific data, such as image data of a patient's joint, e.g., the diseased or damaged joint to be surgically repaired. An implant or implant component may be made of a single material. Alternatively, an implant or implant component may be made from at least two different materials. For example, the joint-facing surface of an implant or implant component may be made from a material such as ceramic, whereas the body or the rest of the implant or implant component may be made from a different material such as metal. As detailed below, different types of materials can be employed to manufacture an implant or implant component as described here. Further, an implant or implant component described herein can be modular or include modular parts.

Further, patient-specific data collected preoperatively can be used to engineer one or more optimized surgical cuts to the patient's bone and to design or select a corresponding implant component having or more bone-facing surfaces or facets (i.e., 'bone cuts') that specifically match one or more of the patient's resected bone surfaces. The surgical cuts to the patient's bone can be optimized (i.e., patient-engineered) to enhance one or more parameters, such as: (1) deformity correction and limb alignment (2) maximizing preservation of bone, cartilage, or ligaments, or (3) restoration or optimization of joint kinematics or biomechanics. Based on the optimized surgical cuts and, optionally, on other desired features of the implant component, the implant component's bone-facing surface can be designed or selected to, at least in part, negatively-match the shape of the patient's resected bone surface.

Also provided are tools, such as for example surgical tools including guide tools. Such tools may also have one or more patient-adapted (e.g., patient-specific or patient-engineered) features. 3D guidance surgical tools, also referred to herein as a 3D guidance surgical templates, that may be used for surgical assistance can include, without limitation, using templates, jigs and/or molds, including 3D guidance molds. It is to be understood that the terms "template," "jig," "mold," "3D guidance mold," and "3D guidance template," shall be used interchangeably within the detailed description and any appended claims to describe the surgical tool unless the context indicates otherwise.

A surgical tool may include a template that has at least a portion (e.g., a contact surface) for engaging a portion of a joint (e.g., a surface associated with a joint, an articular and/or non-articular surface portion of a joint), and the portion (e.g., the contact surface) substantially conforms with (or is substantially a negative of) the portion (e.g., the surface) associated with a joint. The template may further include at least one guide (e.g., a guide aperture or cutting slot) for directing movement of a surgical instrument. The template may be a single component or may include two or more components or pieces. The two or more components or pieces can be linked reversibly or irreversibly when in use, e.g., in surgery, and such linkage can be an attachment mechanism or through cross-reference (e.g., a second component registers to or cross-references a portion of the joint prepared by a first component). In related embodiments, the surface associated with the joint may be an articular surface, a non-articular surface, a cartilage surface, a weight bearing surface, a non-weight surface or a bone surface. The contact surface may be made of different materials (e.g., a biocompatible material). The contact surface can sustain heat sterilization without deforming.

Further provided are methods of joint arthroplasty, in particular, methods of hip arthroplasty. The method may include obtaining patient-specific data, such as data from an image of a hip joint and optionally one or more images of other joints (ankle, knee, etc.) including data encompassing a surface, e.g., an articular surface or bone surface associated with the hip joint. Also included are data encompassing one or more acetabular or femoral dimensions (e.g., size, thickness, or curvature, including angles such as femoral neck angle), and desired leg length. The patient-specific data may also include the degree of anteversion or retroversion or rotation of a patient's hip joint and thus the degree of necessary correction. The patient-specific data may optionally include information on one or more abnormalities associated with the hip joint (e.g., osteophyte, protrusion acetabula).

Based on the patient-specific data, one or more surgical tools are created having at least one contact surface that substantially conforms with at least a portion of the surface associated with the hip joint. Other patient-adapted features can also be derived from the patient-specific data and built in the surgical tools, e.g., by including one or more guides in the surgical tools that have a predetermined position and orientation based on the patient-adapted features to define a predetermined path for directing movement of one or more surgical instruments.

Also based on the patient-specific data, an implant may be designed, selected, and/or engineered, which may include one or more patient-adapted features. Such an implant may include a single component or multiple components. An implant component may be designed or selected to include one or more patient-adapted features. Alternatively, an implant component may be selected from a library of premade implant components, and such selection may be based on the individual patient-specific data or follow a standard applicable to different patients.

The patient-specific data may also allow a surgeon to determine the surgical approach, such as an anterior, lateral, or posterior approach. The patient-specific data may also allow a surgeon to evaluate the degree of anteversion or retroversion or rotation of the patient's hip joint and determine the degree of necessary correction, which may be determined in conjunction with the selection of the surgical approach.

## HIP REPLACEMENT SYSTEMS GENERALLY

Depending on a patient's hip conditions, total or partial hip arthroplasty may be recommended. Typical considerations on designing or selecting a hip replacement system may include bone preservation, different or patient-specific anatomy, (e.g., leg length, neck angle, e.g., "offset" and "short neck" stems), material selection (e.g., to ensure patient safety; to improve implant stability, etc.). The following subsections briefly describe certain, non-limiting commercial examples of hip replacement systems. Various embodiments of the disclosure can be adapted and utilized to improve existing designs or systems, to develop new hip replacement systems that may or may not include one or more existing components.

## TOTAL HIP REPLACEMENT

Total hip arthroplasty is intended to provide increased patient mobility and reduce pain by replacing the damaged hip joint articulation in patients where there is evidence of sufficient sound bone to seat and support the components. Total hip replacement is typically indicated in the following conditions: a severely painful or disabled joint from osteoarthritis, traumatic arthritis, rheumatoid arthritis, or congenital hip dysplasia, avascular necrosis of the femoral head, acute traumatic fracture of the femoral head or neck, failed previous hip surgery including joint reconstruction, internal fixation, arthrodesis, hemiarthroplasty, surface replacement arthroplasty, or total hip replacement, certain cases of ankylosis.

A total hip placement system may include modular components. An example of a modular hip replacement system is the S-ROM® Modular Hip System.

The modular nature allows the S-ROM® Modular Hip System to provide solutions for a full range of surgical scenarios, from primary total hip arthroplasty to the most complex revision or other challenges. In this modular system, the stem's independent neck and sleeve allows for 360 degrees of version adjustment and enables a surgeon to place the proximal sleeve in the best possible bone stock without affecting the stem biomechanics.

The S-ROM® prosthesis is a proximally modular cementless stem that separates the critical functions of intramedullary fixation and extramedullary biomechanics. The porous-coated proximal sleeve can be oriented and rotated to accommodate the best remaining calcar bone to optimize fixation. The slotted stem achieves rotational stability in the distal femur through its splines and proximally provides independent adjustment of version, height and offset. A variety of base neck lengths, along with a broad range of femoral head diameters and lengths, provide additional versatility in fine-tuning soft tissue balance around the hip.

## HIP IMPLANTS AND IMPLANT COMPONENTS

An implant of the disclosure may include a single component or multiple components (i.e., two or more components). The term "implant component" as used herein can include: (i) one of two or more devices that work together in an implant or implant system, or (ii) a complete implant or implant system, for example, in embodiments in which an implant is a single, unitary device. The term "match" as used herein is envisioned to include one or both of a negative-match, as a convex surface fits a concave surface, and a positive-match, as one surface is identical to another surface.

Exemplary patient-adapted (i.e., patient-specific or patient-engineered) features of the implant components described herein are identified in Table 1. One or more of these implant component features can be selected or designed based on patient-specific data/parameters, such as information derived from electronic image data obtained from an image of a patient's joint and optionally other related anatomy.

**Table 1: Exemplary implant features that can be patient-adapted based on patient-specific information (or patient-specific data including one or more measurements or parameters)**

| Category | Exemplary feature |
|---|---|
| **Implant or implant or components generally** | One or more portions of, or all of, an external implant component curvature |
| | One or more portions of, or all of, an internal implant dimension |
| | One or more portions of, or all of, an internal or external implant angle |
| | Portions or all of one or more of the ML (medio-lateral), AP (anterior-posterior), SI (superior-inferior) dimension of the internal and external component and component features |
| | An locking mechanism (e.g., material, configuration) |
| | An locking mechanism dimension between a plastic or non-metallic insert and a metal backing component in one or more dimensions |
| | Component height |
| | Component profile |
| | Component 2D or 3D shape |
| | Component volume |

| Category | Exemplary feature |
|---|---|
| | Composite implant height |
| | Insert width |
| | Insert shape |
| | Insert length |
| | Insert height |
| | Insert profile |
| | Insert curvature |
| | Insert angle |
| | Distance between two curvatures or concavities |
| | Polyethylene or plastic width |
| | Polyethylene or plastic shape |
| | Polyethylene or plastic length |
| | Polyethylene or plastic height |
| | Polyethylene or plastic profile |
| | Polyethylene or plastic curvature |
| | Polyethylene or plastic angle |
| | Component stem width |
| | Component stem shape |
| | Component stem length |
| | Component stem height |
| | Component stem profile |
| | Component stem curvature |
| | Component stem position |
| | Component stem thickness |
| | Component stem angle |
| | Component peg width |
| | Component peg shape |
| | Component peg length |
| | Component peg height |
| | Component peg profile |
| | Component peg curvature |
| | Component peg position |
| | Component peg thickness |

| Category | Exemplary feature |
| --- | --- |
| | Component peg angle |
| | Slope of an implant surface |
| | Number of sections, facets, or cuts on an implant surface |
| Acetabular Cup | One or more acetabular dimensions, e.g., superior-inferior (SI) diameter; anterior-posterior (AP) diameter; medio-lateral (ML) diameter, one or more oblique diameters; acetabular depth; anatomical acetabular center point; biomechanical acetabular center point such as center of rotation; acetabular angle |
| | Acetabular cup position, e.g., anteversion, retroversion, rotation |
| | Composite acetabular dimensions (e.g., size, thickness, geometry/shape or angle) |
| | Acetabular reaming depth |
| | Acetabular material (e.g., coating, texture) |
| Femoral Component(s) | Femoral head, neck and diaphysis dimensions |
| | Femoral head or neck resection surface, region |
| | Femoral head or neck resection angle, region |
| | Femoral neck angle (cortical or endosteal) |
| | Femoral anteversion or retroversion |
| | Femoral neck diameter (cortical or endosteal) |
| | Femoral shaft medio-lateral dimensions (cortical or endosteal) |
| | Femoral shaft anterior-posterior dimensions (cortical or endosteal) |
| | Femoral shaft length |
| | Femoral shaft composite curvature or profile |
| | Femoral shaft bone-contacting surface configuration |
| | Femoral offset |
| | Femoral neck collar (and collar size and/or shape) |
| Features influenced by Surgical Instruments | Patient-adapted guide tools |
| | Reamer size and/or shape; one or more reamer dimensions |
| | Broach size and/or shape; one or more broach dimensions |
| | Impactor size and/or shape; one or more impactor dimensions |

| Category | Exemplary feature |
|---|---|
| **Features influenced by Surgical or Surgeon's Preferences** | Femoral neck resection level |
| | Femoral neck resection angle |
| | Mesialization (e.g., 1, 2, 3, 4, 5, 6, 7, 8, 9, 10, 10, 12 mm), e.g., mesialized reaming of the acetabulum |
| | Lateralization, e.g., lateralized femoral stem or lateral offset of the stem |
| | Acetabular cup positioning and orientation (e.g., angle of inclination, tilt) |
| | Acetabular cup size (diameter, thickness or offset, |
| | Standard or custom or patient-adapted implants or implant components (e.g., modular implants or non-modular; acetabular cup, insert, femoral head, femoral shaft) |
| | The surgeon's preference for an orthopedic implant type |
| | The surgeon's preference for particular parts of the implant (e.g., modularity, material) |
| | The degree of acceptable orthopedic implant sizes (e.g., a restriction on the range of implant sizes that may be recommended), |
| | The amount of bone that will be resected, |
| | The planned location and/or orientation of the orthopedic implant |
| | Fixation type (e.g., cement or cementless) |
| | Material type, finish, and other features such as head size and other preferences such as metal-on-metal, metal-on-ceramic, ceramic-on-ceramic, metal-on-poly, etc. |
| | The surgeon's standard preferences (i.e., preferences applicable to all patients, which can include one or more of the above parameters) |
| | The surgeon's patient-specific preferences (i.e., preferences that the surgeon for a particular patient based on patient-specific data or information, which can include one or more of the above parameters) |

Traditional implants and implant components can have surfaces and dimensions that are a poor match to a particular patient's biological feature(s). The patient-adapted implants, guide tools, and related methods described herein improve upon these deficiencies. The following two subsections describe two particular improvements, with respect to the bone-facing surface and the joint-facing surface of an implant component; however, the principles described herein are applicable to any aspect of an implant component.

Other patient-adaptable parameters or features are also included in Table 1, such as surgical instruments and surgeon's preferences. One or more of these patient-adaptable parameters or features can serve as constraint data to design, select, or adapt one or implant or implant components, one or more surgical guide tools or instruments, and the surgical plan for a patient. A system described herein can be configured to process these parameters or features in an iterative process, e.g., any two or more parameters listed below can serve as constraint data for each other to achieve a final, optimized output.

In some embodiments, the implant design can be chosen from a hierarchy of pre-designed orthopedic implants, e.g., in a virtual or physical library, in which each implant could reflect a general size group option, an anatomical size option, a biomechanical size option, or any combinations thereof. The anatomical and biomechanical size options potentially being, at least in some embodiments, sets of different and at least somewhat independent features of the orthopedic implant design. In some embodiments, at least one of the optimization algorithms utilizes a defined relationship between several orthopedic factors and orthopedic responses in order to determine optimal parameters for the orthopedic procedure to achieve desired orthopedic responses.

In some embodiments, optimization systems and methods may be used to optimize parameters of an orthopedic procedure other than or in addition to anatomic and biomechanical fit of an implant for the particular patient. For example, these systems and methods may be utilized to optimize other aspects of a patient's treatment such as selection of and optimization of additional treatments, such as custom orthotics or rehabilitation regimens.

In some examples, there may be provided a computer-implemented method of optimizing parameters relating to a joint procedure involving the implantation of at least one orthopedic implant into a joint of a particular patient, the method comprising: receiving in a computer processor information concerning the particular patient, including: (i) information relating at least in part to a model of the particular patient's joint, including image data of the patient's joint and optionally image data of the patient's related anatomy, e.g., a related joint (such as hip, knee and ankle joints being related); (ii) information relating at least in part to an axis associated with the particular patient's joint in relation to the model of the particular patient's joint; receiving in the computer processor information that defines at least one relationship relating a plurality of orthopedic responses to a plurality of orthopedic factors, wherein at least one of the orthopedic factors relates to at least one of a position and an orientation of the orthopedic implant relative to the joint, including preferences by the patient's surgeon (such preferences may include a preferred surgical approach, mesialization of the acetabular cup or not, either generally preferred by the surgeon for different patients or preferences specific to the patient based on the patient's own data or information); and (iv) at least one of the orthopedic factors relates to an articular surface shape geometry of the orthopedic implant; in the computer processor, using the received information concerning the particular patient, and using the received information that defines the at least one relationship, automatically or semi-automatically determining: (i) at least one of a suggested optimal position and a suggested optimal orientation for the orthopedic implant relative to the particular patient's joint; and (ii) a suggested optimal articular surface shape geometry for the orthopedic implant; outputting from the computer processor information concerning the at least one of the suggested optimal position and orientation for the orthopedic implant and information concerning the suggested optimal articular surface shape geometry for the orthopedic implant. Further determined information can include recommended and/or mandatory surgical procedural steps, e.g., cutting or drilling paths to facilitation implant placement (e.g., size, shape, geometry, position, and/or orientation) and/or configuration of one or more surgical guide tools.

## BONE-FACING SURFACE OF AN IMPLANT COMPONENT

In certain embodiments, the bone-facing surface of an implant can be designed to substantially negatively-match one or more bone surfaces. For example, in certain embodiments at least a portion of the bone-facing surface of a patient-adapted implant component can be designed to substantially negatively-match the shape of subchondral bone, cortical bone, endosteal bone, or bone marrow. A portion of the implant also can be designed for resurfacing, for example, by negatively-matching portions of a bone-facing surface of the implant component to the subchondral bone or cartilage. Accordingly, in certain embodiments, the bone-facing surface of an implant component can include one or more portions designed to engage resurfaced or resected bone, for example, by having a surface that negatively-matches uncut subchondral bone or cartilage, and one or more portions designed to engage cut bone, for example, by having a surface that negatively-matches a cut subchondral bone.

In certain embodiments, the bone-facing surface of an implant component includes multiple surfaces, e.g., planar or substantially planar surfaces also referred to herein as bone cuts. One or more of the bone cuts on the bone-facing surface of the implant component can be selected or designed to substantially negatively-match one or more surfaces of the patient's joint, including one or more of a resected surface, a resurfaced surface, and an unaltered surface, including one or more of bone, cartilage, and other biological surfaces. These bone-facing surface features can be applied to various joint implants, including knee, hip, spine, and shoulder joint implants.

In certain embodiments, corresponding sections of an implant component can include different thicknesses (e.g., distance between the component's bone-facing surface and joint-facing surface), surface features, bone cut features, section volumes, or other features. For example, corresponding lateral and medial or sections of a tibial implant component surface can include different thicknesses, section volumes, bone cut angles, and bone cut surface areas. One or more of the thicknesses, section volumes, bone cut angles, bone cut surface areas, bone cut curvatures, numbers of bone cuts, peg placements, peg angles, and other features may vary between two or more sections (e.g., corresponding sections on lateral and medial condyles) of an implant component. Alternatively or in addition, one, more, or all of these features can be the same in corresponding sections of an implant component. An implant design that allows for independent features on different sections of an implant allows various options for achieving one or more goals, including, for example, (1) deformity correction and limb alignment (2) preserving bone, cartilage, or ligaments, (3) preserving or optimizing other features of the patient's anatomy, such as leg length, (4) restoring or optimizing joint kinematics or biomechanics, such as correcting anteversion or retroversion, femoral or acetabular, or achieving a desired degree of rotation of the hip implant; or (5) restoring or optimizing joint-line location or joint gap width.

Alternatively or in addition, corresponding sections of an implant component can be designed to include the same features, for example, the same thickness or at least a threshold thickness. For example, when the corresponding implant sections are exposed to similar stress forces, similar minimum thicknesses can be used in response to those stresses. Alternatively or in addition, an implant design can include a rule, such that a quantifiable feature of one section is greater than, greater than or equal to, less than, or less than or equal to the same feature of another section of the implant component. For example, in certain embodiments, an implant design can include a lateral portion that is thicker than or equal in thickness to the corresponding medial portion. Similarly, in certain embodiments, an implant design can include a lateral height that is higher than or equal to the corresponding medial height.

In certain embodiments, one or more of an implant component's bone cut or bone cut facet features (e.g., thickness, section volume, cut angle, surface area, or other features) can be patient-adapted. For example, as described more fully below, patient-specific data, such as imaging data of a patient's joint, can be used to select or design an implant component (and, optionally, a corresponding surgical procedure or surgical tool) that matches a patient's anatomy or optimizes a parameter of that patient's anatomy. Alternatively or in addition, one or more aspects of an implant component, for example, one or more bone cuts, can be selected or designed to match predetermined resection cuts. "Predetermined" as used herein includes, for example, preoperatively determined (e.g., preoperatively selected or designed). For example, predetermined resection cuts can include resection cuts determined preoperatively, optionally in conjunction with a selection or design of one or more implant component features or one or more guide tool features. Similarly, a surgical guide tool can be selected or designed to guide the predetermined resection cuts.

## JOINT-FACING SURFACE OF AN IMPLANT COMPONENT

In various embodiments described herein, the outer, joint-facing surface of an implant component includes one or more patient-adapted (e.g., patient-specific or patient-engineered) features. For example, in certain embodiments, the joint-facing surface of an implant component can be designed to match the shape of the patient's biological structure or anatomy (i.e., to achieve a near anatomical fit). The joint-facing surface can include, for example, the bearing surface portion of the implant component that engages an opposing biological structure or implant component in the joint to facilitate typical movement of the joint. The patient's biological structure can include, for example, cartilage, bone, or one or more other biological structures. The patient's biological structure can also include one or more abnormalities associated with the joint to be repaired, such as for example, cartilage loss, osteophytes, flattening, eburnation, cyst formation, bone sclerosis, other arthritic or congenital deformity, and particular in a hip joint, protrusion acetabuli.

For example, in certain embodiments, the joint-facing surface of an implant component is designed to match the shape of the patient's articular cartilage, which may include the patient's existing articular surface shape and/or a calculated or corrected articular surface shape designed or engineered for the patient. For example, the joint-facing surface can substantially positively-match one or more features of the patient's existing cartilage surface or healthy cartilage surface or a calculated cartilage surface, on the articular surface that the component replaces. Alternatively, it can substantially negatively-match one or more features of the patient's existing cartilage surface or healthy cartilage surface or a calculated cartilage surface, on the opposing articular surface in the joint. As described below, corrections can be performed to the shape of diseased cartilage by designing surgical steps (and, optionally, patient-adapted surgical tools) to re-establish a normal or near normal cartilage shape that can then be incorporated into the shape of the joint-facing surface of the component. These corrections can be implemented and, optionally, tested in virtual two-dimensional and three-dimensional models. The corrections and testing can include kinematic analysis or surgical steps.

In certain embodiments, the joint-facing surface of an implant component can be designed to positively-match the shape of subchondral bone. For example, the joint-facing surface of an implant component can substantially positively-match one or more features of the patient's existing subchondral bone surface or healthy subchondral bone surface or a calculated subchondral bone surface, on the articular surface that the component attaches to on its bone-facing surface. Alternatively, it can substantially negatively-match one or more features of the patient's existing subchondral bone surface or healthy subchondral bone surface or a calculated subchondral bone surface, on the opposing articular surface in the joint. Corrections can be performed to the shape of subchondral bone to re-establish a normal or near normal articular shape that can be incorporated into the shape of the component's joint-facing surface. A standard thickness can be added to the joint-facing surface, for example, to reflect an average cartilage thickness. Alternatively, a variable thickness can be applied to the component. The variable thickness can be selected to reflect a patient's actual or healthy cartilage thickness, for example, as measured in the individual patient or selected from a standard reference database.

In certain embodiments, the joint-facing surface of an implant component can include one or more standard features. The standard shape of the joint-facing surface of the component can reflect, at least in part, the shape of typical healthy subchondral bone or cartilage. For example, the joint-facing surface of an implant component can include a curvature having standard radii or curvature of in one or more directions. Alternatively or in addition, an implant component can have a standard thickness or a standard minimum thickness in select areas. Standard thickness(es) can be added to one or more sections of the joint-facing surface of the component or, alternatively, a variable thickness can be applied to the implant component.

Certain embodiments can include, in addition to a first implant component, a second implant component having an opposing joint-facing surface. The second implant component's bone-facing surface or joint-facing surface can be designed as described above. Moreover, in certain embodiments, the joint-facing surface of the second component can be designed, at least in part, to match (e.g., substantially negatively-match) the joint-facing surface of the first component. Designing the joint-facing surface of the second component to complement the joint-facing surface of the first component can help reduce implant wear and optimize kinematics. Thus, in certain embodiments, the joint-facing surfaces of the first and second implant components can include features that do not match the patient's existing anatomy, but instead negatively-match or nearly negatively-match the joint-facing surface of the opposing implant component.

However, when a first implant component's joint-facing surface includes a feature adapted to a patient's biological feature, a second implant component having a feature designed to match that feature of the first implant component also is adapted to the patient's same biological feature. By way of illustration, when a joint-facing surface of a first component is adapted to a portion of the patient's cartilage shape, the opposing joint-facing surface of the second component designed to match that feature of the first implant component also is adapted to the patient's cartilage shape. When the joint-facing surface of the first component is adapted to a portion of a patient's subchondral bone shape, the opposing joint-facing surface of the second component designed to match that feature of the first implant component also is adapted to the patient's subchondral bone shape. When the joint-facing surface of the first component is adapted to a portion of a patient's cortical bone, the joint-facing surface of the second component designed to match that feature of the first implant component also is adapted to the patient's cortical bone shape. When the joint-facing surface of the first component is adapted to a portion of a patient's endosteal bone shape, the opposing joint-facing surface of the second component designed to match that feature of the first implant component also is adapted to the patient's endosteal bone shape. When the joint-facing surface of the first component is adapted to a portion of a patient's bone marrow shape, the opposing joint-facing surface of the second component designed to match that feature of the first implant component also is adapted to the patient's bone marrow shape.

The opposing joint-facing surface of a second component can substantially negatively-match the joint-facing surface of the first component in one plane or dimension, in two planes or dimensions, in three planes or dimensions, or in several planes or dimensions. For example, the opposing joint-facing surface of the second component can substantially negatively-match the joint-facing surface of the first component in the coronal plane only, in the sagittal plane only, or in both the coronal and sagittal planes.

In creating a substantially negatively-matching contour on an opposing joint-facing surface of a second component, geometric considerations can improve wear between the first and second components. Similarly, the radii of a convex curvature on the opposing joint-facing surface of the second component can be selected to match or to be slightly smaller in one or more dimensions than the radii of a concave curvature on the joint-facing surface of the first component. In this way, contact surface area can be maximized between articulating convex and concave curvatures on the respective surfaces of first and second implant components.

The bone-facing surface of the second component can be designed to negatively-match, at least in part, the shape of articular cartilage, subchondral bone, cortical bone, endosteal bone or bone marrow (e.g., surface contour, angle, or perimeter shape of a resected or native biological structure). It can have any of the features described above for the bone-facing surface of the first component, such as having one or more patient-adapted bone cuts to match one or more predetermined resection cuts.

Many combinations of first component and second component bone-facing surfaces and joint-facing surfaces are possible. Table 2 provides illustrative combinations that may be employed.

Table 2: Exemplary combinations of patient-specific (P), patient-engineered (PE), and standard (St) features[1] in an implant

| | Implant feature number[2] | | | | | | | | | | | | |
|---|---|---|---|---|---|---|---|---|---|---|---|---|---|
| Implant system number | 1 | 2 | 3 | 4 | 5 | 6 | 7 | 8 | 9 | 10 | 11 | 12 | 13 |
| 1 | P | P | P | P | P | P | P | P | P | P | P | P | P |
| 2 | PE | PE | PE | PE | PE | PE | PE | PE | PE | PE | PE | PE | PE |
| 3 | St | St | St | St | St | St | St | St | St | St | St | St | St |
| 4 | P | St | St | St | St | St | St | St | St | St | St | St | St |
| 5 | P | P | St | St | St | St | St | St | St | St | St | St | St |
| 6 | P | P | P | St | St | St | St | St | St | St | St | St | St |

| Implant system number | Implant feature number[2] | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | 13 |
| 7 | P | P | P | P | St | St | St | St | St | St | St | St | St |
| 8 | P | P | P | P | P | St | St | St | St | St | St | St | St |
| 9 | P | P | P | P | P | P | St | St | St | St | St | St | St |
| 10 | P | P | P | P | P | P | P | St | St | St | St | St | St |
| 11 | P | P | P | P | P | P | P | P | St | St | St | St | St |
| 12 | P | P | P | P | P | P | P | P | P | St | St | St | St |
| 13 | P | P | P | P | P | P | P | P | P | P | St | St | St |
| 14 | P | P | P | P | P | P | P | P | P | P | P | St | St |
| 15 | P | P | P | P | P | P | P | P | P | P | P | P | St |
| 16 | P | PE | PE | PE | PE | PE | PE | PE | PE | PE | PE | PE | PE |
| 17 | P | P | PE | PE | PE | PE | PE | PE | PE | PE | PE | PE | PE |
| 18 | P | P | P | PE | PE | PE | PE | PE | PE | PE | PE | PE | PE |
| 19 | P | P | P | P | PE | PE | PE | PE | PE | PE | PE | PE | PE |
| 20 | P | P | P | P | P | PE | PE | PE | PE | PE | PE | PE | PE |
| 21 | P | P | P | P | P | P | PE | PE | PE | PE | PE | PE | PE |
| 22 | P | P | P | P | P | P | P | PE | PE | PE | PE | PE | PE |
| 23 | P | P | P | P | P | P | P | P | PE | PE | PE | PE | PE |
| 24 | P | P | P | P | P | P | P | P | P | PE | PE | PE | PE |
| 25 | P | P | P | P | P | P | P | P | P | P | PE | PE | PE |
| 26 | P | P | P | P | P | P | P | P | P | P | P | PE | PE |
| 27 | P | P | P | P | P | P | P | P | P | P | P | P | PE |
| 28 | PE | St | St | St | St | St | St | St | St | St | St | St | St |
| 29 | PE | PE | St | St | St | St | St | St | St | St | St | St | St |
| 30 | PE | PE | PE | St | St | St | St | St | St | St | St | St | St |
| 31 | PE | PE | PE | PE | St | St | St | St | St | St | St | St | St |
| 32 | PE | PE | PE | PE | PE | St | St | St | St | St | St | St | St |
| 33 | PE | PE | PE | PE | PE | PE | St | St | St | St | St | St | St |
| 34 | PE | PE | PE | PE | PE | PE | PE | St | St | St | St | St | St |
| 35 | PE | PE | PE | PE | PE | PE | PE | PE | St | St | St | St | St |
| 36 | PE | PE | PE | PE | PE | PE | PE | PE | PE | St | St | St | St |

| Implant feature number[2] | | | | | | | | | | | | |
|---|---|---|---|---|---|---|---|---|---|---|---|---|
| Implant system number | 1 | 2 | 3 | 4 | 5 | 6 | 7 | 8 | 9 | 10 | 11 | 12 | 13 |
| 37 | PE | PE | PE | PE | PE | PE | PE | PE | PE | PE | St | St | St |
| 38 | PE | PE | PE | PE | PE | PE | PE | PE | PE | PE | PE | St | St |
| 39 | PE | PE | PE | PE | PE | PE | PE | PE | PE | PE | PE | PE | St |
| 40 | P | PE | St | St | St | St | St | St | St | St | St | St | St |
| 41 | P | PE | PE | St | St | St | St | St | St | St | St | St | St |
| 42 | P | PE | PE | PE | St | St | St | St | St | St | St | St | St |
| 43 | P | PE | PE | PE | PE | St | St | St | St | St | St | St | St |
| 44 | P | PE | PE | PE | PE | PE | St | St | St | St | St | St | St |
| 45 | P | PE | PE | PE | PE | PE | PE | St | St | St | St | St | St |
| 46 | P | PE | PE | PE | PE | PE | PE | PE | St | St | St | St | St |
| 47 | P | PE | PE | PE | PE | PE | PE | PE | PE | St | St | St | St |
| 48 | P | PE | PE | PE | PE | PE | PE | PE | PE | PE | St | St | St |
| 49 | P | PE | PE | PE | PE | PE | PE | PE | PE | PE | PE | St | St |
| 50 | P | PE | PE | PE | PE | PE | PE | PE | PE | PE | PE | PE | St |
| 51 | P | P | PE | St | St | St | St | St | St | St | St | St | St |
| 52 | P | P | PE | PE | St | St | St | St | St | St | St | St | St |
| 53 | P | P | PE | PE | PE | St | St | St | St | St | St | St | St |
| 54 | P | P | PE | PE | PE | PE | St | St | St | St | St | St | St |
| 55 | P | P | PE | PE | PE | PE | PE | St | St | St | St | St | St |
| 56 | P | P | PE | PE | PE | PE | PE | PE | St | St | St | St | St |
| 57 | P | P | PE | PE | PE | PE | PE | PE | PE | St | St | St | St |
| 58 | P | P | PE | PE | PE | PE | PE | PE | PE | PE | St | St | St |
| 59 | P | P | PE | PE | PE | PE | PE | PE | PE | PE | PE | St | St |
| 60 | P | P | PE | PE | PE | PE | PE | PE | PE | PE | PE | PE | St |
| 61 | P | P | P | PE | St | St | St | St | St | St | St | St | St |
| 62 | P | P | P | PE | PE | St | St | St | St | St | St | St | St |
| 63 | P | P | P | PE | PE | PE | St | St | St | St | St | St | St |
| 64 | P | P | P | PE | PE | PE | PE | St | St | St | St | St | St |
| 65 | P | P | P | PE | PE | PE | PE | PE | St | St | St | St | St |
| 66 | P | P | P | PE | PE | PE | PE | PE | PE | St | St | St | St |

| | Implant feature number[2] | | | | | | | | | | | | |
|---|---|---|---|---|---|---|---|---|---|---|---|---|---|
| Implant system number | 1 | 2 | 3 | 4 | 5 | 6 | 7 | 8 | 9 | 10 | 11 | 12 | 13 |
| 67 | P | P | P | PE | PE | PE | PE | PE | PE | PE | St | St | St |
| 68 | P | P | P | PE | PE | PE | PE | PE | PE | PE | PE | St | St |
| 69 | P | P | P | PE | PE | PE | PE | PE | PE | PE | PE | PE | St |
| 70 | P | P | P | P | PE | St | St | St | St | St | St | St | St |
| 71 | P | P | P | P | PE | PE | St | St | St | St | St | St | St |
| 72 | P | P | P | P | PE | PE | PE | St | St | St | St | St | St |
| 73 | P | P | P | P | PE | PE | PE | PE | St | St | St | St | St |
| 74 | P | P | P | P | PE | PE | PE | PE | PE | St | St | St | St |
| 75 | P | P | P | P | PE | PE | PE | PE | PE | PE | St | St | St |
| 76 | P | P | P | P | PE | PE | PE | PE | PE | PE | PE | St | St |
| 77 | P | P | P | P | PE | PE | PE | PE | PE | PE | PE | PE | St |
| 78 | P | P | P | P | P | PE | St | St | St | St | St | St | St |
| 79 | P | P | P | P | P | PE | PE | St | St | St | St | St | St |
| 80 | P | P | P | P | P | PE | PE | PE | St | St | St | St | St |
| 81 | P | P | P | P | P | PE | PE | PE | PE | St | St | St | St |
| 82 | P | P | P | P | P | PE | PE | PE | PE | PE | St | St | St |
| 83 | P | P | P | P | P | PE | PE | PE | PE | PE | PE | St | St |
| 84 | P | P | P | P | P | PE | PE | PE | PE | PE | PE | PE | St |
| 85 | P | P | P | P | P | P | PE | St | St | St | St | St | St |
| 86 | P | P | P | P | P | P | PE | PE | St | St | St | St | St |
| 87 | P | P | P | P | P | P | PE | PE | PE | St | St | St | St |
| 88 | P | P | P | P | P | P | PE | PE | PE | PE | St | St | St |
| 89 | P | P | P | P | P | P | PE | PE | PE | PE | PE | St | St |
| 90 | P | P | P | P | P | P | PE | PE | PE | PE | PE | PE | St |
| 91 | P | P | P | P | P | P | P | PE | St | St | St | St | St |
| 92 | P | P | P | P | P | P | P | PE | PE | St | St | St | St |
| 93 | P | P | P | P | P | P | P | PE | PE | PE | St | St | St |
| 94 | P | P | P | P | P | P | P | PE | PE | PE | PE | St | St |
| 95 | P | P | P | P | P | P | P | PE | PE | PE | PE | PE | St |
| 96 | P | P | P | P | P | P | P | P | PE | St | St | St | St |

| Implant system number | Implant feature number[2] | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | 13 |
| 97 | P | P | P | P | P | P | P | P | PE | PE | St | St | St |
| 98 | P | P | P | P | P | P | P | P | PE | PE | PE | St | St |
| 99 | P | P | P | P | P | P | P | P | PE | PE | PE | PE | St |
| 100 | P | P | P | P | P | P | P | P | P | PE | St | St | St |
| 101 | P | P | P | P | P | P | P | P | P | PE | PE | St | St |
| 102 | P | P | P | P | P | P | P | P | P | PE | PE | PE | St |
| 103 | P | P | P | P | P | P | P | P | P | P | PE | St | St |
| 104 | P | P | P | P | P | P | P | P | P | P | PE | PE | St |
| 105 | P | P | P | P | P | P | P | P | P | P | P | PE | St |

1: S = standard, off-the-shelf, P = patient-specific, PE = patient-engineered (e.g., constant coronal curvature, derived from the patient's coronal curvatures along articular surface)

2: Each of the thirteen numbered implant features represents a different exemplary implant feature, for example, for a hip implant the thirteen features can include, but are not limited to: (1) acetabular component's bone facing surface including the rim, (2) acetabular component's joint-facing surface including the rim, (3) interlock cup or insert, (4) femoral component's bearing or joint-facing surface, (5) femoral component's head resection surface, (6) femoral component's neck resection surface, (7) femoral head resection angle, (8) femoral neck resection angle, (9) femoral neck angle, (10) femoral component length and resulting leg length, (11) femoral shaft medio-lateral dimension, (12) femoral shaft anterior-posterior dimension, (13) femoral shaft length.

## MULTIPLE-COMPONENT IMPLANT

As described here in, an implant may include one or more implant components. For example, a hip implant of the disclosure may include an acetabular component and a femoral component, which may further include a femoral head component and a femoral shaft component. The implant may further include an interlock cup.

A multiple-component implant may include at least two components, each of which includes one or more patient-adapted features. Alternatively, one or more components may be selected from a library of premade implant components, and such section can be based on the patient-specific data as described herein.

Accordingly, the implants and implant systems described herein include any number of patient-adapted implant components and any number of non-patient-adapted implant components.

A multiple-component implant may include two components, each with one or more features, standard or patient-adapted, that accommodate each other so as to achieve the desired result (e.g., near anatomical fit) upon implantation. For example, an implant designed or selected for repairing a patient's hip joint can include an acetabular component and a femoral component, one or both of these components may include one or more patient-adapted features designed and configured to correct acetabular anteversion or retroversion, or femoral anteversion or retroversion associated with the a patient's hip joint.

In certain embodiments, the degree of acetabular anteversion or retroversion designed or selected for the acetabular component can directly relate to and work in a synchronized manner with the degree of femoral anteversion or retroversion designed or selected for the femoral component. For example, if a surgeon determines that 10 degrees of acetabular anteversion is necessary for the patient, the femoral component in an implant designed or selected for this patient may include 10 degrees of femoral retroversion or a different degree of femoral retroversion. Alternatively, if a surgeon determines that 10 degrees of acetabular anteversion is necessary for the patient, the femoral component in an implant designed or selected for this patient may include 10 degrees of femoral anteversion or a different degree of femoral anteversion.

Accordingly, the femoral component anteversion or retroversion or the acetabular component anteversion or retroversion can be adapted to or adjusted for the surgical approach, e.g. an anterior approach, lateral approach, or posterior approach. This can be included in the design of a patient-specific instrument (e.g. acetabular reaming jig or femoral neck cutting jig or femoral reaming jig). It can also be included in the selection of pre-manufactured or premade femoral or acetabular implant components with a desirable anteversion or retroversion. It can also be included in the design of the femoral component(s) or acetabular component.

In certain embodiments, the angle of the acetabular cup designed or selected for the acetabular component can directly relate to and work in a synchronized manner with the femoral neck angle designed or selected for the femoral component. For example, if a surgeon determines that 20 degrees of acetabular cup angle is necessary for the patient, the femoral component in an implant designed or selected for this patient may include a femoral neck angle of 70 degrees. Alternatively, if a surgeon determines that 25 degrees of acetabular anteversion is necessary for the patient, the femoral component in an implant designed or selected for this patient may include a femoral neck angle of 75 degrees.

The acetabular cup angle can be derived from or determined based on the patient-specific data. For example, the acetabular cup angle can be patient-matched or adapted to the patient's anatomy, but can be a result of compromising, for example, between a desirable acetabular angle for a particular implant design and the patient's native acetabular angle.

Similarly, the femoral neck angle can be derived from or determined based on the patient-specific data. For example, the femoral neck angle can be patient-matched or adapted to the patient's anatomy, but can be a result of compromising, for example, between a desirable femoral neck angle for a particular implant design and the patient's native femoral neck angle.

In certain embodiments, the acetabular cup angle and femoral neck angle can be adjusted relative to each other for and based on a particular implant design, and further based on the patient-specific data.

Accordingly, the femoral component neck angle or the acetabular component acetabular angle can be adapted to or adjusted for the surgical approach, e.g. an anterior approach, lateral approach, or posterior approach. This can be included in the design of a patient-specific instrument (e.g. acetabular reaming jig or femoral neck cutting jig or femoral reaming jig). It can also be included in the selection of pre-manufactured femoral or acetabular implant components with a desirable femoral neck or acetabular cup angle. It can also be included in the design of the femoral component(s) or acetabular component.

Similarly, in certain embodiments, the degree of acetabular cup rotation and the degree of femoral component rotation can be adjusted relative to each other for and based on a particular implant design, and further based on the patient-specific data.

Similarly, in certain embodiments, the orientation of the acetabular component and the orientation of the femoral component can be adjusted relative to each other for and based on a particular implant design, and further based on the patient-specific data.

## EXEMPLARY HIP IMPLANT SYSTEMS AND COMPONENTS

In certain embodiments, a hip implant is provided. The implant can include a femoral component, which can have a femoral head component and a femoral neck component, and an acetabular component, which can have an acetabular cup component and an acetabular liner component. The femoral and/or acetabular components may include one or more patient-adapted features as described herein.

Various hip implant and implant component configurations are illustrated in the drawings.

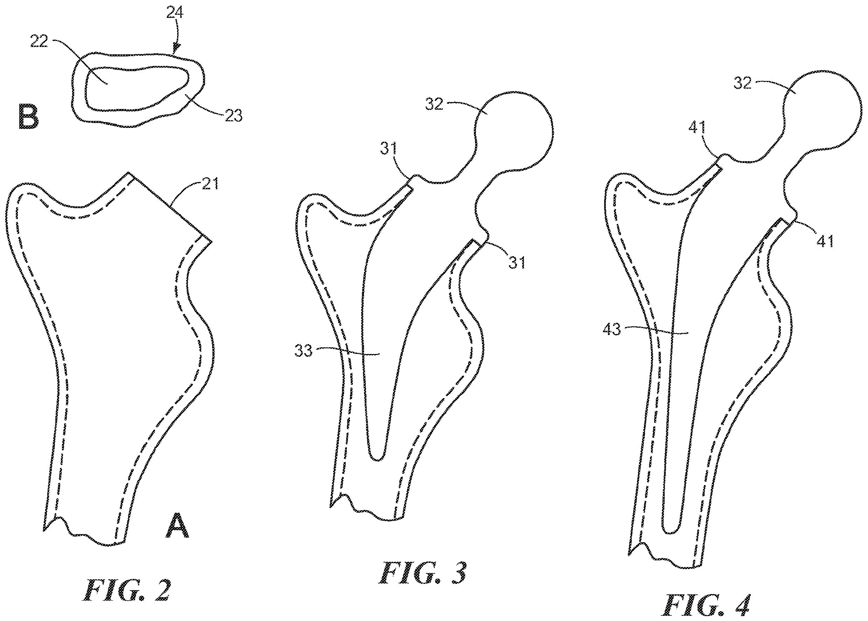

For example, the implant illustrated in FIG. 3 includes a collar 31 connecting the femoral head 32 and the femoral stem 33. The collar 31 is patient-specific or adapted to the individual patient with patient-specific parameters. For example, the collar 31, or at least a portion thereof, is configured to match the cortical bone of the cut bone surface of the femur (e.g., a peripheral edge of the collar 31 matches with a peripheral edge of the cortical bone). In certain embodiments, additionally or alternatively, the collar 31, or at least a portion thereof, is configured to match the endosteal bone of the cut bone surface of the femur ((e.g., a peripheral edge of the collar 31 matches with a peripheral edge of the endosteal bone).

An alternative femoral implant with a long stem is shown in FIG. 4. The implant includes a collar 41 connecting the femoral head 42 and the femoral stem 43. The collar 41 is patient-specific or adapted to the individual patient with patient-specific parameters. For example, the collar 41, or at least a portion thereof, is configured to match the cortical bone of the cut bone surface of the femur (e.g., a peripheral edge of the collar 41 matches with a peripheral edge of the cortical bone). In certain embodiments, additionally or alternatively, the collar 41, or at least a portion thereof, is configured to match the endosteal bone of the cut bone surface of the femur ((e.g., a peripheral edge of the collar 41 matches with a peripheral edge of the endosteal bone).

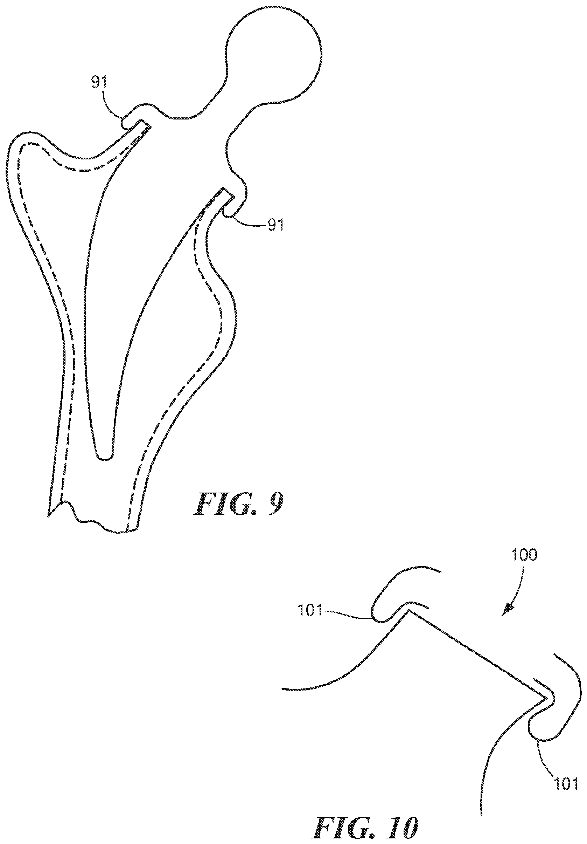

FIG. 10 shows a portion of the femoral implant as implanted on a resected surface of a hip femur of a patient. The portion includes an outer sleeve 100. As indicated, the outer sleeve 100 is configured to be rounded on its outer surface and periphery to avoid potential point loading. The outer sleeve 100 includes at least a portion 101 and a portion 102, configured (e.g., sized and shaped) differently in order to match the portion of the hip femur each engages.

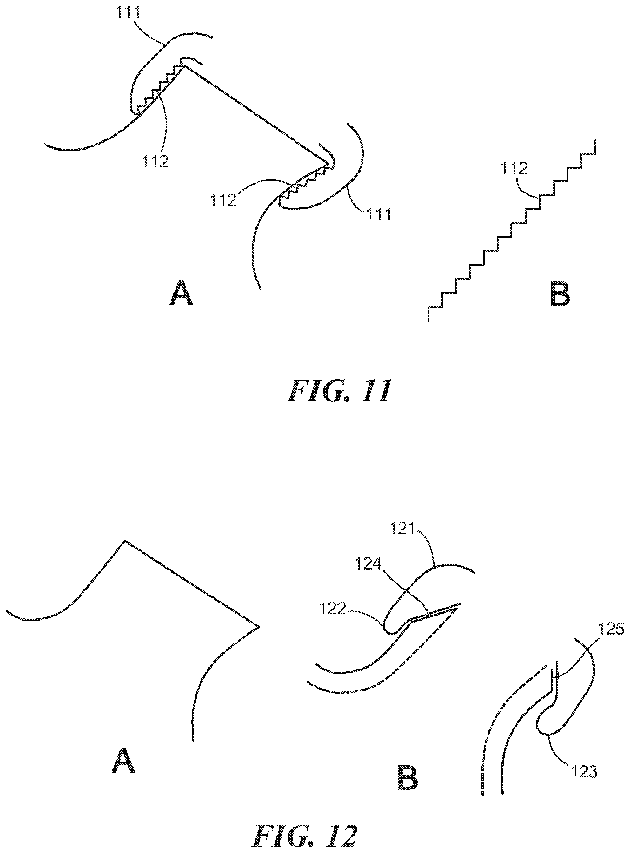

FIG. 11 shows a portion of the femoral implant with an outer sleeve 111 as implanted on a resected surface of a hip femur of a patient (panel A) and an amplified view of a portion of the bone-contact surface 112 of the outer sleeve (panel B). Panel B shows a step ladder design of the bone-contacting surface 112, which converts shear force to compressive force.

FIG. 12 shows a portion of a resected hip femur (panel A) and the portion of a resected hip femur after further burring or milling on or about the resection surface to facilitate engagement (or improve the fit) with a flanged outer sleeve 121. As shown in panel B, different portions (122, 123) of the outer sleeve 121 are configured differently to match (or conform with) the shapes of the corresponding outer bone surface portions of the resected femoral neck (124, 125). In certain embodiments, the bone surface portions are burred or milled. As indicated, such burring or milling is patient-specific, following the size, shape and/curvature of the patient's native bone, and can also be further adapted or optimized with patient-specific parameters.

In most hip replacements, the femoral neck is cut at an angle that is near perpendicular to the femoral neck axis. In one embodiment of the invention, the biomechanical axis is determined based on scan or other data. The biomechanical axis information is then entered into a surgical plan that is designed to cut the femoral neck perpendicular or near perpendicular to the biomechanical axis.

By cutting the femoral neck perpendicular or near perpendicular to the biomechanical axis, the contact area and support area for the collar portion of a short stem or long stem femoral component can be increased, thereby increasing bone support. In addition, loading can be favorably converted from shear type stresses to more compressive loading and stresses. If the cut is perpendicular to the biomechanical axis, compressive stress will predominate.

If intervening structures such as a high greater trochanter or a low femoral head (in case of a short neck) would interfere with a cut that is perpendicular to the mechanical axis, the cut can be optionally adjusted so that it remains near the biomechanical axis, but stays clear of these or other interfering structures.

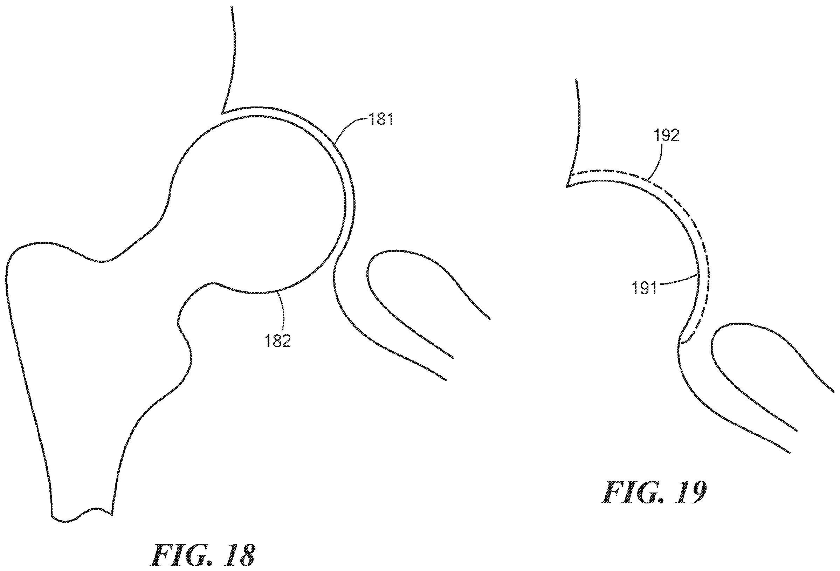

FIG. 18 shows a native hip joint including the acetabulum 181 engaged with the femoral head 182.

FIG. 19 shows the acetabulum 191 with the dashed line 192 indicating a planned ream depth, e.g., 2 mm.

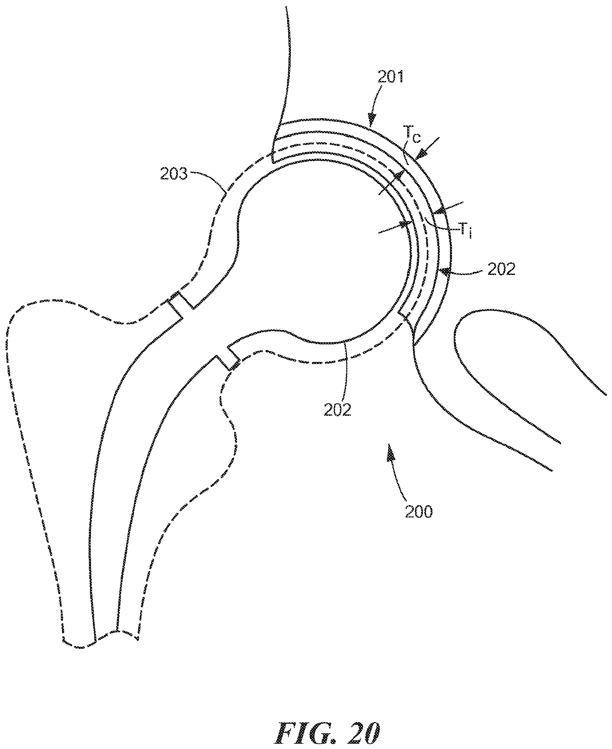

FIG. 20 shows a hip replacement system 200 including an acetabular cup 201 and a femoral head 202. In certain embodiments, the acetabular cup 201 includes a metal cup backing having a thickness (Tc) of 2 mm or 3 mm. The acetabular cup 201 further includes an insert, e.g., made of UHMWPE, having a thickness (Ti) of 3, 4 or 5 mm or another thickness as desired. The dashed line 203 indicates the position or contour of the native femoral head. As shown, the femoral head 202 is smaller than the native femoral head due to the acetabular offset created by the composite thickness of the acetabular cup (backing and insert) in view of the reaming depth.

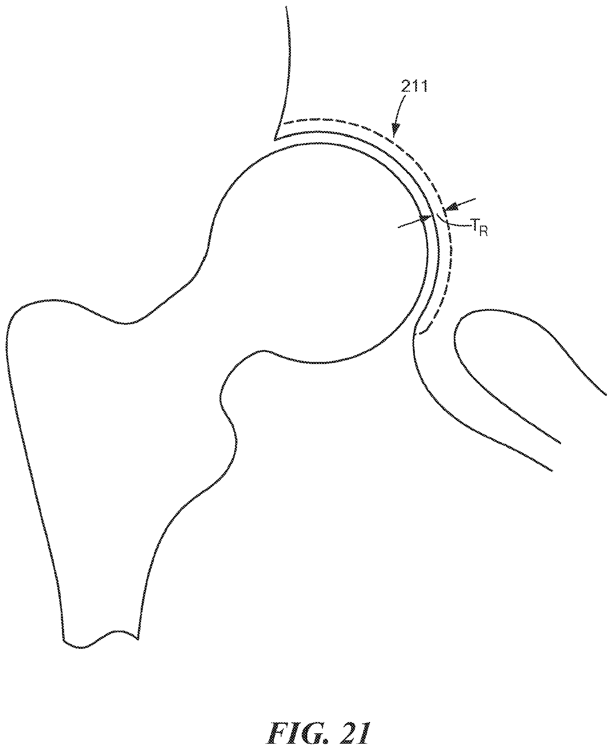

FIG. 21 shows a native hip joint being prepared for hip replacement or resurfacing. The dashed line 211 indicates an intended reaming depth ($T_R$) to accommodate an acetabular cup. As described herein, the intended reaming depth can be predetermined, determined with patient-specific parameters, or adapted to patient-specific parameters including but not limited to the femoral neck resection level, composite thickness of the implant (including acetabular and femoral components), femoral neck/shaft angle, acetabular cup position or orientation.

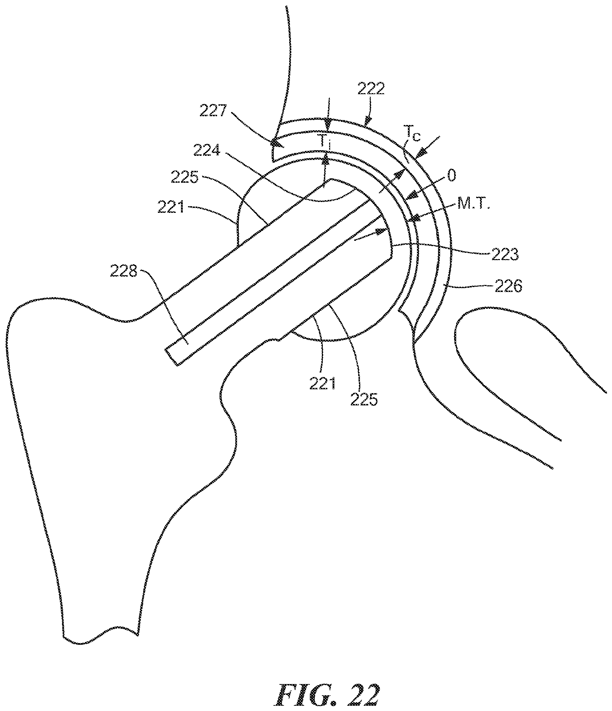

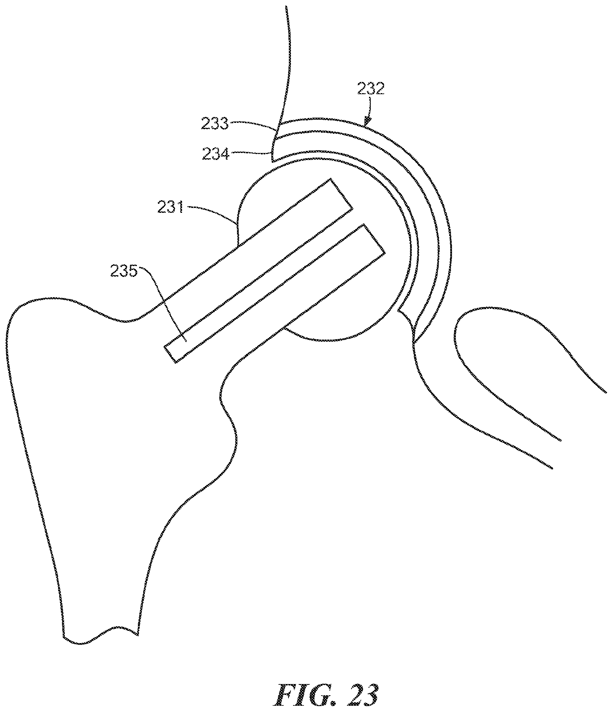

FIGS. 22 and 23 enclosed herein show illustrative hip implant systems having a single axis. The hip implant includes an acetabular cup having a generally hemisphere shape and a femoral head having a shape that fits within the acetabular cup and an anchoring means (e.g., a central peg) to help fix the femoral head onto the femur.

As shown in FIG. 22, the hip replacement implant includes a femoral component 221 and an acetabular component 222. The femoral head of the hip joint is prepared by milling or other means to receive the femoral component 221. The outer, joint-facing surface portion 223 of the femoral component 221 is configured to articulate with the joint-facing surface of the acetabular component 222. The bone-facing surface portions 224 and 225 may be flat or rounded to engage and negatively match (conform with) the prepared bone surface of the femoral head. As indicated, the articulating portion of the femoral component 221 may have a minimum material thickness (MT), which can be patient-adapted or optimized. The acetabular component 222 includes a backing (first acetabular) component 226 having a thickness Tc and engages (and conforms) with the acetabular bone surface. The acetabular component 222 also includes an insert (second acetabular) component 227 having a thickness Ti and provides the articulating surface against the femoral component 221. The insert or second acetabular component is locked, fixed or removably, onto the backing or first acetabular component, as detailed herein. The hip implant system further includes a central peg 228, the size (e.g., length, width) or shape of which can be patient-adapted or optimized. The hip implant system may also employ an alignment guide (e.g., k-wire, not shown) that helps position the femoral component 221, which may in turn determine the position or orientation of the acetabular component 222.

As shown in FIG. 23, the hip implant includes a femoral component 231 and an acetabular component 232. The acetabular component 232 includes a first, backing component 233 and a second, insert component 234. The femoral component includes a central peg 235. As described herein, various features or portions of these components can be adapted and optimized for an individual patient based on patient-specific parameters.

In certain embodiments, an acetabular cup includes a non-metal, e.g., cross-linked and oxidation resistant UHMWPE, bearing surface. The acetabular cup further includes a metal backing component for a non-metal component presenting the non-metal bearing surface. The bone-facing surface of the metal backing component negatively matches the shape of the reamed acetabulum. In an illustrative embodiment, the acetabulum is subjected to 1 mm reaming, the metal backing component is 2 mm thick, and the non-metal component is 4 mm. Accordingly, the acetabular cup requires 5 mm additional joint space. When the corresponding femoral head component, e.g., made of metal, includes another 3 mm material thickness, the femur must be resected to provide enough joint surface to accommodate the composite requirements of the acetabular cup and femoral head component (e.g., in the illustrative example, 8 mm of bone removal would be required). Material thicknesses of each component or composite thicknesses can be used to determine bone removal. Alternatively, a desired level of bone removal is determined first, with or without reference to the patient, and material thickness of each component can then be derived. Material thickness can be customized to each individual patient, e.g., based on patient-specific Finite Element Analysis (FEA). Material selection can also be customized to each individual patient, e.g., based on patient-specific bone structural or density parameters.

The following exemplary parameters of illustrative hip implant system can be optimized for or adapted to each individual patient: shape or geometry of the acetabular cup component (e.g., radius), driven by the patient's acetabulum; shape or geometry of the femoral head component (e.g., cylinder width and height), driven by the femoral resection level, patient's bone characteristics (e.g., trabecular microarchitecture, bone density), etc.; size or shape of the central peg (e.g., width, length or thickness) of the femoral head component.

As shown in FIG. 9, a hip implant system optionally includes a femoral sleeve configured to provide additional support on the femoral neck. The femoral implant includes an outer sleeve 91, at least a portion of which rests on the cut bone surface of the femur and another portion extends beyond the peripheral edge of the cut bone surface and engages at least a portion of the side surface adjacent to the cut bone surface. The outer sleeve 91 is configured to adapt to the patient based on patient-specific parameters.

Examples of various aspects of the femoral sleeve can be designed or selected based on the individual patient's anatomy or additional patient information include, but are not limited to, its material thickness, its widths or radii along the femoral neck, and its length. The femoral sleeve includes an outer surface for engaging the prepared femoral bone surface; the outer surface has a curvature that matches the curvature of the patient's prepared femoral bone surface and is configured to convert shear force to compression when the femoral head cylinder component engages the femoral sleeve. Material thickness can be optimized with patient-specific FEA.

The patient-specific design, selection or adaptation/optimization of the femoral collar can be based on one or more of the following parameters: the shape of the cut femur (e.g., matching the shape of the cut cortical bone), the shape of the greater trochanter, the shape of the lesser trochanter, endosteal bone of the femur, trabecular bone of the femur (including distance of the collar position adjacent to the trabecular bone), trabecular bone microarchitecture and macroarchitecture, and other bone quality parameters such as bone mineral density.

The illustrative resurfacing system optionally includes one or more patient-adapted surgical tools having one or more guides. One such surgical tool may include guide to accommodate a k-wire configured to extend into the femoral canal to achieve the desired alignment of the femoral head component.

## EXEMPLARY PATIENT-ADAPTED ACETABULAR COMPONENTS

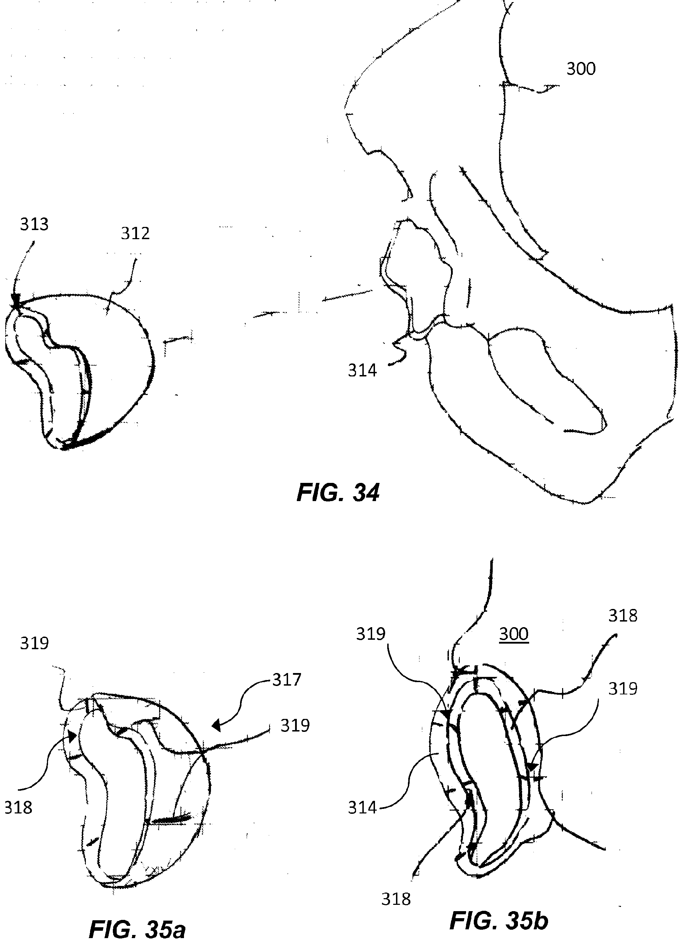

Various embodiments can include one or more patient-adapted acetabular implant components (e.g., acetabular cup, acetabular insert or liner, acetabular screws). For example, an acetabular cup can be selected and/or designed based on patient-specific information (e.g., CT imagining information), utilizing, for example, techniques discussed herein. In some embodiments, patient-specific information can be utilized to design a cup that maximizes coverage of the surface of the reamed acetabular cavity, which can facilitate fixation (e.g., initial fixation) of the cup, and can increase potential for bone on-growth and/or in-growth. For example, as illustrated in Fig. 34, in some embodiments, an acetabular cup 312 can be designed with a rim 313 that substantially matches at least a portion (and in some preferred embodiments, substantially all) of the rim 314 of the acetabular cavity of the pelvis 300. Additionally or alternatively, in some embodiments, the depth of the cup (e.g., the distance from the apex of the cup to the rim 313) can be slightly shorter than corresponding depths (e.g., around the perimeter of the rim) of the reamed bone to, for example, facilitate prevention of stem impingement.

Acetabular cup 312 can further include a locking mechanism in a predetermined, patient-adapted position and/or orientation for locking an insert in the cup. The predetermined position and/or orientation can be selected to allow the insert to face a direction that would maximize range of motion of a stem neck. For example, in some embodiments, a standard (i.e., non-patient-specific), zero degree insert may be utilized with a patient-adapted acetabular cup, and the patient-adapted position and/or orientation of the cup locking mechanism for the insert can allow for maximum range of motion of the stem neck and reduced potential for impingement, wear osteolysis and dislocation. Further, use of a standard insert can reduce cost of the implant system, as compared to a system utilizing a patient-specific insert.

Additionally, some embodiments can include one or more acetabular trial implant components. For example, a patient-adapted acetabular cup trial 317 can be utilized to facilitate positioning and placement of an acetabular cup. For example, Fig. 35a illustrates an exemplary cup trial 317, which includes a rim 318 that substantially matches at least a portion (and in some preferred embodiments, substantially all) of the rim 314 of the acetabular cavity of the pelvis 300. Optionally, the rim 318 of the cup trial can include markings 319 (e.g., laser markings, grooves, etchings) on the face of the rim 318 and/or outer surface of the cup trial 317. In some embodiments, the markings can have a predetermined position and/or orientation relative to at least a portion of the rim 314 of the acetabular cavity of the pelvis 300 when the cup trial 317 is properly positioned and oriented within the acetabular cavity such that the rim 318 is in substantially matching alignment with the rim 314 of the acetabular cavity. For example, the cup trial 317 may be placed within the prepared acetabulum and aligned to match the acetabular rim 314, as shown in Fig. 35b. Then, optionally, marks may be made by the surgeon on the acetabular rim 314 (and, optionally, nearby portions of bone) aligned with the markings 319 on the trial cup. The trial may then be removed. The acetabular cup 312 can have markings on its rim 313 and outer surface (e.g., porous coating) identical to at least some of the markings 319 on the trial 317. Upon implantation, the acetabular cup 312 may first be aligned with the markings on the rim of the acetabulum, and then fixed to the acetabulum. Using such a technique with alignment markings and a trial cup can help ameliorate problems associated with pelvis shift during surgery, which can reduce accuracy of placement of cups based on anatomic landmarks, resulting in increased potential for wear, implant loosening, and dislocation.

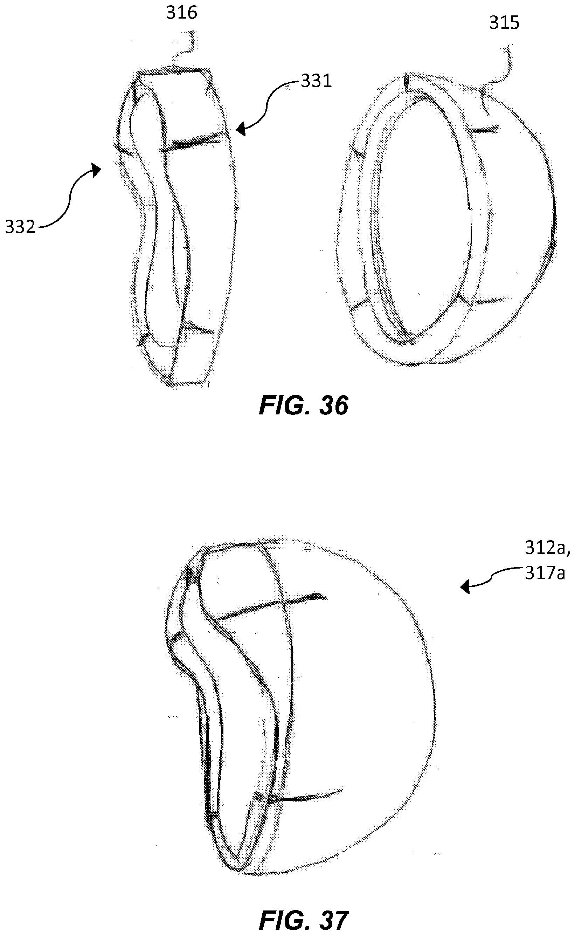

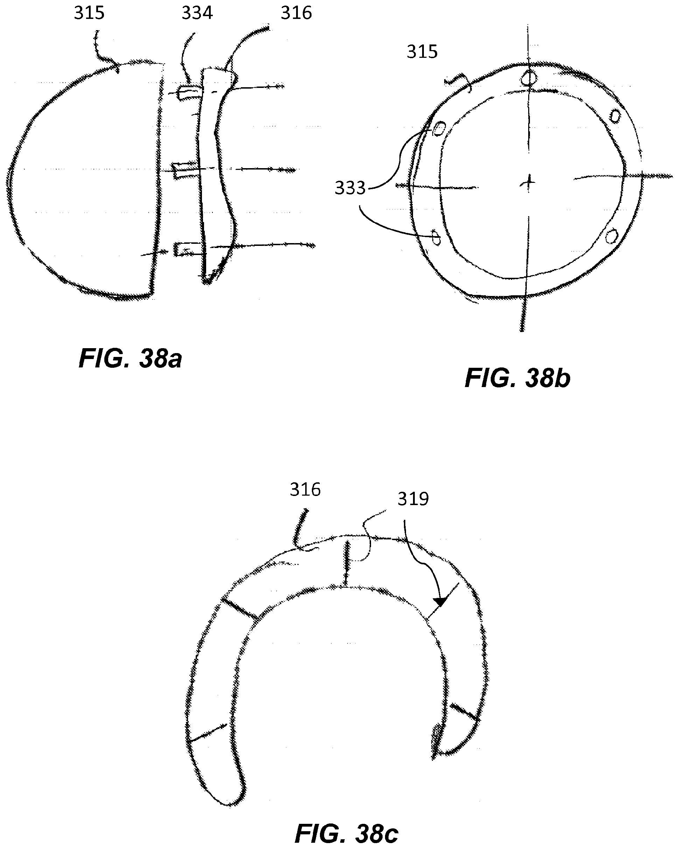

Further, in some embodiments, an acetabular cup 312a and/or cup trial 317a can be formed of multiple, releasably attachable portions, such as, for example, a cup portion 315 and a rim portion 316, as illustrated in Fig. 36. Cup portion 315 may have a substantially hemispherical shape, and in some embodiments, may be selected from one or more standard sizes. Rim portion 316 can have a cup side 331, which is configured to releasably attach to cup portion 315, and a rim side 332, which can be patient-specific and sized and shaped to match at least a portion (and in some preferred embodiments, substantially all) of the rim 314 of the acetabular cavity of the pelvis 300. A variety of attachment mechanism can be used to releasably attach cup portion 315 and rim portion 316. For example, as illustrated in Fig. 38a, the rim side of rim portion 316 can include a plurality of fixation pins 334. As depicted in the front view, illustrated in Fig. 38b, cup portion 315 can include a plurality of receiving apertures 333, configured to receive corresponding fixation pins 334. Further, in some embodiments, rim side 332 of rim portion 316 (illustrated in the front view depicted in Fig. 38c) can include markings 319, as discussed above. In some embodiments, in use, the cup portion 315 and rim portion 316 may be attached, as illustrated in Fig. 37, and the composite acetabular cup 312a and/or cup trial 317a may be placed within the prepared acetabulum and aligned to match the acetabular rim 314. Then, rim portion 316 may be optionally removed. Such embodiments, with a cup portion releasably attachable to a rim portion, can allow for accurate positioning of the cup using alignment markings (e.g., as discussed herein above) on outer surfaces of the rim for accurate placement of the cup, while also providing the surgeon the option to remove the rim portion, and use only a hemispherical cup portion.

In certain embodiments, the surgical planning systems and methods described herein can include utilizing patient-specific information (e.g., CT imagining information) for predetermining position, orientation, and/or length of acetabular cup screws. For example, in various embodiments, one or more screw holes may be positioned and/or oriented within an acetabular cup such that safe placement of screws passed through the screw holes when the acetabular cup is positioned and/or aligned at the implantation site is maximized. Safe placement of the screws can prevent inadvertent damage caused to vascular structures proximate to the implantation site by any drilling used for the placement of the screws and/or placement of the screws themselves. To maximize safe placement, one or more safe areas or zones may be identified as preferred and/or intended locations for screw placement. Such safe areas may include, for example, the area falling within lines from the anterior-inferior iliac spine through the center of the acetabulum and posterior by a line from the sciatic notch to the center of the acetabulum. Safe areas may also include, for example, portions of the pelvis. Accordingly, in various embodiments, the acetabular cup may be designed with one or more screw holes positioned and/or oriented for alignment within a safe area, and the length of drills and/or screws can be selected and/or designed to not penetrate beyond the safe area.

Various embodiments can also include one or more jigs (e.g., standard, patient-adapted, or combinations thereof) selected and/or designed for preparing tissue for implantation of an acetabular cup. One or more jigs may be selected and/or designed to guide drilling for acetabular screws based on one or more of patient-specific information; pre-determined screw placements (as discussed above); and a desired version, orientation, and/or inclination for the acetabular cup (e.g., cup liner and/or cup metal backing). Certain embodiments can include a jig having a patient-specific surface for placement on at least a portion of one or more of the acetabular rim, cortical bone, and the acetabular articular surface. The jig can further include one or more drill guide features, such that once the jig is properly positioned and/or aligned at the implantation site, one or more acetabular cup screw holes may be drilled (e.g., at a predetermined positioned, orientation, and/or depth, as described above). In some embodiments, such a jig may be configured for positioning before reaming of the acetabulum. Optionally, a patient-adapted drill stop may be selected and/or designed based on, for example, thickness of the acetabular bone and/or surrounding bone, in order to prevent drilling beyond the predetermined depth.

Additionally or alternatively, various embodiments can include a jig having a surface patient-specific based on at least a portion of the acetabular fossa. In some embodiments, such an acetabular fossa jig can include one or more surfaces configured to fit a reamed acetabular fossa. The acetabular fossa jig can further include one or more extension features, which extend outside/beyond the acetabular fossa and have patient-specific contact surfaces capable of referencing and/or aligning the jig relative to other patient-specific surfaces, such as, for example, portions of one or more of the acetabulum, ischium, sacrum, and pubis. Such patient specific contact surface(s) located outside the acetabular fossa can facilitated aligning the jig at a desired version, inclination, and/or orientation for the cup liner and/or the cup metal backing. Once the acetabular fossa jig (and optionally any extension features) is properly positioned and/or aligned at the implantation site, drill guides included in the acetabular fossa jig and/or one or more extension features may be utilized to drill one or more acetabular cup screw holes (e.g., at predetermined a predetermined positioned, orientation, and/or depth, as described above). In some embodiments, the acetabular fossa jig may also be configured to accommodate one or more spacers to which the jig can be linked, for example, in the event that the surgeon over-reamed a portion of the acetabulum. This can help facilitate screw hole alignment accuracy, even in the presence of over-reaming. Optionally, a patient-adapted drill stop may be selected and/or designed based on, for example, thickness of the acetabular bone and/or surrounding bone, in order to prevent drilling beyond the predetermined depth.

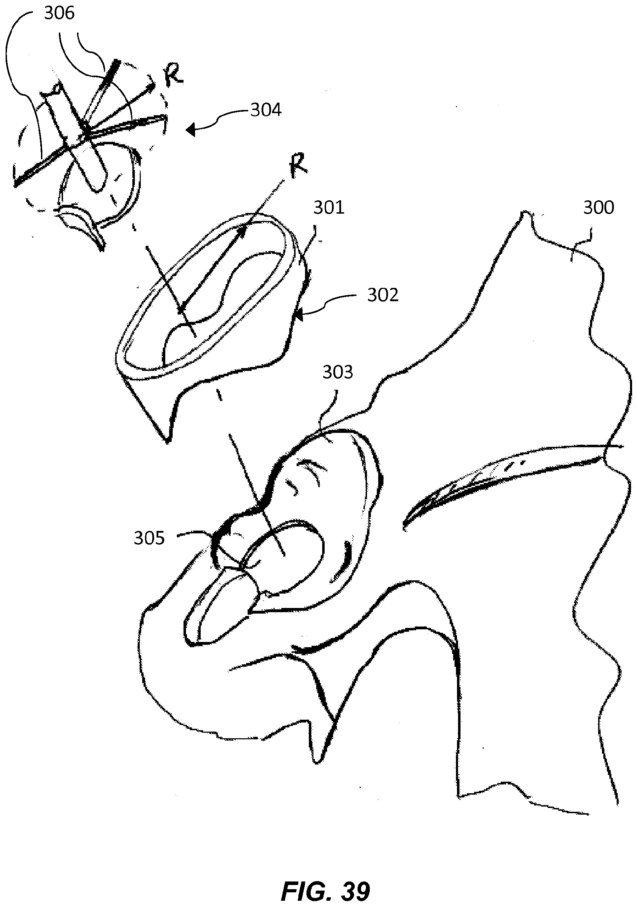

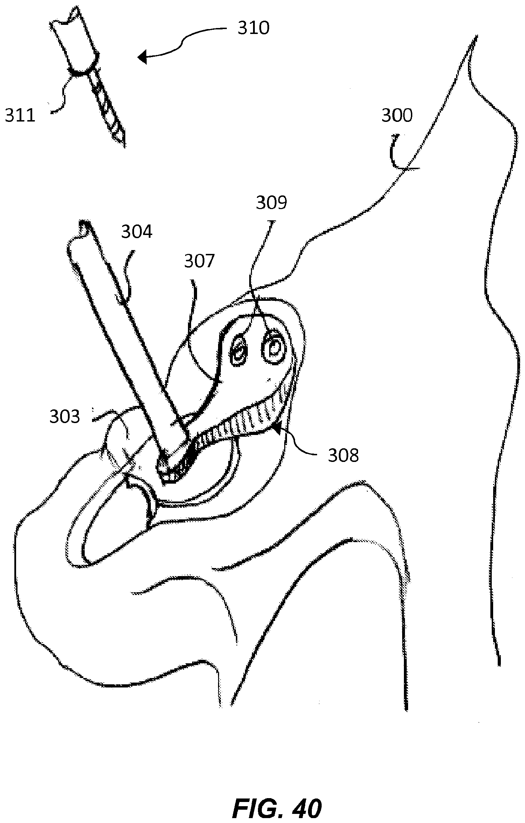

Figs. 39 and 40 illustrate perspective views of exemplary acetabular jig embodiments relative to a patient's pelvis 300. As shown, an acetabular rim jig 301 can include a patient-specific surface 302 sized and shaped to engage the rim of the patient's acetabulum 303. An acetabular fossa jig 304 can likewise have a perimeter surface sized and shaped for engaging the patient's acetabular fossa 305. The acetabular fossa jig 304 can further include one or more extension features 306. While the extensions features 306 extend beyond the acetabular fossa jig 304 perimeter, the extension features 306 may each have a length smaller than a relative distance R, where the relative distance R is the distance from about the center of the acetabular fossa to relative positions on the inner wall of acetabular rim jig 301, thus permitting acetabular fossa jig 304 to be positioned within acetabular rim jig 301. As shown in Fig. 40, a drill guide feature 307 can be attached to acetabular fossa jig 304 and extend beyond the acetabular fossa 305. Drill guide feature 307 can comprise an extension feature 306 or can be a separate structure. Drill guide feature 307 can include a patient-specific surface 308 for engagement with a portion of the acetabulum 303. One or more drill guide holes 309 can be formed in drill guide feature 307. Drill guide holes 309 can have pre-determined positions and orientations, as discussed above, and may be configured to receive a drill assembly 310. Furthermore, drill assembly 310 can include a drill stop 311, to limit the drill depth to a pre-determined safe distance.

## ADDITIONAL EXEMPLARY HIP IMPLANT SYSTEMS FEATURES AND METHODS OF MAKING THEM

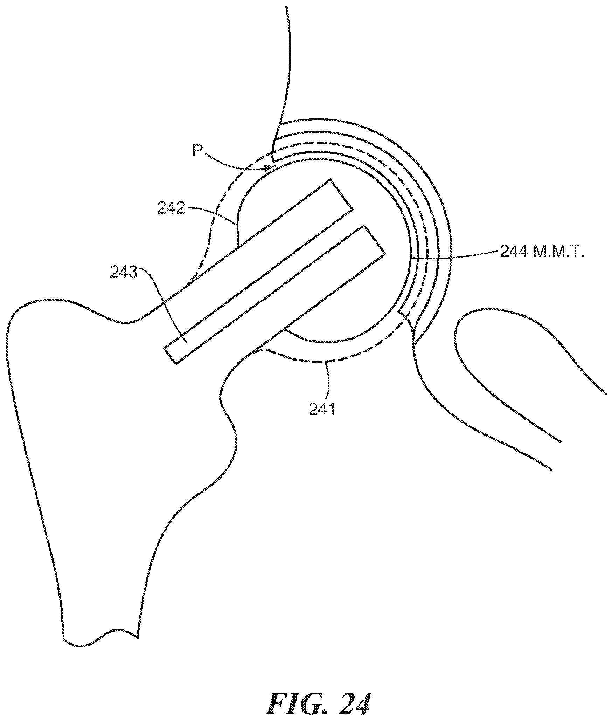

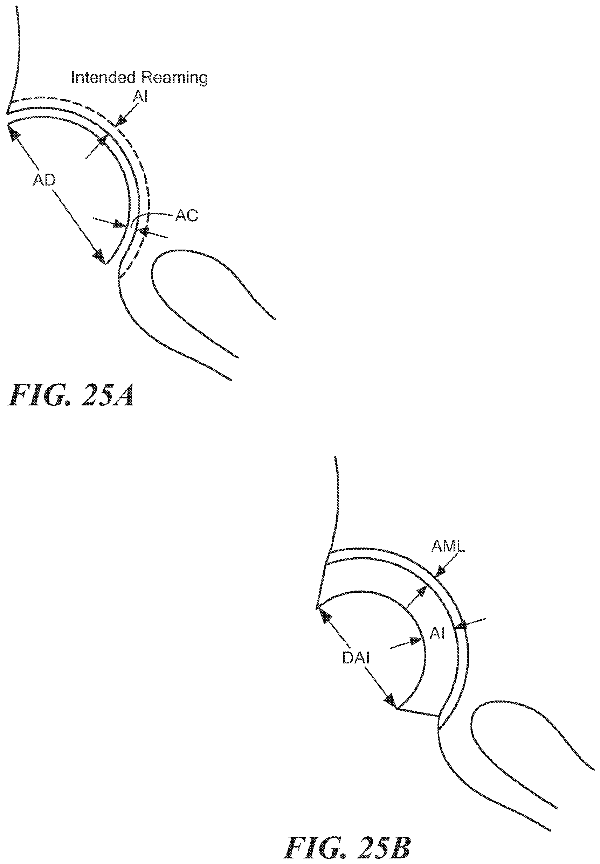

Referring to FIGS. 24, 25A-25D, the following calculations illustrate the determination or derivation of various parameters involved in the design or selection of some embodiments of hip implant systems of this disclosure.

As illustrated in FIG. 24, the dotted contour 241 indicates the native femoral head surface. The femoral component 242 includes a central peg 243. The femoral component 242 includes at least a portion 244 with minimum material thickness (MMT), which can be determined with patient-specific parameters, including the patient's body habitus. Desired play (P) between acetabular and femoral components is indicated.

As shown in FIG. 23, the width of the central peg (e.g., central peg 228, central peg 235, central peg 243 above) and the length of the central peg can be adapted to an individual patient. The width can be selected, adapted or designed based on the femoral neck width or the femoral head diameter, optimizing the amount of bone resection or removal vs. the minimum peg width required for biomechanical stability in a given patient. The width can be further adjusted based on the patient's anthropometric data and general bone size. The length of the central peg can be selected, adapted or designed based on femoral neck length and also femoral shaft width. Optionally, the central peg can extend into portions of the femoral shaft.

As shown in FIG. 25A, the acetabulum has a diameter AD. An intended reaming depth A1 is indicated, and the dotted contour (i.e., the intended or prepared acetabular rim) indicates the bone surface after reaming for engaging with the acetabular cup. The acetabular cartilage thickness, AC, is also indicated. As described herein, the intended reaming depth and resulting dimensions of the acetabular rim can be used to create a reamer that is adapted to the individual patient.

As shown in FIG. 25B, an acetabular implant has an insert with a thickness AI and a metal backing with a thickness AML (acetabular metal liner). The diameter of the acetabular insert is shown as DAI.

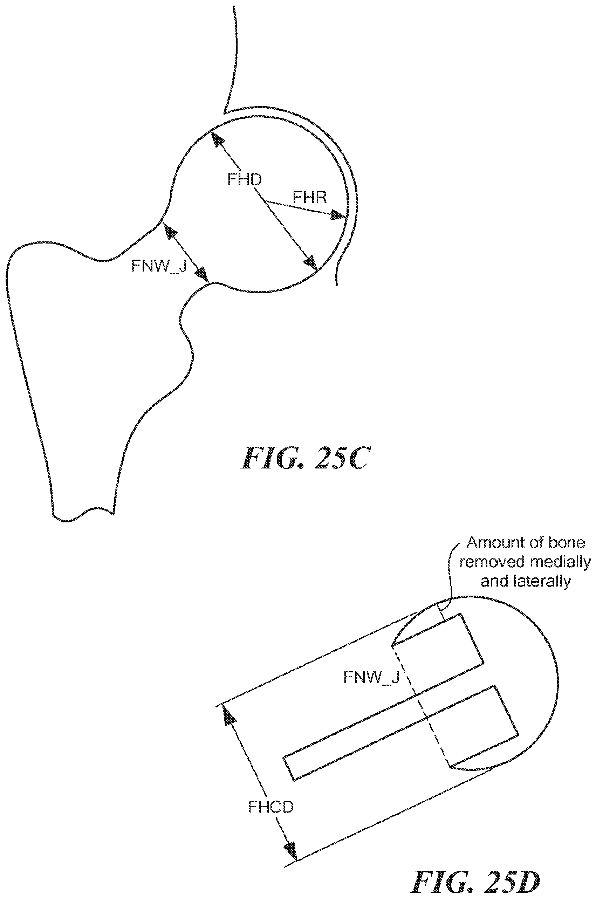

FIG. 25C shows a femoral head and neck of a hip joint of a patient. Femoral cartilage thickness (FC) can optionally be measured or estimated. Femoral head diameter (FHD) and femoral head radius (FHR) are also indicated. Femoral neck width at the head neck junction (FNW_J) is also indicated.

FIG. 25D shows a femoral head having a femoral head component diameter (FHCD) as indicated. Diameter of the mill for amount of bone removed medially and laterally from femoral head to can be matched to FNW_J. Amounts of bone removed medially and lateral from femoral head can be determined. As described herein, the intended milling parameters (e.g., diameter of the mill, amount of bone removed) can be used to create a milling tool that is adapted to the individual patient.

The following example illustrates the calculation of various implant parameters:

Native acetabular diameter (AD) of a patient: 5.8 cm
Estimated or actual acetabular cartilage (AC) thickness of the patient: 2mm (to be assessed only optionally)
Acetabular metal liner (AML) thickness: 2mm
Acetabular [optionally polyethylene] insert (AI): 4mm
Surgical plan: depth of intended acetabular reaming (AR): 2mm
Resultant offset (O)of the patients native acetabular joint space:

$$O = AML + AI - AR - AC$$

In the above example: O = 2mm + 4mm - 2mm - 2mm = 2mm, and therefore, the acetabular bearing surface will be moved distally by 2mm.

The distal displacement of the acetabular bearing surface means that the resultant diameter and radius of the femur facing bearing surface of the acetabular insert will be smaller than the diameter and radius of the native bearing surface of the patient. Typically, the diameter will be smaller by 2 X O and the radius will be smaller by 1 X O and plus additional reductions / offsets needed to allow sufficient play between the femoral and the acetabular implant bearing surface.

In the above example, the diameter of the femur facing bearing surface of the acetabular insert (DAI) will be 5.8 cm -0.4cm = 5.4cm.

The implication is that the matching bearing surface of the femoral head component will need to be slightly smaller than 5.4cm in this patient. A matching component can be selected, adapted to this size or designed for these dimensions.

Native femoral head diameter (FHD) of the patient: 5.7mm
Native femoral head radius (FHR) of the patient: 2.85mm
Estimated or actual femoral cartilage (FC) thickness of the patient: 2mm (to be assessed only optionally)
Minimum material thickness (MMT) of the resurfacing femoral component: 3mm
Desired play (P) between acetabular and femoral component: 0.5mm
Amount of bone to be removed (BR) near fovea capitis region (and optionally other femoral head regions):

$$BR = (FHD - DAI) + P + MMT = (5.7\,cm - 5.4cm) + 0.1cm + 0.3cm = 6.5mm$$

If a cylindrical mill is used, the amount of bone to be removed medially and laterally can, for example, be determined by the femoral neck width at the head neck junction (FNW_J).

In the above example, FNW_J of this particular patient is: 4.4cm; Femoral head component diameter (FHCD): FHCD = DAI - P = 5.3cm; Diameter of the mill for amount of bone removed medially and laterally from femoral head to match to FNW_J: 4.4cm; Amount of bone removed medially from femoral head: (5.7cm - 4.4cm)/2 = 0.65cm; and Amount of bone removed laterally from femoral head: (5.7cm - 4.4cm)/2 = 0.65cm.

These calculations and optimizations for implant selection, adaptation or design as described above can be initiated from the femoral side and then carried through on the acetabular side. Thus, the amount or depth of acetabular remaining can be determined based on the desired amount of femoral bone removal or the desired femoral implant component thickness or combinations thereof. If the resultant acetabular reaming would be excessive, both the amount of femoral bone removal (including medial and lateral bone removal with, for example, a mill and removal of bone near the fovea capitis region) and the depth of acetabular reaming can be optimized against each other for a given material thickness of the different components. The material thickness can include a desirable threshold value including a minimum material thickness, e.g. of polyethylene as a means of allowing for or compensation for future wear or of metal as a means of avoiding component fractures. The material thickness of each component can be adjusted based on patient-specific information or parameters including weight, height, sex, age, femoral head size, acetabular size or dimensions etc. In addition, the component thickness can be adjusted using kinematic modeling and finite element modeling, both of which can include patient-specific parameters (e.g. the preceding parameters as well as bone shape, dimensions, bone density, trabecular structure etc.).

While this example is directed to a metal-on-polyethylene system, the same design or selection rationale can be applied to design or select a metal-on-ceramic, all-polyethylene or all-ceramic system. Various material combinations are possible. Some of the following exemplary combinations can be used to avoid metal on metal bearings.

| Femoral Implant (or First Implant Component) | Acetabular cup (or Second Implant Component) |
| --- | --- |
| Metal | Metal backing with polyethylene insert |
| Metal | Metal backing with ceramic insert |
| Metal | All polyethylene component |
| Metal | All ceramic component |
| Metal with ceramic coating | Metal backing with polyethylene insert |
| Metal with ceramic coating | Metal backing with ceramic insert |
| Metal with ceramic coating | All polyethylene component |
| Metal with ceramic coating | All ceramic component |
| Ceramic | Metal backing with polyethylene insert |
| Ceramic | Metal backing with ceramic insert |
| Ceramic | All polyethylene component |
| Ceramic | All ceramic component |

## COLLECTING AND MODELING PATIENT-SPECIFIC DATA

As mentioned above, certain embodiments include implant components designed and made using patient-specific data that is collected preoperatively. The patient-specific data can include points, surfaces, or landmarks, collectively referred to herein as "reference points." In certain embodiments, the reference points can be selected and used to derive a varied or altered surface, such as, without limitation, an ideal surface or structure. For example, the reference points can be used to create a model of the patient's relevant biological feature(s) or one or more patient-adapted surgical steps, tools, and implant components. Further, the reference points can be used to design a patient-adapted implant component having at least one patient-specific or patient-engineered feature, such as a surface, dimension, or other feature.

Sets of reference points can be grouped to form reference structures used to create a model of a joint or an implant design. Designed implant surfaces can be derived from single reference points, triangles, polygons, or more complex surfaces, such as parametric or subdivision surfaces, or models of joint material, such as, for example, articular cartilage, subchondral bone, cortical bone, endosteal bone or bone marrow. Various reference points and reference structures can be selected and manipulated to derive a varied or altered surface, such as, without limitation, an ideal surface or structure.

The reference points can be located on or in the joint that receive the patient-adapted implant. For example, the reference points can include weight-bearing surfaces or locations in or on the joint, a cortex in the joint, or an endosteal surface of the joint. The reference points also can include surfaces or locations outside of but related to the joint. Specifically, reference points can include surfaces or locations functionally related to the joint. For example, in embodiments directed to the hip joint, reference points can include one or more locations ranging from the hip down to knee, the ankle or foot. The reference points also can include surfaces or locations homologous to the joint receiving the implant. For example, in embodiments directed to a knee, a hip, or a shoulder joint, reference points can include one or more surfaces or locations from the contralateral knee, hip, or shoulder joint.

In certain embodiments, an imaging data collected from the patient, for example, imaging data from one or more of x-ray imaging, digital tomosynthesis, cone beam CT, non-spiral or spiral CT, non-isotropic or isotropic MRI, SPECT, PET, ultrasound, laser imaging, photo-acoustic imaging, is used to qualitatively or quantitatively measure one or more of a patient's biological features, one or more of normal cartilage, diseased cartilage, a cartilage defect, an area of denuded cartilage, protrusion acetabuli, osteophyte and other abnormalities, acetabular wall thickness, subchondral bone, cortical bone, endosteal bone, bone marrow, a ligament, a ligament attachment or origin, menisci, labrum, a joint capsule, articular structures, or voids or spaces between or within any of these structures. The qualitatively or quantitatively measured biological features can include, but are not limited to, one or more of length, width, height, depth or thickness; curvature, for example, curvature in two dimensions (e.g., curvature in or projected onto a plane), curvature in three dimensions, or a radius or radii of curvature; shape, for example, two-dimensional shape or three-dimensional shape; area, for example, surface area or surface contour; perimeter shape; or volume of, for example, the patient's cartilage, bone (subchondral bone, cortical bone, endosteal bone, or other bone), ligament, or voids or spaces between them.

In certain embodiments, measurements of biological features can include any one or more of the illustrative measurements identified in Table 3.

**Table 3: Exemplary patient-specific measurements of biological features that can be used in the creation of a model or in the selection or design of an implant component**

| Anatomical feature | Exemplary measurement |
|---|---|
| Joint-line, joint gap | Location relative to proximal reference point |
| | Location relative to distal reference point |
| | Angle |
| | Gap distance between opposing surfaces in one or more locations |
| | Location, angle, or distance relative to contralateral joint |
| Soft tissue tension or balance | Joint gap distance |
| | Joint gap differential, e.g., medial to lateral |
| Medullary cavity | Shape in one or more dimensions |
| | Shape in one or more locations |
| | Diameter of cavity |
| | Volume of cavity |
| Subchondral bone | Shape in one or more dimensions |

| Anatomical feature | Exemplary measurement |
|---|---|
| | Shape in one or more locations |
| | Thickness in one or more dimensions |
| | Thickness in one or more locations |
| | Angle, e.g., resection cut angle |
| Cortical bone | Shape in one or more dimensions |
| | Shape in one or more locations |
| | Thickness in one or more dimensions |
| | Thickness in one or more locations |
| | Angle, e.g., resection cut angle |
| | Portions or all of cortical bone perimeter at an intended resection level |
| Endosteal bone | Shape in one or more dimensions |
| | Shape in one or more locations |
| | Thickness in one or more dimensions |
| | Thickness in one or more locations |
| | Angle, e.g., resection cut angle |
| Cartilage | Shape in one or more dimensions |
| | Shape in one or more locations |
| | Thickness in one or more dimensions |
| | Thickness in one or more locations |
| | Angle, e.g., resection cut angle |
| Femoral head | 2D or 3D shape of a portion or all |
| | Height in one or more locations |
| | Length in one or more locations |
| | Width in one or more locations |
| | Depth in one or more locations |
| | Thickness in one or more locations |
| | Curvature in one or more locations |
| | Slope in one or more locations or directions |
| | Angle, e.g., resection cut angle |
| | Anteversion or retroversion |
| | Portions or all of bone perimeter at an intended resection level |
| | Resection surface at an intended resection level |

| Anatomical feature | Exemplary measurement |
| --- | --- |
| | Abnormalities, e.g., osteophytes |
| **Femoral neck** | 2D or 3D shape of a portion or all |
| | Height in one or more locations |
| | Length in one or more locations |
| | Width in one or more locations |
| | Depth in one or more locations |
| | Thickness in one or more locations |
| | Angle in one or more locations |
| | Neck axis in one or more locations |
| | Curvature in one or more locations |
| | Slope in one or more locations or directions |
| | Angle, e.g., resection cut angle |
| | Anteversion or retroversion |
| | Leg length |
| | Portions or all of cortical bone perimeter at an intended resection level |
| | Resection surface at an intended resection level |
| | Size and shape of the outer surface (the entire neck or one or more portions thereof) |
| | Abnormalities, e.g., osteophytes |
| **Femoral shaft** | 2D or 3D shape of a portion or all |
| | Height in one or more locations |
| | Length in one or more locations |
| | Width in one or more locations |
| | Depth in one or more locations |
| | Thickness in one or more locations |
| | Angle in one or more locations |
| | Shaft axis in one or more locations |
| | Curvature in one or more locations |
| | Angle, e.g., resection cut angle |
| | Anteversion or retroversion |
| | Leg length (desired or measured, and/or contralateral leg length) |

| Anatomical feature | Exemplary measurement |
| --- | --- |
| | Portions or all of cortical bone perimeter at an intended resection level |
| | Resection surface at an intended resection level |
| | Other defects or abnormalities (e.g., osteophyte) |
| **Acetabulum** | 2D or 3D shape of a portion or all |
| | Height in one or more locations |
| | Length in one or more locations |
| | Width in one or more locations |
| | Depth in one or more locations |
| | Thickness in one or more locations |
| | Curvature in one or more locations |
| | Slope in one or more locations or directions |
| | Angle, e.g., resection cut angle |
| | Anteversion or retroversion |
| | Portions or all of cortical bone perimeter at an intended resection level |
| | Resection surface at an intended resection level including the size and shape of the surface, the size and the shape of the surface perimeter/rim |
| | Other defects or abnormalities (e.g., protrusion acetabuli) |

In certain embodiments, the model that includes at least a portion of the patient's joint also can include or display, as part of the model, one or more resection cuts, one or more drill holes, (e.g., on a model of the patient's femur), one or more guide tools, or one or more implant components that have been designed for the particular patient using the model. Moreover, one or more resection cuts, one or more drill holes, one or more guide tools, or one or more implant components can be modeled and selected or designed separate from a model of a particular patient's biological feature.

**MODELING AND ADDRESSING JOINT DEFECTS**

In certain embodiments, the reference points or measurements described above can be processed using mathematical functions to derive virtual, corrected features, which may represent a restored, ideal or desired feature from which a patient-adapted implant component can be designed. For example, one or more features, such as surfaces or dimensions of a biological structure can be modeled, altered, added to, changed, deformed, eliminated, corrected or otherwise manipulated (collectively referred to herein as "variation" of an existing surface or structure within the joint).

Variation of the joint or portions of the joint can include, without limitation, variation of one or more external surfaces, internal surfaces, joint-facing surfaces, uncut surfaces, cut surfaces, altered surfaces, or partial surfaces as well as osteophytes, subchondral cysts, geodes or areas of eburnation, joint flattening, contour irregularity, loss of normal shape, bone sclerosis, other arthritic or congenital deformity, and other abnormalities that may be particular to a joint (e.g., protrusion acetabuli in a hip joint). The surface or structure can be or reflect any surface or structure in the joint, including, without limitation, bone surfaces, ridges, plateaus, cartilage surfaces, ligament surfaces, or other surfaces or structures. The surface or structure derived can be an approximation of a healthy joint surface or structure or can be another variation. The surface or structure can be made to include pathological alterations of the joint. The surface or structure also can be made whereby the pathological joint changes are virtually removed in whole or in part.

Once one or more reference points, measurements, structures, surfaces, models, or combinations thereof have been selected or derived, the resultant shape can be varied, deformed or corrected. In certain embodiments, the variation can be used to select or design an implant component having an ideal or optimized feature or shape, e.g., corresponding to the deformed or corrected joint features or shape. For example, in one application of this embodiment, the ideal or optimized implant shape reflects the shape of the patient's joint before he or she developed arthritis.

Alternatively or in addition, the variation can be used to select or design a patient-adapted surgical procedure to address the deformity or abnormality. For example, the variation can include surgical alterations to the joint, such as virtual resection cuts, virtual drill holes, virtual removal of osteophytes, or virtual building of structural support in the joint that may be desired for a final outcome for the patient. Corrections can be used to address osteophytes, subchondral voids, and other patient-specific defects or abnormalities. In the case of osteophytes, a design for the bone-facing surface of an implant component or guide tool can be selected or designed after the osteophyte has been virtually removed. Alternatively, the osteophyte can be integrated into the shape of the bone-facing surface of the implant component or surgical tool (e.g., a guide tool).

In addition to osteophytes and subchondral voids, the methods, surgical strategies, guide tools, and implant components described herein can be used to address various other patient-specific joint defects or phenomena. In certain embodiments, correction can include the virtual removal of tissue, for example, to address an articular defect, to remove subchondral cysts, or to remove diseased or damaged tissue (e.g., cartilage, bone, or other types of tissue), such as osteochondritic tissue, necrotic tissue, or torn tissue. In such embodiments, the correction can include the virtual removal of the tissue (e.g., the tissue corresponding to the defect, cyst, disease, or damage) and the bone-facing surface of the implant component can be derived after the tissue has been virtually removed. In certain embodiments, the implant component can be selected or designed to include a thickness or other features that substantially matches the removed tissue or optimizes one or more parameters of the joint. Optionally, a surgical strategy or one or more guide tools can be selected or designed to reflect the correction and correspond to the implant component.

Certain embodiments described herein include collecting and using data from imaging tests to virtually determine in one or more planes one or more of an anatomical axis and a mechanical axis and the related misalignment of a patient's limb. The imaging tests that can be used to virtually determine a patient's axis and misalignment can include one or more of such as x-ray imaging, digital tomosynthesis, cone beam CT, non-spiral or spiral CT, non-isotropic or isotropic MRI, SPECT, PET, ultrasound, laser imaging, and photoacoustic imaging, including studies utilizing contrast agents. Data from these tests can be used to determine anatomical reference points or limb alignment, including alignment angles within the same and between different joints or to simulate normal limb alignment. Using the image data, one or more mechanical or anatomical axes, angles, rotations, anteversion/retroversion, orientations, planes or combinations thereof can be determined In certain embodiments, such axes, angles, or planes can include, or be derived from, one or more of a Whiteside's line, Blumensaat's line, transepicondylar line, femoral shaft axis, femoral neck axis, acetabular angle, lines tangent to the superior and inferior acetabular margin, lines tangent to the anterior or posterior acetabular margin, femoral shaft axis, tibial shaft axis, transmalleolar axis, posterior condylar line, tangent(s) to the trochlea of the knee joint, tangents to the medial or lateral patellar facet, lines tangent or perpendicular to the medial and lateral posterior condyles, lines tangent or perpendicular to a central weight-bearing zone of the medial and lateral femoral condyles, lines transecting the medial and lateral posterior condyles, for example through their respective centerpoints, lines tangent or perpendicular to the tibial tuberosity, lines vertical or at an angle to any of the aforementioned lines, or lines tangent to or intersecting the cortical bone of any bone adjacent to or enclosed in a joint. Moreover, estimating a mechanical axis, an angle, or plane also can be performed using image data obtained through two or more joints, such as the knee and ankle joint, for example, by using the femoral shaft axis and a centerpoint or other point in the ankle, such as a point between the malleoli.

As one example, if surgery of the hip is contemplated, the imaging test can include acquiring data through at least one of, or several of, a hip joint, knee joint or ankle joint. As another example, if surgery of the hip joint is contemplated, an acetabular center axis (ACA) can be determined. Conventionally, the anterior pelvic plane (APP) is used to identify the cup and acetabular orientation in navigated THR. As known in the art, the APP is based on the two anterior superior iliac spines (ASIS) and the two pubic tubercles. It has been shown that ACA registration (e.g., 3 points on the acetabular rim) is more accurate with respect to an individual patient than APP registration.

Similarly, any of these determinations can be made in any desired planes, e.g., sagittal or coronal, in two or three dimensions.

## LEG/LIMB LENGTH

In a hip replacement procedure, one important consideration is the resultant leg length of the patient after the surgery. For example, leg-length discrepancy has been a known complication after total hip arthroplasty. Such discrepancy has been associated with complications including nerve palsy, low back pain, and abnormal gait. Further, patients undergoing THR usually require limb lengthening. Conventional methods of intra-operative limb length measurement are based on the distance between two reference points marked on the pelvis and the femur. However, the location of the reference point on the pelvis varies in each case and the line between the two reference points is generally not parallel to the limb lengthening axis, resulting in a discrepancy between intraoperative limb length and post-operative radiographic measurement.

Accordingly, hip implant components and surgical instruments including patient-adapted instruments or jigs can be selected or designed to achieve a desired leg length, for example the same leg length that the patient had in the affected extremity prior to the surgery, the desired lengthened leg length, or the desired length equality between the two limbs.

Leg length can be determined preoperatively, for example with use of a clinical examination, an x-ray, a CT scan, a CT scout scan, or an MRI scan or any other technology. Leg length can be determined using anatomical landmarks known in the art, e.g. location of the ankle joint line, knee joint line, hip joint line, center of the femoral head.

To illustrate, a best fitting implant or implant components can be selected or a patient-adapted implant or implant components can be designed based the patient-specific data. A composite thickness of the acetabular component of the implant can then be determined. A composite length of femoral component can also be determined, based on a combination of stem length, femoral neck length, femoral neck angle, femoral ante or retroversion (as planned or desired).

A virtual simulation of the surgical procedure is then conducted. The surgical approach may begin with the acetabulum or alternatively the femur. The following factors may contribute to the resultant leg length: length of femoral diaphysis or neck reaming to accommodate stem; location of femoral head or neck resection; angle of femoral head or neck resection. The virtual modeling can optimize one or more of these factors so that the resultant leg length accounting for composite femoral and acetabular component thickness or length is identical or similar to desired leg length, e.g. leg length prior to resection, or leg length of contralateral side or combinations thereof, or either with a surgeon selected offset applied.

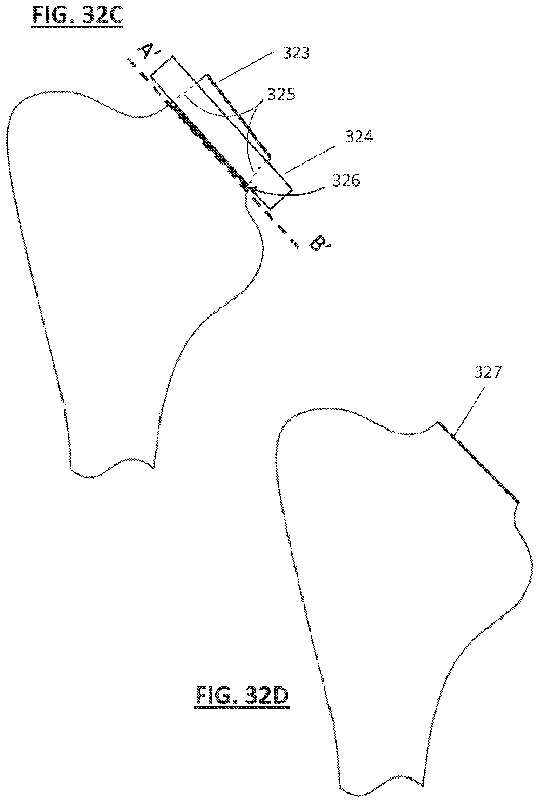

Patient-specific jigs can be adapted or designed from the above simulations. For example, the angle of any resection guides can be designed to accommodate the desired resection angles, e.g. femoral neck resection angle, or to accommodate a desired resection level. To illustrate, FIG. 32C illustrates a femoral neck resection guide tool 324 created for a patient; the guide tool has an interior or bone-facing surface 325 that engages the resected femoral neck 323 at a predetermined location obtained by, e.g., the above simulations and other preoperative analyses as described herein to obtain patient-specific information. In certain embodiments, the bone-facing surface 325 may be designed or selected to have one or more portions that register or reference to one or more osteophytes of the femoral neck 322, particularly, the resected femoral neck 323. The guide tool 324 includes a guide 326 that defines a resection plane or path as indicated by the dashed line A'B'. The guide tool 324 can include one or more features, e.g., embedded in the bone-facing surface or an outer surface portion, that register or reference to one or more other anatomical landmarks (e.g., normal or abnormal bone features, associated soft tissues) of the hip joint. Such anatomical landmarks may be identified through analyzing electronic image data of the hip joint. Through virtual modeling and simulation, location (including orientation) of the guide 326, which defines the resection plane or path A'B', can be preoperatively determined to achieve a desired femoral neck angle, femoral stem length, and final, desired leg length. The virtual modeling and simulation may take into consideration anchoring of the femoral stem at a desired femoral neck resection level as well as bone preservation, achieving a final, composite result of maximizing anchoring strength for the femoral stem while preserving bone of the femoral neck. The registration or anatomical references of the guide tool discussed above improve accuracy of positioning the femoral neck resection guide, thereby allowing the femoral neck resection at the predetermined resection plane or path, ultimately achieving the desired leg length. The resection plane can also be determined based on one or more of the following constraints: maximized bone preservation (of the femoral neck), sufficient or optimized access to the femoral canal, and accurate alignment of the femoral stem to be implanted. In certain embodiments, the leg length can be determined or maintained by referencing one or more anatomical landmarks (e.g., normal or abnormal bone features including osteophytes).

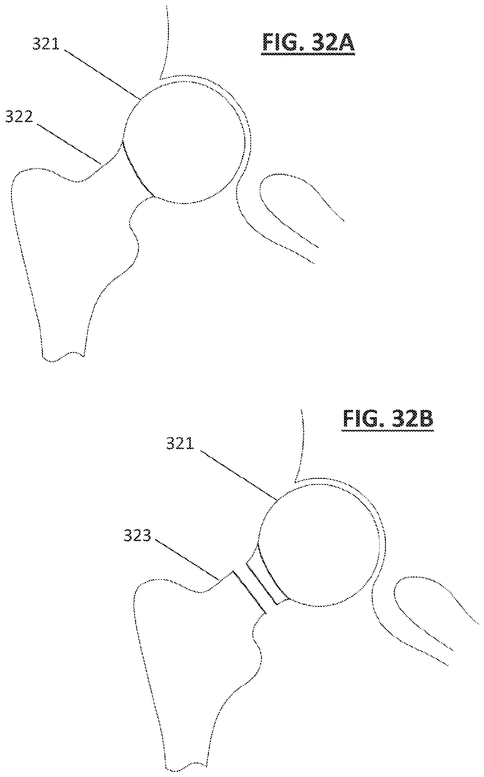

The femoral neck resection guide, e.g., the guide tool 324 as shown in FIG. 32C, can be useful in a hip arthroplasty that utilizes a minimally invasive, anterior approach. Typically, with that approach, soft tissues include muscles around the hip joint remain intact after the initial incision allowing the surgeon to expose the femoral head and neck junction. Such an approach requires the surgeon to separate the femur from the acetabulum by, e.g., first separating the femoral head first (e.g., FIGS. 32A and 32B) from the femoral neck with a first resection or partially cut the femoral head so that the remaining femur can be separated from the acetabulum. Then, the surgeon needs to cut the femoral neck again before preparing for implanting the femoral stem. That second femoral neck resection can be inaccurate due to the separation from the acetabulum and lack of accurate anatomical reference. Therefore, a guide tool (e.g., the tool 324) that fits over the femoral neck 323 already separated from the femoral head 321 (or alternatively, the remaining femoral head and femoral neck when a partial femoral head cut is performed first) at a predetermined location or position through a conforming or negatively-matching interior surface 325, optionally further including registration or references to anatomical references (e.g., osteophytes of the femur) improves the accuracy of the second femoral neck resection by directing the resection tool (e.g., a surgical saw) at the predetermined resection plane or path A'B'.

In certain embodiments, the patient-adapted surgical tool also includes features derived from other patient-specific information such as one or more of bone quality as reflected by, e.g., microanatomy such as trabecular structure, biomechanical, biomotion, and kinematic measurements and preferences by the patient's surgeon (e.g., mesialization of the acetabular component or mesialized reaming, lateralization of the femoral component or femoral stem).

Accordingly, a method of surgical planning for a hip arthroplasty is provided. In certain examples, the method includes simulating a femoral neck or a partial femoral head (e.g., to release the resected femur from the acetabulum) resection as described herein. In certain embodiments, the femoral neck resection guide gives rise to a desired femoral neck angle, anchoring of the femoral stem, and resultant leg length as simulated. The simulation can further include designing, selecting and/or adapting a femoral neck resecting guide which includes a patient-specific portion (e.g., an interior surface or surface portion or an attached or attachable feature) that references to or registers one or more anatomical landmarks of the patient's hip joint. The anatomical landmarks may include normal (e.g., macroanatomical landmarks) and/or abnormal (e.g., osteophytes) features of the patient's hip joint. The surgical planning method can further include constraints from a desired surgical technique or approach (e.g., a minimally invasive anterior approach) and/or a surgeon's preference (e.g., generally or specific to the individual patient).

Accordingly, a method of hip arthroplasty is provided. In certain examples, the method utilizes a minimally invasive technique. For example, a surgeon may use an anterior approach on a patient and make a small incision to allow for the separation of the patient's femoral head from the femoral neck. The minimal invasiveness of such an approach results in nearly intact soft tissue including muscle and ligaments associated with the patient's hip joint. In certain embodiments, the surgeon separates the femoral head from the neck by a free-hand approach, for example, approximately at the base of the femoral head that meets the neck or alternatively resecting the femoral head partially to free the femur from the acetabulum of the patient's hip joint. Alternatively, a cut guide may be selected or designed, preoperatively or intraoperatively, for the patient, that allows the surgeon to separate the femoral head from the neck at a particular location, which can be planned preoperatively, or determined intraoperatively.

In certain embodiments, the surgeon next uses a femoral neck cut/resection guide as described herein, by engaging the guide with the now-separated femoral neck at a predetermined location and/or orientation by positioning the guide so as to ensure that its patient-specific surface, e.g., at least a portion of its interior surface, conforms to or registers the corresponding portion of the separated femoral neck, and/or its patient-specific portion references to or registers an anatomical landmark of the hip joint such as a bone abnormality including an osteophyte. In certain embodiments, the surgeon next cuts the femoral neck at the predetermined location and/or orientation as directed by an instrument guide integrally formed in the femoral neck cut guide. Alternatively, the surgeon attaches an instrument guide to the femoral neck resection guide that has engaged the femoral neck at the predetermined location or orientation. The instrument guide is attached to the femoral neck resection guide at a predetermined location and/or orientation. The femoral neck resection guide can then be removed from or left engaged with the femoral neck. The instrument guide is configured to direct the movement of a surgical instrument that allows for the preoperatively planned femoral neck cut.

Certain embodiments also provide one or more stop features that restrict the movement of the surgical instrument as directed by the instrument guide, either integrally formed in the femoral neck cut guide, or separately formed but attached/linked and/or referencing to the femoral neck cut guide when used in surgery. The one or more stop features can be configured to include patient-specific information, e.g., size, shape and/or location of soft tissue associated with the hip joint, so as to prevent undesirable damages to such soft tissue (e.g., blood vessel) during the surgery.

When changes are made on multiple articular surfaces or implant components, these can be made in reference to or linked to each other. For example, in the hip, a change made to a femoral neck cut based on patient-specific biomotion data can be referenced to or linked with a concomitant change on an opposing acetabular surface (e.g., a bone cut such as reaming depth on the opposing surface or a parameter of the acetabular component such as a radius), for example, if less femoral bone is resected, the computer program may elect to remove more acetabular bone or select an acetabular component of a different (e.g., smaller) size.

Similarly, in a hip implant, if an acetabular component shape is changed, for example on an external or joint-facing surface, this can be accompanied by a change in the femoral component shape. This is, for example, particularly applicable when at least portions of the femoral head bearing surface negatively-match the acetabular joint-facing surface.

Similarly, in a hip implant, if a femoral component shape is changed, for example on an external surface, this can be accompanied by a change in an acetabular component shape. This is, for example, particularly applicable when at least portions of the acetabular joint-facing surface substantially negatively-match the femoral joint-facing surface. For example, the acetabular rim can be altered, for example via reaming or cutting. These surgical changes and resultant change on cortical bone profile can be virtually simulated and a new resultant peripheral margin(s) can be derived. The derived peripheral bone margin or shape can then be used to design or select an implant that substantially matches, in at least a portion, the altered rim or joint margin or edge.

## LOCKING MECHANISMS

An implant or implant component as disclosed herein may have at least two parts, made of the same or different materials, such as metal or polymeric material (e.g., oxidation resistant UHMWPE). Various embodiments of implants herein can include a scaffold or stage with one or more polymer inserts that can be inserted and locked into the scaffold. One exemplary embodiment is an acetabular cup implant for a hip joint that is configured to be implanted onto a patient's acetabulum for receiving the femoral head or femoral implant. The acetabular implant comprises at least two components: a first component that engages the acetabulum/socket, which can be made of metal; and a second component that is configured to articulate with the femoral head component of a femoral implant, which can be made of non-metal, e.g., plastic polymer, to provide a non-metal articulating surface.

The first acetabular component can be shaped generally as a hemisphere that fits the patient's acetabulum. In certain embodiments, the first acetabular component includes a first surface that engages with the acetabulum and a second surface that engages the femoral implant and provides the articulating surface. The first surface is preferably designed or selected with one or more patient-adapted features (e.g., size, shape, curvature) and provides an anatomical or near anatomical fit with the patient's acetabulum.

The second surface of the first acetabular component can be substantially flat or can have at least one or more curved portions. There can be a wall that spans the perimeter (anterior, posterior, medial, or lateral) of the second surface. This wall can optionally contain grooves along the inner surface for accepting an insert component of the implant, e.g., the second acetabular component. The wall can extend into the middle of the second surface of the first acetabular component from the posterior side towards the anterior side, approximately halfway between the medial and lateral sides, creating a peninsular wall on the second surface. The outward facing sides of this peninsular wall can optionally be sloped for mating with the insert component of the implant. Towards the end of the peninsular wall, receptacles can optionally be cut into either side of the wall for receiving an optional locking member formed into the surface of the insert of the implant. Perpendicular to the peninsular wall there can be one or more grooves cut into the second surface of the first acetabular component for accepting a notched portion extending from a surface of the insert of the implant. One (e.g., anterior) side of the second surface of the first acetabular component can contain at least one slanted surface that acts as a ramp to assist with proper alignment and engagement of the insert component with the first acetabular component.

The articulating component, or insert component, has a first surface and a second surface. The first surface of the insert component can be shaped to align with the shape or geometry of the joint or the bearing surface of the opposite implant component by having one or more concave surfaces that are articulate with the convex surfaces of the femoral implant.

The lower surface of the insert component can be flat and is configured to mate with the second surface of the first component of the implant. The posterior side of the implant can be cut out from approximately half way up the medial side of the implant to approximately halfway down the lateral side of the implant to align with the geometrically matched wall of the second surface of the first acetabular component. The remaining structure on the lower surface of the implant can have a ledge extending along the medial and posterior sides of the surface for lockably mating with the grooves of the interior walls of the second surface of the first acetabular component. Approximately halfway between the medial side and the lateral side of the implant, a canal can be formed from the posterior side of the implant towards the anterior side of the implant, for mating with the peninsular wall of the second surface of the first acetabular component. This canal can run approximately 3/4 the length of the implant from the posterior to anterior of the lower surface of the implant. The exterior walls of this canal can be sloped inward from the bottom of the canal to the top of the canal creating a surface that dovetails with the sloped peninsular walls of the second surface of the first acetabular component. This dovetail joint can assist with proper alignment of the insert into the first acetabular component and then locks the insert into the first acetabular component once fully inserted. At the anterior end of the canal, there can be a locking mechanism consisting of bendable fingers that snap optionally into the receptacles cut into the interior of the peninsular walls upon insertion of the insert into the first acetabular component of the implant, thereby locking the implant component into the first acetabular component. Perpendicular to the canal running 3/4 the length of the lower surface of the insert can be at least one notch for mating with the at least one groove cut out of the upper surface of the first acetabular component. Engagement between the first acetabular component and the second acetabular component can be fixed or reversible

Similarly, a femoral implant or implant component in certain embodiments includes two components, with a first femoral component engages the patient's femur and a second femoral component that engages the first femoral component and provides the articulating surface to engage with the articulating surface of the acetabular implant component. The first femoral component can be made of metal, whereas the second femoral component can include or provide a non-metal articulating surface. Engagement between the first femoral component and the second femoral component can be fixed or reversible.

Thus, multiple locking mechanisms can be designed into the opposing surfaces of the walls and canal of the insert and the implant component, as well as the notch and groove and they can help to lock the insert into place on the first acetabular or femoral component and resist against various motions within the joint.

## MANUFACTURING AND MACHINING

The implants and implant components of this disclosure can be machined, molded, casted, manufactured through additive techniques such as laser sintering or electron beam melting or otherwise constructed out of a metal or metal alloy such as cobalt chromium. Similarly, an insert component may be machined, molded, manufactured through rapid prototyping or additive techniques or otherwise constructed out of a plastic polymer such as ultra high molecular weight polyethylene.

An example of such a plastic polymer is vitamin E-infused or cross-linked high or ultra-high molecular weight polyethylene. Other examples of plastic polymers can be found in the art, such as those described in U.S. Patent Application Publication Nos. 20110112646 20110109017, 20070004818, etc. Ultra-high molecular weight polyethylene (UHMWPE) generally refers to linear non-branched chains of ethylene having molecular weights in excess of about 500,000, preferably above about 1,000,000, and more preferably above about 2,000,000. Often the molecular weights can reach about 8,000,000 or more. Oxidation resistant cross-linked polymeric material, such as ultra-high molecular weight polyethylene (UHMWPE), is desired in medical devices because it significantly increases the wear resistance of the devices. The conventional method of crosslinking is by exposing the UHMWPE to ionizing radiation. Other methods also include doping the UHMWPE with antioxidants, such as vitamin E.

Other known materials, such as ceramics including ceramic coating, may be used as well, for one or both components, or in combination with the metal, metal alloy and polymer described above. It can be appreciated by those of skill in the art that an implant may be constructed as one piece out of any of the above, or other, materials, or in multiple pieces out of a combination of materials. For example, an implant may include one or more surfaces, particularly joint-facing surfaces or bearing surfaces that includes a coating of a material other than metal (e.g., a ceramic coating or a plastic polymer coating or insert component), whereas the implant or implant component includes a metal backing. For example, an implant or implant component constructed of a polymer with a two-piece insert component constructed one piece out of a metal alloy and the other piece constructed out of ceramic.

Each of the components may be constructed as a "standard" or "blank" in various sizes or may be specifically formed for each patient based on the patient-specific data. Computer modeling may be used and a library of virtual standards may be created for each of the components. A library of physical standards may also be amassed for each of the components.

Imaging data including shape, geometry, e.g., radius (or radii) (e.g., of the acetabulum), M-L, A-P, and S-I dimensions, then can be used to select the standard component, e.g., a femoral component or an acetabular component that most closely approximates the select features of the patient's anatomy. Typically, these components are selected so that they are slightly larger than the patient's articular structure that are be replaced in at least one or more dimensions. The standard component is then adapted to the patient's unique anatomy, for example by removing overhanging material, e.g. using machining or other further shaping.

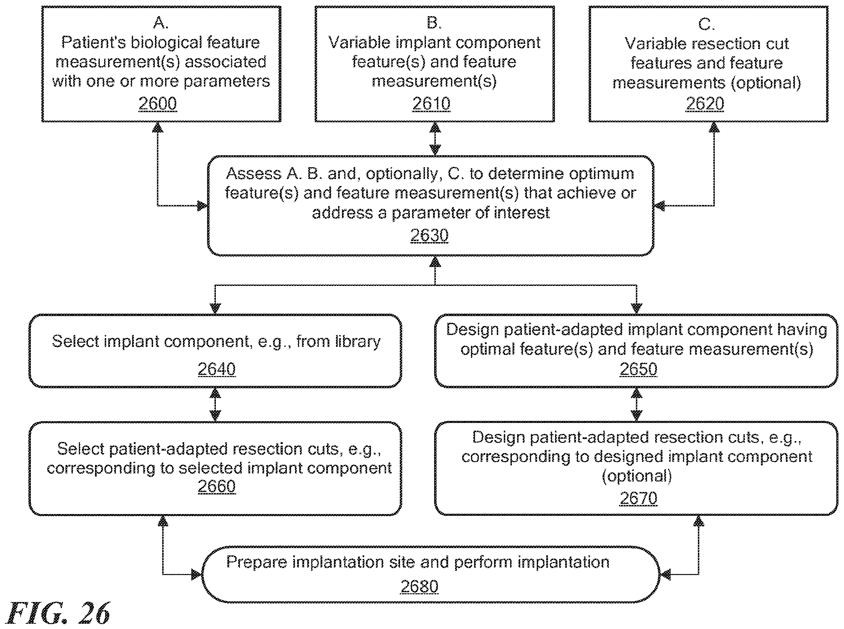

Thus, referring to the flow chart shown in FIG. 26 in a first step 2600, the imaging data is analyzed, either manually or with computer assistance, to determine the patient-specific parameters relevant for placing the implant component. These parameters can include patient-specific articular anatomy, dimensions, shape or geometry and also information about ligament location, size, and orientation, as well as potential soft-tissue impingement, and, optionally, kinematic information.

As illustrated in FIG. 26 is a flow chart illustrating the process of assessing and selecting and/or designing one or more implant component features and/or feature measurements, and, optionally assessing and selecting and/or designing one or more resection cut features and feature measurements, for a particular patient. Using the techniques described herein or those suitable and known in the art, one or more of the patient's biological features and/or feature measurements are obtained 2600. In addition, one or more variable implant component features and/or feature measurements are obtained 2610. Optionally, one or more variable resection cut features and/or feature measurements are obtained 2620. Moreover, one or more variable guide tool features and/or feature measurements also can optionally be obtained. Each one of these step can be repeated multiple times, as desired.

The obtained patient's biological features and feature measurements, implant component features and feature measurements, and, optionally, resection cut and/or guide tool features and/or feature measurements then can be assessed to determine the optimum implant component features and/or feature measurements, and optionally, resection cut and/or guide tool features and/or feature measurements, that achieve one or more target or threshold values for parameters of interest 2630 (e.g., by maintaining or restoring a patient's healthy joint feature). As noted, parameters of interest can include, for example, one or more of (1) joint deformity correction; (2) limb alignment correction; (3) bone, cartilage, and/or ligaments preservation at the joint; (4) preservation, restoration, or enhancement of one or more features of the patient's biology, for example, trochlea and trochlear shape; (5) preservation, restoration, or enhancement of joint kinematics, including, for example, ligament function and implant impingement; (6) preservation, restoration, or enhancement of the patient's joint-line location and/or joint gap width; and (7) preservation, restoration, or enhancement of other target features. This step can be repeated as desired. For example, the assessment step 2630 can be reiteratively repeated after obtaining various feature and feature measurement information 2600, 2610, 2620.

Once the one or more optimum implant component features and/or feature measurements are determined, the implant component(s) can be selected 2640, designed 2650, or selected and designed 2640, 2650. For example, an implant component having some optimum features and/or feature measurements can be designed using one or more CAD software programs or other specialized software to optimize additional features or feature measurements of the implant component. One or more manufacturing techniques described herein or known in the art can be used in the design step to produce the additional optimized features and/or feature measurements. This process can be repeated as desired.

Optionally, one or more resection cut features and/or feature measurements can be selected 2660, designed 2670, or selected and further designed 2660, 2670. For example, a resection cut strategy selected to have some optimum features and/or feature measurements can be designed further using one or more CAD software programs or other specialized software to optimize additional features or measurements of the resection cuts, for example, so that the resected surfaces substantially match optimized bone-facing surfaces of the selected and designed implant component. This process can be repeated as desired.

Moreover, optionally, one or more guide tool features and/or feature measurements can be selected, designed, or selected and further designed. For example, a guide tool having some optimum features and/or feature measurements can be designed further using one or more CAD software programs or other specialized software to optimize additional features or feature measurements of the guide tool. One or more manufacturing techniques described herein or known in the art can be used in the design step to produce the additional, optimized features and/or feature measurements, for example, to facilitate one or more resection cuts that, optionally, substantially match one or more optimized bone-facing surfaces of a selected and designed implant component. This process can be repeated as desired.

As will be appreciated by those of skill in the art, the process of selecting and/or designing an implant component feature and/or feature measurement, resection cut feature and/or feature measurement, and/or guide tool feature and/or feature measurement can be tested against the information obtained regarding the patient's biological features, for example, from one or more MRI or CT or x-ray images from the patient, to ensure that the features and/or feature measurements are optimum with respect to the selected parameter targets or thresholds. Testing can be accomplished by, for example, superimposing the implant image over the image for the patient's joint. In a similar manner, load-bearing measurements and/or virtual simulations thereof may be utilized to optimize or otherwise alter a derived implant design. For example, where a proposed implant for a hip implant has been designed, it may then be virtually inserted into a biomechanical model or otherwise analyzed relative to the load-bearing conditions (or virtually simulations thereof) it may encounter after implantation. These conditions may indicate that one or more features of the implant are undesirable for varying reasons (i.e., the implant design creates unwanted anatomical impingement points, the implant design causes the joint to function in an undesirable fashion, the joint design somehow interferes with surrounding anatomy, the joint design creates a cosmetically-undesirable feature on the repaired limb or skin covering thereof, FEA or other loading analysis of the joint design indicates areas of high material failure risk, FEA or other loading analysis of the joint design indicates areas of high design failure risk, FEA or other loading analysis of the joint design indicates areas of high failure risk of the supporting or surrounding anatomical structures, etc.). In such a case, such undesirable features may be accommodated or otherwise ameliorated by further design iteration and/or modification that might not have been discovered without such analysis relative to the "real world" measurements and/or simulation.

Such load-bearing/modeling analysis may also be used to further optimize or otherwise modify the implant design, such as where the implant analysis indicates that the current design is "over-engineered" in some manner than required to accommodate the patient's biomechanical needs. In such a case, the implant design may be further modified and/or redesigned to more accurately accommodate the patient's needs, which may have an unintended (but potentially highly-desirable) consequence of reducing implant size or thickness, increasing or altering the number of potential implant component materials (due to altered requirements for material strength and/or flexibility), increasing estimate life of the implant, reduce wear or otherwise altering one or more of the various design "constraints" or limitations currently accommodated by the present design features of the implant.

Once optimum features and/or feature measurements for the implant component, and optionally for the resection cuts and/or guide tools, have been selected and/or designed, the implant site can be prepared, for example by removing cartilage and/or resectioning bone from the joint surface, and the implant component can be implanted into the joint 2680.

The joint implant component bone-facing surface, and optionally the resection cuts and guide tools, can be selected and/or designed to include one or more features that achieve an anatomical or near anatomical fit with the existing surface or with a resected surface of the joint. Moreover, the joint implant component joint-facing surface, and optionally the resection cuts and guide tools, can be selected and/or designed, for example, to replicate the patient's existing joint anatomy, to replicate the patient's healthy joint anatomy, to enhance the patient's joint anatomy, and/or to optimize fit with an opposing implant component. Accordingly, both the existing surface of the joint and the desired resulting surface of the joint can be assessed. This technique can be particularly useful for implants that are not anchored into the bone.

As will be appreciated by those of skill in the art, the physician, or other person can obtain a measurement of a biological feature (e.g., a hip joint) 2600 and then directly select 2640, design, 2650, or select and design 2640, 2650 a joint implant component having desired patient-adapted features and/or feature measurements. Designing can include, for example, design and manufacturing.

In the step 2640, one or more standard components, e.g., a femoral component or an acetabular component or acetabular insert, are selected. These are selected so that they are at least slightly greater than one or more of the derived patient-specific articular dimensions and so that they can be shaped to the patient-specific articular dimensions. Alternatively, these are selected so that they do not interfere with any adjacent soft-tissue structures. Combinations of both are possible.

If an implant component is used that includes an insert, e.g., a polyethylene insert and a locking mechanism in a metal or ceramic base, the locking mechanism can be adapted to the patient's specific anatomy in at least one or more dimensions. The locking mechanism can also be patient adapted in all dimensions. The location of locking features can be patient adapted while the locking feature dimensions, for example between an acetabular cup and an acetabular insert, can be fixed. Alternatively, the locking mechanism can be pre-fabricated; in this embodiment, the location and dimensions of the locking mechanism also is considered in the selection of the pre-fabricated components, so that any adaptations to the metal or ceramic backing relative to the patient's articular anatomy do not compromise the locking mechanism. Thus, the components can be selected so that after adaptation to the patient's unique anatomy a minimum material thickness of the metal or ceramic backing is maintained adjacent to the locking mechanism.

In some embodiments, a pre-manufactured metal backing blank can be selected so that its exterior dimensions are slightly greater than the derived patient-specific dimensions or geometry in at least one or more directions, while, optionally, at the same time not interfering with ligaments. The pre-manufactured metal backing blank can include a pre-manufactured locking mechanism for an insert, e.g. a polyethylene insert. The locking mechanism can be completely pre-manufactured, i.e. not requiring any patient adaptation. Alternatively, the locking mechanism can have pre-manufactured components, e.g. an anterior locking tab or feature, with other locking features that will be machined later based on patient-specific dimensions, e.g. a posterior locking tab or feature at a distance from the anterior locking feature that is derived from patient-specific imaging data. In this setting, the pre-manufactured metal blank will be selected so that at least the anterior locking feature will fall inside the derived patient-specific articular dimensions. In a specific embodiment, all pre-manufactured locking features on the metal backing and an insert will fall inside the derived patient-specific articular dimensions. Thus, when the blank is adapted to the patient's specific geometry, shape, or dimensions (e.g., size, thickness, or curvature), the integrity of the lock is not compromised and will remain preserved.

It is contemplated that all combinations of pre-manufactured and patient adapted lock features are possible, including pre-manufactured lock features on a medial insert and patient-specific lock features on a lateral insert or the reverse. Other locations of lock features are possible.

Those of skill in the art can appreciate that a combination of standard and customized components may be used in conjunction with each other. For example, a standard tray component may be used with an insert component that has been individually constructed for a specific patient based on the patient's anatomy and joint information.

The step of designing or selecting an implant or surgical tool as described herein can include both configuring one or more features, measurements, or dimensions of the implant or surgical tool (e.g., derived from patient-specific data from a particular patient and adapted for the particular patient) and manufacturing the implant. In certain embodiments, manufacturing can include making the implant or guide tool from starting materials, for example, metals or polymers or other materials in solid (e.g., powders or blocks) or liquid form. In addition or alternatively, in certain embodiments, manufacturing can include altering (e.g., machining) an existing implant component or guide tool, for example, a standard blank implant component or guide tool or an existing implant or guide tool (e.g., selected from a library). The manufacturing techniques to making or altering an implant component or guide tool can include any techniques known in the art today and in the future. Such techniques include, but are not limited to additive as well as subtractive methods, i.e., methods that add material, for example to a standard blank, and methods that remove material, for example from a standard blank.

Various technologies appropriate for this purpose are known in the art, for example, as described in Wohlers Report 2009, State of the Industry Annual Worldwide Progress Report on Additive Manufacturing, Wohlers Associates, 2009 (ISBN 0-9754429-5-3), available from the web www.wohlersassociates.com; Pham and Dimov, Rapid manufacturing, Springer-Verlag, 2001 (ISBN 1-85233-360-X); Grenda, Printing the Future, The 3D Printing and Rapid Prototyping Source Book, Castle Island Co., 2009; Virtual Prototyping & Bio Manufacturing in Medical Applications, Bidanda and Bartolo (Eds.), Springer, Dec. 17, 2007 (ISBN: 10: 0387334297; 13: 978-0387334295); Bio-Materials and Prototyping Applications in Medicine, Bartolo and Bidanda (Eds.), Springer, Dec. 10, 2007 (ISBN: 10: 0387476822; 13: 978-0387476827); Liou, Rapid Prototyping and Engineering Applications: A Toolbox for Prototype Development, CRC, Sep. 26, 2007 (ISBN: 10: 0849334098; 13: 978-0849334092); Advanced Manufacturing Technology for Medical Applications: Reverse Engineering, Software Conversion and Rapid Prototyping, Gibson (Ed.), Wiley, January 2006 (ISBN: 10: 0470016884; 13: 978-0470016886); and Branner et al., "Coupled Field Simulation in Additive Layer Manufacturing," 3rd International Conference PMI, 2008 (10 pages).

Below is a non-exhaustive list of exemplary techniques for forming or altering a patient-specific and/or patient-engineered implant component for a patient's anatomy:

CNC refers to computer numerically controlled (CNC) machine tools, a computer-driven technique, e.g., computer-code instructions, in which machine tools are driven by one or more computers. Embodiments of this method can interface with CAD software to streamline the automated design and manufacturing process.

CAM refers to computer-aided manufacturing (CAM) and can be used to describe the use of software programming tools to efficiently manage manufacturing and production of products and prototypes. CAM can be used with CAD to generate CNC code for manufacturing three-dimensional objects.

Casting, including casting using rapid prototyped casting patterns, is a manufacturing technique that employs a mold. Typically, a mold includes the negative of the desired shape of a product. A liquid material is poured into the mold and allowed to cure, for example, with time, cooling, and/or with the addition of a solidifying agent. The resulting solid material or casting can be worked subsequently, for example, by sanding or bonding to another casting to generate a final product.

Welding is a manufacturing technique in which two components are fused together at one or more locations. In certain embodiments, the component joining surfaces include metal or thermoplastic and heat is administered as part of the fusion technique.

Forging is a manufacturing technique in which a product or component, typically a metal, is shaped, typically by heating and applying force.

Rapid prototyping refers generally to automated construction of a prototype or product, typically using an additive manufacturing technology, such as EBM, SLS, SLM, SLA, DMLS, 3DP, FDM and other technologies, as briefly explained below:

EBM® refers to electron beam melting (EBM®), which is a powder-based additive manufacturing technology. Typically, successive layers of metal powder are deposited and melted with an electron beam in a vacuum.

SLS refers to selective laser sintering (SLS), which is a powder-based additive manufacturing technology. Typically, successive layers of a powder (e.g., polymer, metal, sand, or other material) are deposited and melted with a scanning laser, for example, a carbon dioxide laser.

SLM refers to selective laser melting™ (SLM), which is a technology similar to SLS; however, with SLM the powder material is fully melted to form a fully-dense product.

SLA or SL refers to stereolithography (SLA or SL), which is a liquid-based additive manufacturing technology. Typically, successive layers of a liquid resin are exposed to a curing, for example, with UV laser light, to solidify each layer and bond it to the layer below. This technology typically requires the additional and removal of support structures when creating particular geometries.

DMLS refers to direct metal laser sintering (DMLS), which is a powder-based additive manufacturing technology. Typically, metal powder is deposited and melted locally using a fiber optic laser. Complex and highly accurate geometries can be produced with this technology. This technology supports net-shaping, which means that the product generated from the technology requires little or no subsequent surface finishing.

LC refers to LaserCusing®(LC), which is a powder-based additive manufacturing technology. LC is similar to DMLS; however, with LC a high-energy laser is used to completely melt the powder, thereby creating a fully-dense product.

3DP refers to three-dimensional printing (3DP), which is a high-speed additive manufacturing technology that can deposit various types of materials in powder, liquid, or granular form in a printer-like fashion. Deposited layers can be cured layer by layer or, alternatively, for granular deposition, an intervening adhesive step can be used to secure layered granules together in bed of granules and the multiple layers subsequently can be cured together, for example, with laser or light curing.

LENS® refers to Laser Engineered Net Shaping™ (LENS®), which is a powder-based additive manufacturing technology. Typically, a metal powder is supplied to the focus of the laser beam at a deposition head. The laser beam melts the powder as it is applied, in raster fashion. The process continues layer by and layer and requires no subsequent curing. This technology supports net-shaping, which means that the product generated from the technology requires little or no subsequent surface finishing.

FDM refers to fused deposition modeling™ (FDM) is an extrusion-based additive manufacturing technology. Typically, beads of heated extruded polymers are deposited row by row and layer by layer. The beads harden as the extruded polymer cools.

## SURGICAL TOOLS

A variety of traditional guide tools are available to assist surgeons in preparing a joint for an implant, for example, for resecting one or more of a patient's biological structures during a joint implant procedure. However, these traditional guide tools typically are not designed to match the shape (contour) of a particular patient's biological structure(s). Moreover, these traditional guide tools typically are not designed to impart patient-optimized placement for the resection cuts. Thus, using and properly aligning traditional guide tools, as well as properly aligning a patient's limb (e.g., in rotational alignment, version, or alignment in another dimension) in order to orient these traditional guide tools, can be an imprecise and complicated part of the implant procedure.

Certain examples described herein provide improved surgical guide tools and methods for preparing a patient's biological structure during a joint implant procedure. In various embodiments, 3D guidance surgical tools can include guides or guide apertures. It is to be understood that the term guide or guide aperture shall be used interchangeably within the detailed description and appended claims to describe guide surfaces, guide elements, limiter or shielding elements, captured cut guides, and/or uncaptured cut guides.

Various embodiments described herein include the use of a guide tool having at least one patient-specific bone-facing surface portion that substantially negatively-matches at least a portion of a biological surface at the patient's joint. The guide tool further can include at least one guide for directing movement of a surgical instrument, for example, a securing pin or a cutting tool. One or more of the guides or apertures can be designed to guide the surgical instrument to deliver a patient-optimized placement for, for example, a securing pin or resection cut. In addition or alternatively, one or more of the guides or apertures can be designed to guide the surgical instrument to deliver a standard placement for, for example, a securing pin or resection cut. As used herein, "jig" also can refer to guide tools, for example, to guide tools that guide resecting of a patient's biological structure. Alternatively, certain guide tools can be used for purposes other than guiding a drill or cutting tool. For example, balancing and trial guide tools can be used to assess joint or joint implant alignment and/or fit of one or more implant components or inserts.

In various embodiments, the apertures, holes, guides and/or resection cut slots in a particular guide tool can be substantially, horizontal, substantially diagonal, or substantially vertical, for example, as compared to the patient's mechanical axis and/or anatomical axis. Moreover, one or more of the resection cut slots can allow for a complete resection cut or a partial resection cut, e.g., scoring of the patient's bone to establish a resection cut that can be finished after removing the tool. This approach can be advantageous by allowing for faster resection in the absence of the guide tool. Moreover, one or more resection cut slots can include a blade-depth or drill-depth stop. This is particularly useful for step resection cuts, for example, vertical step resection cuts, that connect two facets or planes of a resected surface.

Various examples disclosed herein include systems, methods, and devices for performing a series of bone cuts to receive a patient-adapted implant. Specifically, a set of jigs can be designed in connection with the design of a patient-adapted implant component. The designed jigs can guide the surgeon in performing one or more patient-adapted cuts to the bone so that those cut bone surface(s) negatively-match patient-adapted bone-facing surfaces of corresponding patient-adapted implant components.

Surgical assistance can be provided by using a device applied to an outer surface portion of a joint, e.g., the outer surface of the articular cartilage or the bone, including the subchondral bone, in order to match the alignment of the articular repair system and the recipient site or the joint. The device can be round, circular, oval, ellipsoid, curved or irregular in shape. The shape can be designed, selected or adjusted to match or enclose an area of diseased cartilage or an area slightly larger than the area of diseased cartilage or substantially larger than the diseased cartilage. The shape or size can also be designed, selected or adapted to facilitate a minimally invasive surgical approach. The shape or size can further be designed, selected or adapted to improve the device's ergonomics. The area can encompass the entire articular surface or the weight bearing surface. The device may have a surface that engages a portion of a joint that is a partially or entirely non-articular surface portion.

Mechanical devices can be used for surgical assistance (e.g., surgical tools), for example using gels, molds, plastics or metal. One or more electronic images or intraoperative measurements can be obtained providing object coordinates that define the articular or bone surface and shape. These objects' coordinates can be utilized to either shape the device, e.g. using a CAD/CAM technique, to be adapted to a patient's anatomy or, alternatively, to select a typically pre-made device that has a good fit with a patient's anatomy. The device can have a surface and shape that will match all or at least a portion of the articular cartilage, subchondral bone or other bone surface and shape of the joint, e.g. similar to a substantial negative of the corresponding joint surface (e.g., an articular surface portion, a non-articular surface point or both). The device can include, without limitation, one or more guides such as cut planes, apertures, slots or holes to accommodate surgical instruments such as drills, reamers, curettes, k-wires, screws and saws.

The device may have a single component or multiple components. The components may be attached to the unoperated and operated portions of the intra- or extra-articular anatomy. For example, one component may be attached to the femoral neck, while another component may be in contact with the greater or lesser trochanter. Typically, the different components can be used to assist with different parts of the surgical procedure. When multiple components are used, one or more components may also be attached to a different component rather than the articular cartilage, subchondral bone or other areas of osseous or non-osseous anatomy.

Components may also be designed to fit to the joint after an operative step has been performed. For example, in a hip, one component may be used to perform an initial cut, for example through the femoral neck, while another subsequently used component may be designed to fit on/over the femoral neck after the cut, for example covering the area of the cut with a central opening for insertion of a reamer or the femoral neck resection guide as illustrated in FIG. 32C. Using this approach, subsequent surgical steps may also be performed with high accuracy, e.g. cutting the femoral neck to achieve a predetermined resection level or reaming of the marrow cavity.

In another embodiment, a guide may be attached to a mold to control the direction and orientation of surgical instruments. For example, after the femoral neck has been cut, a mold may be attached to the area of the cut, whereby it fits portions or all of the exposed bone surface. The mold may have an opening adapted for a reamer. Before the reamer is introduced a femoral reamer guide may be inserted into the mold and advanced into the marrow cavity. The position and orientation of the reamer guide may be determined by the femoral mold. The reamer can then be advanced over the reamer guide and the marrow cavity can be reamed with improved accuracy. Similar approaches are feasible in other joints.

All surgical tool components may be disposable. Alternatively, some components may be re-usable. In certain embodiments, one or more single use, disposable components in a surgical kit created for a particular patient may be patient-adapted, and certain single use, disposable components are standard and not adapted for the particular patient. In certain embodiments, reusable components are included in the surgical kit. Typically, these components applied after a surgical step such as a cut as been performed can be reusable, since a reproducible anatomical interface will have been established.

Interconnecting or bridging components may be used. For example, such interconnecting or bridging components may couple the mold attached to the joint with a standard, preferably unmodified or only minimally modified cut block used during hip surgery. Interconnecting or bridging components may be made of plastic or metal. When made of metal or other hard material, they can help protect the joint from plastic debris, for example when a reamer or saw would otherwise get into contact with the mold.

The accuracy of the attachment between the component or mold and the cartilage or subchondral bone or other osseous structures is typically better than 2 mm, more preferred better than 1 mm, more preferred better than 0.7 mm, more preferred better than 0.5 mm, or even more preferred better than 0.5 mm. The accuracy of the attachment between different components or between one or more molds and one or more surgical instruments is typically better than 2 mm, more preferred better than 1 mm, more preferred better than 0.7 mm, more preferred better than 0.5 mm, or even more preferred better than 0.5 mm.

The angular error of any attachments or between any components or between components, molds, instruments or the anatomical or biomechanical axes is preferably less than 2 degrees, more preferably less than 1.5 degrees, more preferably less than 1 degree, and even more preferably less than 0.5 degrees. The total angular error is preferably less than 2 degrees, more preferably less than 1.5 degrees, more preferably less than 1 degree, and even more preferably less than 0.5 degrees.

Typically, a position will be chosen that will result in an anatomically desirable cut plane, drill hole, or general instrument orientation for subsequent placement of an articular repair system or for facilitating placement of the articular repair system. Moreover, the device can be designed so that the depth of the drill, reamer or other surgical instrument can be controlled, e.g., the drill cannot go any deeper into the tissue than defined by the device, and the size of the hole in the block can be designed to essentially match the size of the implant. Information about other joints or axis and alignment information of a joint or extremity can be included when selecting the position of these slots or holes. Alternatively, the openings in the device can be made larger than needed to accommodate these instruments. The device can also be configured to conform to the articular shape. The guides (e.g., apertures, or openings) provided can be wide enough to allow for varying the position or angle of the surgical instrument, e.g., reamers, saws, drills, curettes and other surgical instruments. An instrument guide, typically comprised of a relatively hard material, can then be applied to the device. The device helps orient the instrument guide relative to the three-dimensional anatomy of the joint.

The mold may contact the entire articular surface. In various embodiments, the mold can be in contact with only a portion of the articular surface. Thus, the mold can be in contact, without limitation, with: 100% of the articular surface; 80% of the articular surface; 50% of the articular surface; 30% of the articular surface; 30% of the articular surface; 20% of the articular surface; or 10% or less of the articular surface. An advantage of a smaller surface contact area is a reduction in size of the mold thereby enabling cost efficient manufacturing and, more important, minimally invasive surgical techniques. The size of the mold and its surface contact areas have to be sufficient, however, to ensure accurate placement so that subsequent drilling and cutting can be performed with sufficient accuracy.

In various embodiments, the maximum diameter of the mold is less than 10 cm. In other embodiments, the maximum diameter of the mold may be less than: 8 cm; 5 cm; 4 cm; 3 cm; or even less than 2 cm.

The mold may be in contact with three or more surface points rather than an entire surface. These surface points may be on the articular surface or external to the articular surface. By using contact points rather than an entire surface or portions of the surface, the size of the mold may be reduced.

Reductions in the size of the mold can be used to enable minimally invasive surgery (MIS) in the hip, the knee, the shoulder and other joints. MIS technique with small molds will help to reduce intraoperative blood loss, preserve tissue including possibly bone, enable muscle sparing techniques and reduce postoperative pain and enable faster recovery. Thus, in one embodiment of the disclosure the mold is used in conjunction with a muscle sparing technique. In another embodiment of the disclosure, the mold may be used with a bone sparing technique. In another embodiment of the disclosure, the mold is shaped to enable MIS technique with an incision size of less than 15 cm, or, more preferred, less than 13 cm, or, more preferred, less than 10 cm, or, more preferred, less than 8 cm, or, more preferred, less than 6 cm.

The mold may be placed in contact with points or surfaces outside of the articular surface. For example, the mold can rest on the acetabular rim or the lesser or greater trochanter. Optionally, the mold may only rest on points or surfaces that are not articular surface or external to the articular surface. Furthermore, the mold may rest on points or surfaces within the weight-bearing surface, or on points or surfaces external to the weight-bearing surface.

The mold may be designed to rest on bone or cartilage outside the area to be worked on, e.g. cut, drilled etc. In this manner, multiple surgical steps can be performed using the same mold. For example, in the hip, the mold may be attached external to the acetabular fossa, providing a reproducible reference that is maintained during a procedure. The mold may be affixed to the underlying bone, for example with pins or drills etc.

In additional embodiments, the mold may rest on the articular cartilage. The mold may rest on the subchondral bone or on structures external to the articular surface that are within the joint space or on structures external to the joint space. If the mold is designed to rest on the cartilage, an imaging test demonstrating the articular cartilage can be used in one embodiment. This can, for example, include ultrasound, spiral CT arthrography, MRI using, for example, cartilage displaying pulse sequences, or MRI arthrography. In another embodiment, an imaging test demonstrating the subchondral bone, e.g. CT or spiral CT, can be used and a standard cartilage thickness can be added to the scan. The standard cartilage thickness can be derived, for example, using an anatomical reference database, age, gender, and race matching, age adjustments and any method known in the art or developed in the future for deriving estimates of cartilage thickness. The standard cartilage thickness may, in some embodiments, be uniform across one or more articular surfaces or it can change across the articular surface.

The mold may be adapted to rest substantially on subchondral bone. In this case, residual cartilage can create some offset and inaccurate result with resultant inaccuracy in surgical cuts, drilling and the like. In one embodiment, the residual cartilage is removed in a first step in areas where the mold is designed to contact the bone and the subchondral bone is exposed. In a second step, the mold is then placed on the subchondral bone.

With certain diseases such as advanced osteoarthritis, significant articular deformity can result. The articular surface(s) can become flattened. There can be cyst formation or osteophyte formation. "Tram track" like structures can form on the articular surface. In one embodiment of the disclosure, osteophytes or other deformities may be removed by the computer software prior to generation of the mold. The software can automatically, semi-automatically or manually with input from the user simulate surgical removal of the osteophytes or other deformities, and predict the resulting shape of the joint and the associated surfaces. The mold can then be designed based on the predicted shape. Intraoperatively, these osteophytes or other deformities can then also optionally be removed prior to placing the mold and performing the procedure. Alternatively, the mold can be designed to avoid such deformities. For example, the mold may only be in contact with points on the articular surface or external to the articular surface that are not affected or involved by osteophytes. The mold can rest on the articular surface or external to the articular surface on three or more points or small surfaces with the body of the mold elevated or detached from the articular surface so that the accuracy of its position cannot be affected by osteophytes or other articular deformities. The mold can rest on one or more anatomical features of the joint, e.g., an acetabular rim of a reamed or intact acetabulum of a hip joint. Alternatively, all or portions of the mold may be designed to rest on osteophytes or other excrescences or pathological changes.

The surgeon can, optionally, make fine adjustments between the alignment device and the instrument guide. In this manner, an optimal compromise can be found, for example, between biomechanical alignment and joint laxity or biomechanical alignment and joint function, e.g. in a hip joint anteversion, retroversion, abduction or adduction. By oversizing the openings in the alignment guide, the surgeon can utilize the instruments and insert them in the instrument guide without damaging the alignment guide. Thus, in particular if the alignment guide is made of plastic, debris will not be introduced into the joint. The position and orientation between the alignment guide and the instrument guide can be also be optimized with the use of, for example, interposed spacers, wedges, screws and other mechanical or electrical methods known in the art.

A surgeon may desire to influence joint laxity as well as joint alignment. This can be optimized for different flexion and extension, abduction, or adduction, internal and external rotation angles. For this purpose, for example, spacers can be introduced that are attached or that are in contact with one or more molds. The surgeon can intraoperatively evaluate the laxity or tightness of a joint using spacers with different thickness or one or more spacers with the same thickness. Ultimately, the surgeon will select an optimal combination of spacers for a given joint and mold. A surgical cut guide can be applied to the mold with the spacers optionally interposed between the mold and the cut guide. In this manner, the exact position of the surgical cuts can be influenced and can be adjusted to achieve an optimal result. Thus, the position of a mold can be optimized relative to the joint, bone or cartilage for soft-tissue tension, ligament balancing or for flexion, extension, rotation, abduction, adduction, anteversion, retroversion and other joint or bone positions and motion. The position of a cut block or other surgical instrument may be optimized relative to the mold for soft-tissue tension or for ligament balancing or for flexion, extension, rotation, abduction, adduction, anteversion, retroversion and other joint or bone positions and motion. Both the position of the mold and the position of other components including cut blocks and surgical instruments may be optimized for soft-tissue tension or for ligament balancing or for flexion, extension, rotation, abduction, adduction, anteversion, retroversion and other joint or bone positions and motion.

In some embodiments a template or trial may be used for assisting the surgical technique and guiding the placement and direction of surgical instruments. In addition, the templates can be utilized for guiding the placement of the implant or implant components. For example, in the hip joint, tilting of the acetabular component is a frequent problem with total hip arthroplasty. A template can be applied to the acetabular wall with an opening in the center large enough to accommodate the acetabular component that the surgeon intends to place. The template can have receptacles or notches that match the shape of small extensions that can be part of the implant or that can be applied to the implant. For example, the implant can have small members or extensions applied to the twelve o'clock and six o'clock positions. By aligning these members with notches or receptacles in the mold, the surgeon can ensure that the implant is inserted without tilting or rotation. These notches or receptacles can also be helpful to hold the implant in place while bone cement is hardening in cemented designs.

One or more templates can be used during the surgery. For example, in the hip, a template can be initially applied to the proximal femur that closely approximates the 3D anatomy prior to the resection of the femoral head. The template can include an opening to accommodate a saw. The opening is positioned to achieve an optimally placed surgical cut for subsequent reaming and placement of the prosthesis. A second template can then be applied to the proximal femur after the surgical cut has been made. The second template can be useful for guiding the direction of a reamer prior to placement of the prosthesis. As can be seen in this, as well as in other examples, templates can be made for joints prior to any surgical intervention. However, it is also possible to make templates that are designed to fit to a bone or portions of a joint after the surgeon has already performed selected surgical procedures, such as cutting, reaming, drilling, etc. The template can account for the shape of the bone or the joint resulting from these procedures. Exemplary surgical tools are disclosed in U.S. Patent Nos. 8,066,708 and 8,083,745.

In certain embodiments, the surgical assistance device comprises an array of adjustable, closely spaced pins (e.g., plurality of individually moveable mechanical elements). One or more electronic images or intraoperative measurements can be obtained providing object coordinates that define the articular or bone surface and shape. These objects' coordinates can be entered or transferred into the device, for example manually or electronically, and the information can be used to create a surface and shape that will match all or portions of the articular or bone surface and shape by moving one or more of the elements. The device can include slots and holes to accommodate surgical instruments such as drills, curettes, k-wires, screws and saws. The position of these slots and holes may be adjusted by moving one or more of the mechanical elements. Typically, a position will be chosen that will result in an anatomically desirable cut plane, reaming direction, or drill hole or instrument orientation for subsequent placement of an articular repair system or for facilitating the placement of an articular repair system.

Information about other joints or axis and alignment information of a joint or extremity can be included when selecting the position of the, without limitation, cut planes, apertures, slots or holes on the template, in accordance with an embodiment of the disclosure. The biomechanical or anatomical axes may be derived as described above.

In another embodiment, the biomechanical axis may be established using non-image based approaches including traditional surgical instruments and measurement tools such as intramedullary rods, alignment guides and also surgical navigation. For example, in a hip joint, optical or radiofrequency markers can be attached to the patient. The lower limb may then be rotated around the hip joint and the position of the markers can be recorded for different limb positions. The center of the rotation will determine the center of the femoral head. Similar reference points may be determined in the ankle joint etc. The position of the templates or, more typically, the position of surgical instruments relative to the templates may then be optimized for a given biomechanical load pattern, for example in abduction or adduction. Thus, by performing these measurements pre- or intraoperatively, the position of the surgical instruments may be optimized relative to the molds and the cuts can be placed to correct underlying axis errors such as varus or valgus malalignment or ante- or retroversion.

Upon imaging, a physical template of a hip joint is generated, in accordance with an embodiment herein. The template can be used to perform image guided surgical procedures such as partial or complete joint replacement or articular resurfacing. The template may include reference points or opening or apertures for surgical instruments such as drills, saws, burrs and the like.

In order to derive the preferred orientation of drill holes, cut planes, saw planes, reaming depth and diameter, depth and diameter of broaching and the like, openings or receptacles in said template or attachments will be adjusted to account for at least one axis. The axis can be anatomical or biomechanical, for example, for a knee joint, a hip joint, an ankle joint, a shoulder joint or an elbow joint.

In one embodiment, only a single axis is used for placing and optimizing such drill holes, saw planes, cut planes, and or other surgical interventions. This axis may be, for example, an anatomical or biomechanical axis. In a specific embodiment, a combination of axis or planes can be used for optimizing the placement of the drill holes, saw planes, cut planes or other surgical interventions. For example, two axes (e.g., one anatomical and one biomechanical) can be factored into the position, shape or orientation of the 3D guided template and related attachments or linkages. For example, two axes, (e.g., one anatomical and biomechanical) and one plane (e.g., the top plane defined by the tibial plateau), can be used. Alternatively, two or more planes can be used (e.g., a coronal and a sagittal plane), as defined by the image or by the patients anatomy.

Angle and distance measurements and surface topography measurements may be performed in these one or more, preferably two or more, preferably three or more multiple planes, as necessary. These angle measurements can, for example, yield information on varus or valgus deformity, flexion or extension deficit, hyper or hypo-flexion or hyper- or hypo-extension, abduction, adduction, internal or external rotation deficit, or hyper-or hypo-abduction, hyper- or hypo-adduction, hyper- or hypo- internal or external rotation.

Single or multi-axis line or plane measurements can then be utilized to determine preferred angles of correction, e.g., by adjusting surgical cut or saw planes or other surgical interventions. Typically, two axis corrections will be preferred over a single axis correction, a two plane correction will be preferred over a single plane correction and so forth.

In accordance with another embodiment of the disclosure, more than one drilling, cut, boring or reaming or other surgical intervention is performed for a particular treatment such as the placement of a joint resurfacing or replacing implant, or components thereof. These two or more surgical interventions (e.g., drilling, cutting, reaming, sawing) are made in relationship to a biomechanical axis, or an anatomical axis or an implant axis. The 3D guidance template or attachments or linkages thereto include one or more openings, guides, apertures or reference planes to make at least one or more drillings, reamings, borings, sawings or cuts in relationship to a biomechanical axis, an anatomical axis, an implant axis or other axis derived therefrom or related thereto.

While in simple embodiments it is possible that only a single cut or drilling will be made in relationship to a biomechanical axis, an anatomical axis, an implant axis or an axis related thereto, in most meaningful implementations, two or more drillings, borings, reamings, cutting or sawings will be performed or combinations thereof in relationship to a biomechanical, anatomical or implant axis.

For example, an initial cut may be placed in relationship to a biomechanical axis of particular joint. A subsequent drilling, cut or other intervention can be performed in relation to an anatomical axis. Both can be designed to achieve a correction in a biomechanical axis or anatomical axis. In another example, an initial cut can be performed in relationship to a biomechanical axis, while a subsequent cut is performed in relationship to an implant axis or an implant plane. Any combination in surgical interventions and in relating them to any combination of biomechanical, anatomical, implant axis or planes related thereto is possible. In many embodiments of the disclosure, it is desirable that a single cut or drilling be made in relationship to a biomechanical or anatomical axis. Subsequent cuts or drillings or other surgical interventions can then be made in reference to said first intervention. These subsequent interventions can be performed directly off the same 3D guidance template or they can be performed by attaching surgical instruments or linkages or reference frames or secondary or other templates to the first template or the cut plane or hole and the like created with the first template.

In another embodiment, a frame can be applied to the bone or the cartilage in areas other than the diseased bone or cartilage. The frame can include holders and guides for surgical instruments. The frame can be attached to one or preferably more previously defined anatomical reference points. Alternatively, the position of the frame can be cross-registered relative to one, or more, anatomical landmarks, using an imaging test or intraoperative measurement, for example one or more fluoroscopic images acquired intraoperatively. One or more electronic images or intraoperative measurements including using mechanical devices can be obtained providing object coordinates that define the articular or bone surface and shape. These objects' coordinates can be entered or transferred into the device, for example manually or electronically, and the information can be used to move one or more of the holders or guides for surgical instruments. Typically, a position will be chosen that will result in a surgically or anatomically desirable cut plane or drill hole orientation for subsequent placement of an articular repair system. Information about other joints or axis and alignment information of a joint or extremity can be included when selecting the position of these slots or holes.

Furthermore, re-useable tools (e.g., molds) can be also be created and employed. Nonlimiting examples of re-useable materials include putties and other deformable materials (e.g., an array of adjustable closely spaced pins that can be configured to match the topography of a joint surface). In other embodiments, the molds may be made using balloons. The balloons can optionally be filled with a hardening material. A surface can be created or can be incorporated in the balloon that allows for placement of a surgical cut guide, reaming guide, drill guide or placement of other surgical tools. The balloon or other deformable material can be shaped intraoperatively to conform to at least one articular surface. Other surfaces can be shaped in order to be parallel or perpendicular to anatomical or biomechanical axes. The anatomical or biomechanical axes can be found using an intraoperative imaging test or surgical tools commonly used for this purpose in hip, knee or other arthroplasties.

In various embodiments, the template may include a reference element, such as a pin, that upon positioning of the template on the articular surface, establishes a reference plane relative to a biomechanical axis or an anatomical axis or plane of a limb. In other embodiments, the reference element may establish an axis that subsequently be used a surgical tool to correct an axis deformity.

In these embodiments, the template can be created directly from the joint during surgery or, alternatively, created from an image of the joint, for example, using one or more computer programs to determine object coordinates defining the surface contour of the joint and transferring (e.g., dialing-in) these co-ordinates to the tool. Subsequently, the tool can be aligned accurately over the joint and, accordingly, the surgical instrument guide or the implant will be more accurately placed in or over the articular surface.

In both single-use and re-useable embodiments, the tool can be designed so that the instrument controls the depth or direction of the drill, i.e., the drill cannot go any deeper into the tissue than the instrument allows, and the size of the hole or aperture in the instrument can be designed to essentially match the size of the implant.

In certain embodiments, a surgical tool includes a reamer for preparing an implantation site in a patient's acetabulum. The reamer can be standard and not adapted to any individual patient. Alternatively, the reamer can be adapted to particular patient, e.g., configured to create a site on the patient's acetabulum to receive a patient-adapted acetabular implant (e.g., an acetabular cup with an insert, the cup having a patient-adapted rim). A patient-adapted, single use, disposable reamer can be manufactured according to the manufacturing methods described herein.

In certain embodiments, a surgical tool includes a broach for preparing an implantation site in a patient's femur. The broach can be standard and not adapted to any individual patient. Alternatively, the broach can be adapted to particular patient, e.g., configured to create a site on the patient's acetabulum to receive a patient-adapted femoral implant (e.g., a femoral stem with an integrated femoral head and neck or a modular femoral head and neck components). A patient-adapted, single use, disposable broach can be manufactured according to the manufacturing methods described herein.

The implant site can be prepared with use of a robotic device. The robotic device can use information from an electronic image for preparing the recipient site.

Identification and preparation of the implant site and insertion of the implant can be supported by a surgical navigation system. In such a system, the position or orientation of a surgical instrument with respect to the patient's anatomy can be tracked in real-time in one or more 2D or 3D images. These 2D or 3D images can be calculated from images that were acquired preoperatively, such as MR or CT images. Non-image based surgical navigation systems that find axes or anatomical structures, for example with use of joint motion, can also be used. The position and orientation of the surgical instrument as well as the mold including alignment guides, surgical instrument guides, reaming guides, drill guides, saw guides, etc. can be determined from markers attached to these devices. These markers can be located by a detector using, for example, optical, acoustical or electromagnetic signals.

Identification and preparation of the implant site and insertion of the implant can also be supported with use of a C-arm system. The C-arm system can afford imaging of the joint in one or, preferably, multiple planes. The multiplanar imaging capability can aid in defining the shape of an articular surface. This information can be used to selected an implant with a good fit to the articular surface. Currently available C-arm systems also afford cross-sectional imaging capability, for example for identification and preparation of the implant site and insertion of the implant. C-arm imaging can be combined with administration of radiographic contrast.

## EXAMPLES

The following examples illustrate various embodiments of designing or selecting a patient-adapted hip replacement or resurfacing system. Any of the embodiments herein are applicable to cemented and non-cemented hip replacement or resurfacing systems. While certain embodiments are described with a number of sequential steps, the same or similar steps can vary in sequence to achieve the same or substantially the same outcome. The steps between different illustrative embodiments are also exchangeable, e.g., to meet the design or selection criteria of a particular patient-adapted hip replacement system. Further, the designing or selecting process can be iterative, that is, one or more steps described herein can be repeated.

As described herein, various designing, determining and selecting steps are carried out with patient-specific image data and optionally additional patient information (e.g., the patient's body habitus). The patient's body habitus includes one or more physical and constitutional characteristics of an individual, such as for example, the patient's weight, height, bone density, and soft tissue thickness).

**Example 1. Designing or Selecting a Hip Replacement System (with a Short or Long Femoral Stem)**

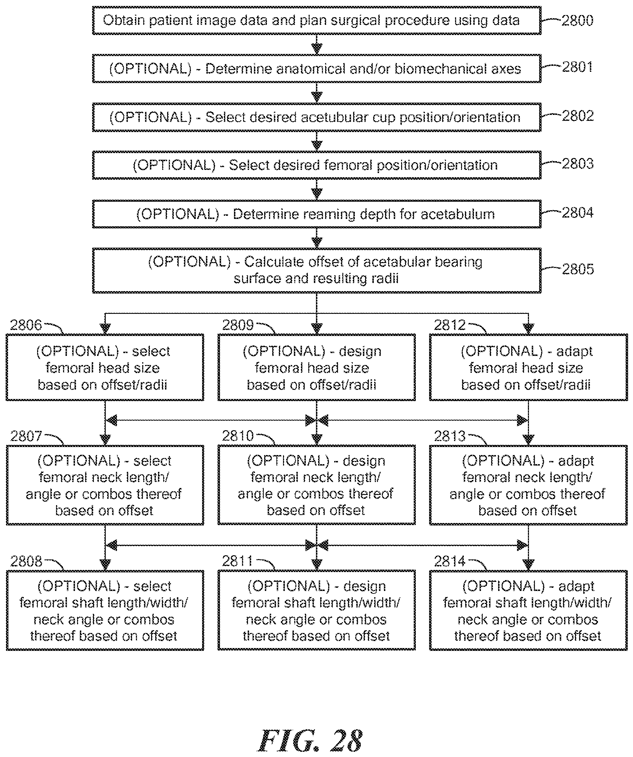

An exemplary process, shown in FIG. 28, begins with obtaining image data of a patient's hip joint(s) (step 2800). Image data of both hip joints of the patient is obtained. For a patient in need of unilateral hip replacement, image data of the hip joint to be replaced is obtained and optionally image data of the contralateral side is also obtained. Various imaging modalities and techniques are described herein. Image data includes data from two-dimensional cross-sectional images. Alternatively or additionally, image data includes data from three-dimensional images.

The image data is collected from images through acetabulum and proximal femur of the patient's hip joint(s). Optionally, images through the patient's corresponding knee joint(s) are also obtained. Further, images through the patient's corresponding ankle joint(s) may also be obtained. Image data of the knee/ankle joints may help optimize the hip replacement system, e.g., by optimizing leg length for the patient.

Step 2800 may also include planning the surgical procedure with the image data and optionally other data, such as for example, additional patient information (e.g., the patient's body habitus). Optionally, the surgical planning includes step 2801 of determining one or more axes of the hip joint to be replaced, such as for example, an anatomical axis of the femur of the hip joint, a biomechanical axis of the femur of the hip joint, an anatomical axis of the acetabulum of the hip joint, a biomechanical axis of the acetabulum of the hip joint.

Optionally, the surgical planning includes step 2802 of determining or selecting a desired acetabular cup position or orientation, such as for example, anteversion. Optionally, the surgical planning includes designing or selecting a desired acetabular cup size, shape or geometry, e.g., the rim of the acetabular cup matching the patient's acetabulum rim (preferably after virtually reamed to a desired depth).

Optionally, the surgical planning includes step 2803 of determining or selecting a desired femoral implant or implant component position or orientation, such as for example, anteversion, the femoral shaft angle the femoral neck angle. The desired femoral implant or implant component position/orientation can be determined in connection with the acetabular cup position/orientation, as described herein.

Optionally, the surgical planning includes step 2804 of determining a desired reaming depth for the acetabulum. The surgical planning may further include virtual reaming of the acetabulum and optionally after virtual removal of one or more deformities, e.g., osteophytes. Alternatively, instead of virtual removal of the one or more deformities, the implant components can be designed or selected by omitting the one or more deformities in the image data.

Optionally, the surgical planning includes step 2805 of calculating the offset of the acetabular bearing surface and resultant radii by estimating the ream depth for the acetabulum and taking into account the added acetabular implant or implant component thickness.

Optionally, the surgical planning includes step 2806, step 2809 or step 28012, including determining or selecting a femoral head size (e.g. outer diameter) based on the offset of acetabular bearing surface and resultant radii. For example, a larger offset of the acetabular bearing surface, as compared to a smaller offset, requires a smaller femoral head.

Optionally, the surgical planning includes steps 2807, step 2810 or step 2813, including determining or selecting a femoral neck length or angle or both. Such determination or selection references the patient's anatomy, e.g., based on the patient's image data, and optionally other patient information. Optionally, such determination or selection is based on the offset of the acetabular bearing surface.

Optionally, the surgical planning includes determining or selecting one or more parameters, e.g., step 2808, step 2811 or step 2814, including determining or selecting a femoral shaft length, a femoral shaft width, the angle between the femoral neck and shaft. Such determination or selection references the patient's anatomy, e.g., based on the patient's image data, and optionally other patient information.

Optionally, femoral neck length or angle or combinations thereof can be selected, designed, adapted/optimized (step 2807, 2810 or 2813) based on the offset of the acetabular bearing surface calculated according to step 2805.

Optionally, femoral shaft including its length, width or neck shaft angle or combinations thereof can be selected, designed, or adapted/optimized (step 2808, 2811 or 2814) based on patient-specific parameters (e.g., obtained by the methods described herein including, e.g., step 2800 and optionally step 2801).

Optionally, femoral head size can be selected, designed or adapted/optimized with patient-specific parameters (step 2806, 2809 or 2812) and based on the offset of the acetabular bearing surface and the resultant radii calculated according to step 2805.

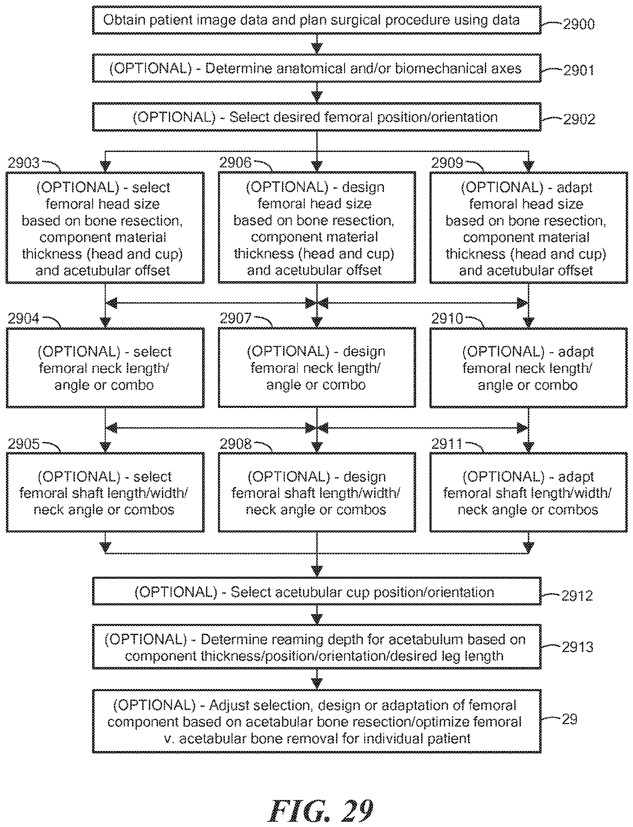

As described above, the hip replacement system is designed or adapted by determining a desired reaming depth of the acetabulum, followed by determining the offset of acetabular bearing surface and subsequently, determining or selecting a femoral head size (e.g. outer diameter), femoral neck (e.g., angle), or femoral shaft (e.g., length or angle) based on the offset of acetabular bearing surface and resultant radii. Alternatively, the hip replacement system can be designed or selected by first determining or selecting one or more of a desired femoral neck and shaft (e.g., size and angle) and femoral head size (e.g., outer diameter), followed by determining or selecting the offset of acetabular bearing surface and subsequently, determining the desired reaming depth of the acetabulum. That alternative process is illustrated in FIG. 29 and may include various combinations of steps selected from steps 2900-2914. To reiterate, the method steps described herein can be carried out in the sequence as described or vary in sequence; the method steps are also exchangeable or repeated among different embodiments where necessary or desired.

**Example 2. Designing or Selecting a Patient-Adapted Hip Resurface System**

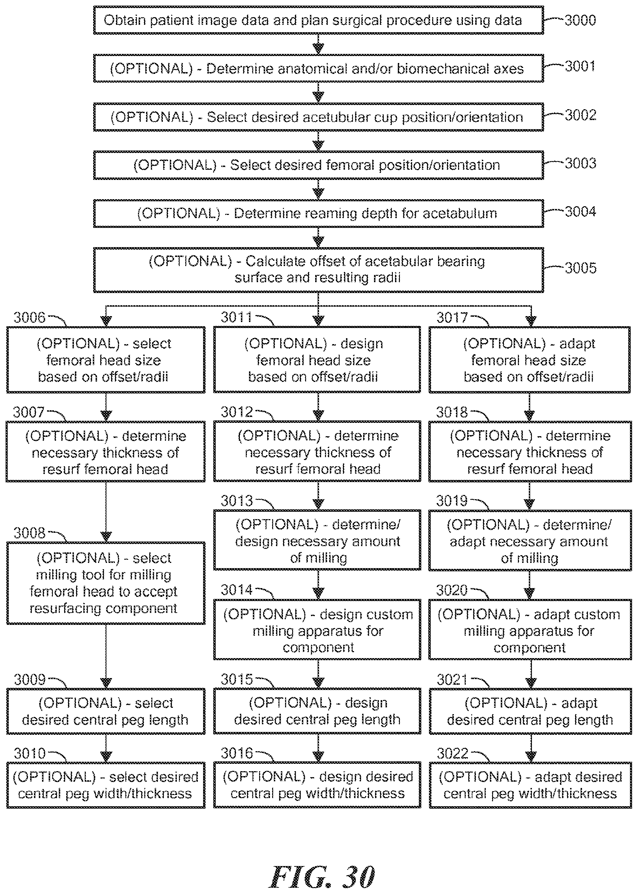

An exemplary process, shown in FIG. 30, begins with obtaining image data of a patient's hip joint(s) (step 3000). Image data of both hip joints of the patient is obtained. For a patient in need of unilateral hip resurfacing, image data of the hip joint to be resurfaced is obtained and optionally image data of the contralateral side is also obtained. Various imaging modalities and techniques are described herein. Image data includes data from two-dimensional cross-sectional images. Alternatively or additionally, image data includes data from three-dimensional images.

The image data is collected from images through acetabulum and proximal femur of the patient's hip joint(s). Optionally, images through the patient's corresponding knee joint(s) are also obtained. Further, images through the patient's corresponding ankle joint(s) may also be obtained. Image data of the knee/ankle joints may help optimize the hip implant system, e.g., by optimizing leg length for the patient.

The surgical procedure is then planned with the image data and optionally additional patient information or patient-specific data/parameters (e.g., the patient's body habitus) (step 3000). The patient's body habitus includes one or more physical and constitutional characteristics of an individual, such as for example, the patient's weight, height, bone density, and soft tissue characteristics such as thickness).

Optionally, the surgical planning includes step 3001 of determining one or more axes of the hip joint to be resurfaced or replaced, such as for example, an anatomical axis of the femur of the hip joint, a biomechanical axis of the femur of the hip joint, an anatomical axis of the acetabulum of the hip joint, a biomechanical axis of the acetabulum of the hip joint.

Optionally, the surgical planning includes step 3002 of determining or selecting a desired acetabular cup position or orientation, such as for example, anteversion. Optionally, the surgical planning includes designing or selecting a desired acetabular cup size, shape or geometry, e.g., the rim of the acetabular cup matching the patient's acetabulum rim (preferably after virtually reamed to a desired depth). Such determination or selection references the patient's anatomy, e.g., based on the patient's image data, and optionally other patient information.

Optionally, the surgical planning includes step 3003 of determining or selecting a desired femoral implant or implant component position or orientation, such as for example, anteversion, the femoral shaft angle the femoral neck angle. The desired femoral implant or implant component position/orientation can be determined in connection with the acetabular cup position/orientation, as described herein.

Optionally, the surgical planning includes step 3004 of determining a desired reaming depth for the acetabulum. The surgical planning may further include virtual reaming of the acetabulum and optionally after virtual removal of one or more deformities, e.g., osteophytes. Alternatively, instead of virtual removal of the one or more deformities, the implant components can be designed or selected by omitting the one or more deformities in the image data.

Optionally, the surgical planning includes step 3005 of calculating the offset of the acetabular bearing surface and resultant radii by estimating the ream depth for the acetabulum and taking into account the added acetabular implant or implant component thickness.

Optionally, the surgical planning includes step 3006, step 3011 or step 3017, including determining or selecting a femoral head size (e.g. outer diameter) based on the offset of acetabular bearing surface and resultant radii. For example, a larger offset of the acetabular bearing surface, as compared to a smaller offset, requires a smaller resurfacing femoral head.

The surgical planning may also include step 3007 or 3012 or 3018 of determining or selecting a necessary material thickness of the resurfacing femoral head component. Such material thickness can be predetermined without reference to the patient's hip joint anatomy. Alternatively, such material thickness can be determined or selected based on the patient's hip joint anatomy.

The surgical planning also includes step 3009, step 3015 or step 3021, including determining or selecting a desired central peg length of the femoral implant or implant component. The central peg length can be designed for the individual patient or selected from a library of premade femoral head components with varying central peg lengths (e.g., step 3010, 3016 or 3022). The central peg length of the selected, premade femoral head component can be further adapted (e.g., by adding or removing materials with CNC machining or laser melting) to the individual patient.

The surgical planning further includes step 3008, steps 3013 and 3014, or steps 3019 and 3020, including designing or selecting one or more surgical tool(s) for preparing the femoral head of the hip joint to be resurfaced or replaced in order to receive the resurfacing femoral head component. For example, one or more milling or broaching tool(s) can be designed or selected, and as described above, a larger offset of the acetabular bearing surface requires more milling or broaching (more bone removal) of the femoral head in order to receive a smaller resurfacing femoral head component. The resurfacing femoral head component can be designed for the individual patient or selected from a library of premade femoral head components. The selected, premade femoral head component can be further adapted (e.g., by adding or removing materials with CNC machining or laser melting) to the individual patient.

As described herein, the size of a femoral head can be designed or adapted based on the offset of the acetabular bearing surface and the resultant radii. The necessary material thickness of the resurfacing femoral head component can be adapted based on the patient's anatomy (e.g., using patient's image data) or additional patient information. Alternatively, the necessary material thickness of the resurfacing femoral head component is predetermined. Optionally, various predetermined material thicknesses are available, and a predetermined thickness is selected based on the patient's anatomy or additional patient information.

As described herein, the necessary amount of bone removal, e.g., milling or broaching of the femoral head or reaming of the acetabulum can be patient-adapted, e.g., determined or designed with reference to the patient's anatomy. The surgical tools for milling, broaching or reaming can be customized and optimized for the individual patient.

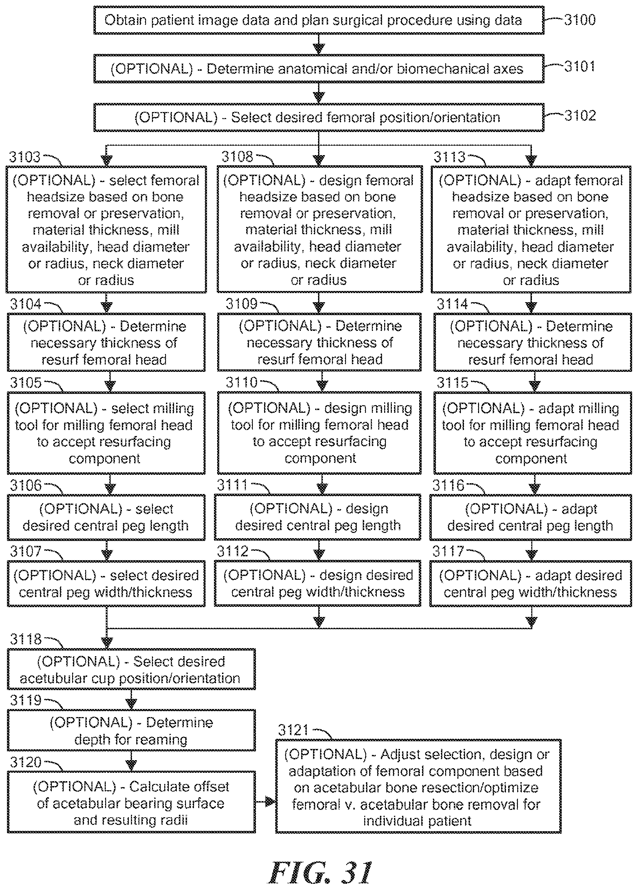

As described above, the hip implant system is designed or selected by determining a desired reaming depth of the acetabulum, followed by determining the offset of acetabular bearing surface and subsequently, determining or selecting a femoral head size (e.g. outer diameter) based on the offset of acetabular bearing surface and resultant radii. Alternatively, the hip implant system can be designed or selected by determining a desired femoral head size (e.g., outer diameter), followed by determining or selecting the offset of acetabular bearing surface and subsequently, determining the desired reaming depth of the acetabulum. FIG. 31 illustrates the alterative process which may include various combinations of steps selected from steps 3100-3121. To reiterate, the method steps described herein can be carried out in the sequence as described or vary in sequence; the method steps are also exchangeable or repeated among different embodiments where necessary or desired.

It will be apparent to those skilled in the art that various modifications and variations can be made to the disclosed devices and methods. Other embodiments will be apparent to those skilled in the art from consideration of the specification and practice of the disclosed embodiments. It is intended that the specification and examples be considered as exemplary only, with a true scope being indicated by the following claims and their equivalents.

## Claims

1. A method of making a patient-adapted acetabular implant component (232) for treatment of a hip joint of a patient, the hip joint including an acetabular cavity, the implant component comprising:

    an acetabular cup component (312),
    wherein the acetabular cup component comprises:

    a cup portion (315) having a substantially hemispherical shape and an attachment mechanism; and a rim portion (316) having a complementary attachment mechanism configured to attach the rim portion to the cup portion (312), wherein the rim side (332) matches at least a portion of the rim of the acetabular cavity (314), wherein the method comprises choosing a cup portion (315) which is a standard, non-patient-specific size,
    wherein
    the method comprises making at least one feature of the acetabular cup component (312) patient-adapted, wherein the at least one patient-adapted feature is derived from image data of at least a portion of the patient's hip joint, wherein the at least one patient-adapted feature of the acetabular cup component (312) is a feature selected from the group consisting of: a locking mechanism; one or more screw holes; a radius of the acetabular cup component; and combinations thereof.

2. The method of claim 1, wherein the locking mechanism of the acetabular cup component (312) is configured in a patient-adapted, predetermined position and/or orientation for locking an insert component (234) in the acetabular cup component (312).

3. The method of claim 1, wherein the locking mechanism of the acetabular cup component (312) is configured in a patient-adapted, predetermined position and/or orientation for locking an insert component (234) in the acetabular cup component (312), and wherein the predetermined position and/or orientation of the locking mechanism is configured to allow the insert component (234) to face a direction that would maximize range of motion of a stem neck, when the acetabular implant component (232) is implanted in the acetabular cavity.

4. The method of claim 1, wherein the patient-adapted acetabular implant component (232) further comprises an insert component (234) with a locking mechanism that is configured to engage the locking mechanism of the acetabular cup component (312) and thereby fix the insert component (234) with respect to the cup component (312) in a patient- adapted, predetermined position and/or orientation.

5. The method of claim 1, wherein the patient-adapted acetabular implant component (232) further comprises an insert component (234) with a locking mechanism that is configured to engage the locking mechanism of the acetabular cup component (312) and thereby fix the insert component (234) with respect to the cup component (312) in a patient- adapted, predetermined position and/or orientation, and wherein the insert component (232) is not patient-specific.

6. The method of claim 1, wherein the patient-adapted acetabular implant component (232) further comprises an insert component (234) with a locking mechanism that is configured to engage the locking mechanism of the acetabular cup component (312) and thereby fix the insert component (234) with respect to the cup component (312) in a patient- adapted, predetermined position and/or orientation, and wherein the insert component (232) is a standard, zero degree insert.

7. The method of claim 1, wherein the patient-adapted acetabular implant component (232) further comprises one or more markings (319) on at least a portion of the rim of the cup component (312) and/or on at least a portion of an outer surface of the cup component (312).

8. The method of claim 1, wherein the patient-adapted acetabular implant component (232) further comprises one or more markings (319) on at least a portion of the rim of the cup component (312) and/or on at least a portion of an outer surface of the cup component (312), and wherein the one or more markings (319) have a predetermined position and/or orientation relative to at least a portion of a rim of the acetabular cavity (314).

9. The method of claim 1, wherein the acetabular implant component (232) is an acetabular trial implant component.

10. A method of making a patient-adapted acetabular implant system for treatment of a hip joint of a patient, the hip joint including an acetabular cavity, the method comprising:

   performing the method of claim 1 to make an acetabular implant component (232); and
   performing the method of claim 9 to make a trial implant component.

11. The method of claim 10,
   wherein the acetabular cup trial component (317) includes one or more markings (319) on at least a portion of a rim and/or an outer surface of the acetabular cup trial component (317) having a predetermined position and/or orientation relative to at least a portion of a rim of the acetabular cavity (314), when the cup trial component (317) is positioned and oriented within the acetabular cavity such that at least a portion of the rim of the acetabular cup trial component (317) is in substantially matching alignment with at least a portion of the rim of the acetabular cavity (314), and wherein the acetabular cup component (312) includes one or more markings (319) on at least a portion of a rim and/or an outer surface of the acetabular cup component (312) having predetermined positions and/or orientations substantially matching corresponding markings on at least a portion of a rim and/or an outer surface of the acetabular cup trial component (317).

12. The method of claim 10, wherein the method further comprises
   making a patient-adapted neck resection guide.


**Patentansprüche**

1. Ein Verfahren zur Herstellung einer patientenangepassten acetabularen Implantatkomponente (232) zur Behandlung eines Hüftgelenks eines Patienten, wobei das Hüftgelenk einen acetabularen Hohlraum aufweist, wobei die Implantatkomponente umfasst:

   eine Hüftpfannenkomponente (312),
   wobei die Hüftpfannenkomponente umfasst:

   einen Pfannenabschnitt (315) mit einer im Wesentlichen halbkugelförmigen Form und einem Befestigungsmechanismus; und einen Randabschnitt (316) mit einem komplementären Befestigungsmechanismus, der so konfiguriert ist, dass er den Randabschnitt an dem Pfannenabschnitt (312) befestigt, wobei die Randseite (332) mit mindestens einem Teil des Randes des Hüftgelenkspfannenhohlraumes (314) übereinstimmt, wobei das Verfahren das Auswählen eines Pfannenabschnitts (315) umfasst, der eine standardmäßige, nicht patientenspezifische Größe hat,

wobei das Verfahren das Anpassen mindestens eines Merkmals der Hüftpfannenkomponente (312) an den Patienten umfasst, wobei das mindestens eine patientenangepasste Merkmal aus Bilddaten von mindestens einem Teil des Hüftgelenks des Patienten abgeleitet ist, wobei das mindestens eine patientenangepasste Merkmal der Hüftpfannenkomponente (312) ein Merkmal ist, das aus der Gruppe ausgewählt wird, bestehend aus: einem Verriegelungsmechanismus; einem oder mehreren Schraubenlöchern; einem Radius der Hüftpfannenkomponente; und Kombinationen davon.

2. Das Verfahren nach Anspruch 1, wobei der Verriegelungsmechanismus der Hüftpfannenkomponente (312) in einer patientenangepassten, vorbestimmten Position und/oder Ausrichtung zum Verriegeln einer Einsatzkomponente (234) in der Hüftpfannenkomponente (312) konfiguriert ist.

3. Das Verfahren nach Anspruch 1, wobei der Verriegelungsmechanismus der Hüftpfannenkomponente (312) in einer patientenangepassten, vorbestimmten Position und/oder Ausrichtung konfiguriert ist, um eine Einsatzkomponente (234) in der Hüftpfannenkomponente (312) zu verriegeln, und wobei die vorbestimmte Position und/oder Ausrichtung des Verriegelungsmechanismus so konfiguriert ist, dass die Einsatzkomponente (234) in eine Richtung weisen kann, die den Bewegungsbereich eines Stielhalses maximieren würde, wenn die acetabulare Implantatkomponente (232) in den acetabularen Hohlraum implantiert wird.

4. Das Verfahren nach Anspruch 1, wobei die patientenangepasste acetabulare Implantatkomponente (232) ferner eine Einsatzkomponente (234) mit einem Verriegelungsmechanismus umfasst, der so konfiguriert ist, dass er in den Verriegelungsmechanismus der Hüftgelenkspfannenkomponente (312) eingreift und dadurch die Einsatzkomponente (234) in Bezug auf die Pfannenkomponente (312) in einer patientenangepassten, vorgegebenen Position und/oder Ausrichtung fixiert.

5. Das Verfahren nach Anspruch 1, wobei die patientenangepasste acetabulare Implantatkomponente (232) ferner eine Einsatzkomponente (234) mit einem Verriegelungsmechanismus umfasst, der so konfiguriert ist, dass er in den Verriegelungsmechanismus der Hüftgelenkpfannenkomponente (312) eingreift und dadurch die Einsatzkomponente (234) in Bezug auf die Pfannenkomponente (312) in einer patientenangepassten, vorbestimmten Position und/oder Ausrichtung fixiert, und wobei die Einsatzkomponente (232) nicht patientenspezifisch ist.

6. Das Verfahren nach Anspruch 1, wobei die patientenangepasste acetabulare Implantatkomponente (232) ferner eine Einsatzkomponente (234) mit einem Verriegelungsmechanismus umfasst, der so konfiguriert ist, dass er in den Verriegelungsmechanismus der Hüftgelenkspfannenkomponente (312) eingreift und dadurch die Einsatzkomponente (234) in Bezug auf die Pfannenkomponente (312) in einer patientenangepassten, vorbestimmten Position und/oder Ausrichtung fixiert, und wobei die Einsatzkomponente (232) ein Standard-Null-Grad-Einsatz ist.

7. Das Verfahren nach Anspruch 1, wobei die patientenangepasste acetabulare Implantatkomponente (232) ferner eine oder mehrere Markierungen (319) auf mindestens einem Teil des Randes der Pfannenkomponente (312) und/oder auf mindestens einem Teil einer Außenfläche der Pfannenkomponente (312) aufweist.

8. Das Verfahren nach Anspruch 1, wobei die patientenangepasste acetabulare Implantatkomponente (232) ferner eine oder mehrere Markierungen (319) auf mindestens einem Teil des Randes der Pfannenkomponente (312) und/oder auf mindestens einem Teil einer Außenfläche der Pfannenkomponente (312) aufweist, und wobei die eine oder die mehreren Markierungen (319) eine vorbestimmte Position und/oder Ausrichtung relativ zu mindestens einem Teil eines Randes des acetabularen Hohlraumes (314) aufweisen.

9. Das Verfahren nach Anspruch 1, wobei die acetabulare Implantatkomponente (232) eine acetabulare Probeimplantatkomponente ist.

10. Ein Verfahren zur Herstellung eines patientenangepassten acetabularen Implantatsystems zur Behandlung eines Hüftgelenks eines Patienten, wobei das Hüftgelenk einen acetabularen Hohlraum aufweist, wobei das Verfahren umfasst:

Durchführen des Verfahrens nach Anspruch 1, um eine acetabulare Implantatkomponente (232) herzustellen; und
Durchführen des Verfahrens nach Anspruch 9, um eine Probeimplantatkomponente herzustellen.

11. Das Verfahren nach Anspruch 10,

wobei die Hüftpfannen-Probekomponente (317) eine oder mehrere Markierungen (319) auf mindestens einem Teil eines Randes und/oder einer Außenfläche der Hüftpfannen-Probekomponente (317) aufweist, die eine vorbestimmte Position und/oder Ausrichtung relativ zu mindestens einem Teil eines Randes des acetabularen Hohlraumes (314) haben, wenn die Pfannen-Probekomponente (317) innerhalb des acetabularen Hohlraumes positioniert und ausgerichtet ist, so dass mindestens ein Teil des Randes der Hüftgelenkspfannen-Probekomponente (317) in im Wesentlichen übereinstimmender Ausrichtung mit mindestens einem Teil des Randes des acetabularen Hohlraumes (314) ist, und

wobei die Hüftpfannenkomponente (312) eine oder mehrere Markierungen (319) auf mindestens einem Abschnitt eines Randes und/oder einer Außenfläche der Hüftpfannenkomponente (312) mit vorbestimmten Positionen und/oder Ausrichtungen aufweist, die im Wesentlichen mit entsprechenden Markierungen auf mindestens einem Abschnitt eines Randes und/oder einer Außenfläche der Hüftpfannen-Probekomponente (317) übereinstimmen.

12. Das Verfahren nach Anspruch 10, wobei das Verfahren ferner das Herstellen einer patientenangepassten Halsresektionsführung umfasst.


## Revendications

1. Procédé de fabrication d'un composant de prothèse acétabulaire adapté au patient (232) pour un traitement d'une articulation de hanche d'un patient, l'articulation de hanche comportant une cavité acétabulaire, le composant de prothèse comprenant :

   un composant de cupule acétabulaire (312),
   dans lequel le composant de cupule acétabulaire comprend :

   une partie de cupule (315) ayant une forme sensiblement hémisphérique et un mécanisme de fixation ; et
   une partie de rebord (316) ayant un mécanisme de fixation complémentaire configuré pour fixer la partie de rebord à la partie de cupule (312), dans lequel le côté de rebord (332) coïncide avec au moins une partie du rebord de la cavité acétabulaire (314), dans lequel le procédé comprend le choix d'une partie de cupule (315) qui est d'une taille standard, non spécifique au patient,
   dans lequel le procédé comprend la fabrication d'au moins une caractéristique du composant de cupule acétabulaire (312) adaptée au patient, dans lequel l'au moins une caractéristique adaptée au patient est dérivée de données d'image d'au moins une partie de l'articulation de hanche du patient, dans lequel l'au moins une caractéristique adaptée au patient du composant de cupule acétabulaire (312) est une caractéristique choisie dans le groupe comprenant : un mécanisme de verrouillage ; un ou plusieurs trous de vis ; un rayon du composant de cupule acétabulaire ; et des combinaisons de ceux-ci.

2. Procédé selon la revendication 1, dans lequel le mécanisme de verrouillage du composant de cupule acétabulaire (312) est configuré dans une position et/ou une orientation prédéterminées, adaptées au patient pour verrouiller un composant d'insert (234) dans le composant de cupule acétabulaire (312).

3. Procédé selon la revendication 1, dans lequel le mécanisme de verrouillage du composant de cupule acétabulaire (312) est configuré dans une position et/ou une orientation prédéterminées, adaptées au patient pour verrouiller un composant d'insert (234) dans le composant de cupule acétabulaire (312), et dans lequel la position et/ou l'orientation prédéterminées du mécanisme de verrouillage sont configurées pour permettre au composant d'insert (234) de faire face à une direction qui maximiserait une plage de mouvement d'un col de tige, lorsque le composant de prothèse acétabulaire (232) est implanté dans la cavité acétabulaire.

4. Procédé selon la revendication 1, dans lequel le composant de prothèse acétabulaire adapté au patient (232) comprend en outre un composant d'insert (234) avec un mécanisme de verrouillage qui est configuré pour mettre en prise le mécanisme de verrouillage du composant de cupule acétabulaire (312) et attacher ainsi le composant d'insert (234) par rapport au composant de cupule (312) dans une position et/ou une orientation prédéterminées, adaptées au patient.

5. Procédé selon la revendication 1, dans lequel le composant de prothèse acétabulaire adapté au patient (232) comprend en outre un composant d'insert (234) avec un mécanisme de verrouillage qui est configuré pour mettre en prise le mécanisme de verrouillage du composant de cupule acétabulaire (312) et attacher ainsi le composant d'insert (234) par rapport au composant de cupule (312) dans une position et/ou une orientation prédéterminées, adaptées au patient, et dans lequel le composant d'insert (232) n'est pas spécifique au patient.

6. Procédé selon la revendication 1, dans lequel le composant de prothèse acétabulaire adapté au patient (232) comprend en outre un composant d'insert (234) avec un mécanisme de verrouillage qui est configuré pour mettre en prise le mécanisme de verrouillage du composant de cupule acétabulaire (312) et attacher ainsi le composant d'insert (234) par rapport au composant de cupule (312) dans une position et/ou une orientation prédéterminées, adaptées au patient, et dans lequel le composant d'insert (232) est un insert standard, de type zéro degré.

7. Procédé selon la revendication 1, dans lequel le composant de prothèse acétabulaire adapté au patient (232) comprend en outre un ou plusieurs marquages (319) sur au moins une partie du rebord du composant de cupule (312) et/ou sur au moins une partie d'une surface extérieure du composant de cupule (312).

8. Procédé selon la revendication 1, dans lequel le composant de prothèse acétabulaire adapté au patient (232) comprend en outre un ou plusieurs marquages (319) sur au moins une partie du rebord du composant de cupule (312) et/ou sur au moins une partie d'une surface extérieure du composant de cupule (312), et dans lequel les un ou plusieurs marquages (319) ont une position et/ou une orientation prédéterminées par rapport à au moins une partie d'un rebord de la cavité acétabulaire (314).

9. Procédé selon la revendication 1, dans lequel le composant de prothèse acétabulaire (232) est un composant de prothèse d'essai acétabulaire.

10. Procédé de fabrication d'un système de prothèse acétabulaire adapté au patient pour un traitement d'une articulation de hanche d'un patient, l'articulation de hanche comportant une cavité acétabulaire, le procédé comprenant :

l'exécution du procédé selon la revendication 1 pour fabriquer un composant de prothèse acétabulaire (232) ; et l'exécution du procédé selon la revendication 9 pour fabriquer un composant de prothèse d'essai.

11. Procédé selon la revendication 10,
dans lequel le composant d'essai de cupule acétabulaire (317) comporte un ou plusieurs marquages (319) sur au moins une partie d'un rebord et/ou d'une surface extérieure du composant d'essai de cupule acétabulaire (317) ayant une position et/ou une orientation prédéterminées par rapport à au moins une partie d'un rebord de la cavité acétabulaire (314), lorsque le composant d'essai de cupule (317) est positionné et orienté à l'intérieur de la cavité acétabulaire de telle sorte qu'au moins une partie du rebord du composant d'essai de cupule acétabulaire (317) est en alignement sensiblement coïncidant avec au moins une partie du rebord de la cavité acétabulaire (314), et dans lequel le composant de cupule acétabulaire (312) comporte un ou plusieurs marquages (319) sur au moins une partie d'un rebord et/ou d'une surface extérieure du composant de cupule acétabulaire (312) ayant des positions et/ou des orientations prédéterminées qui coïncident sensiblement avec des marquages correspondants sur au moins une partie d'un rebord et/ou d'une surface extérieure du composant d'essai de cupule acétabulaire (317).

12. Procédé selon la revendication 10, dans lequel le procédé comprend en outre la fabrication d'un guide de résection de col adapté au patient.

FIG. 2

FIG. 3

FIG. 4

**FIG. 9**

**FIG. 10**

**FIG. 11**

**FIG. 12**

*FIG. 18*

*FIG. 19*

*FIG. 20*

**FIG. 21**

FIG. 22

FIG. 23

**FIG. 24**

**FIG. 25A**

**FIG. 25B**

FHD

FHR

FNW_J

**FIG. 25C**

Amount of bone
removed medially
and laterally

FNW_J

FHCD

**FIG. 25D**

A.
Patient's biological feature measurement(s) associated with one or more parameters 2600

B.
Variable implant component feature(s) and feature measurement(s) 2610

C.
Variable resection cut features and feature measurements (optional) 2620

Assess A. B. and, optionally, C. to determine optimum feature(s) and feature measurement(s) that achieve or address a parameter of interest 2630

Design patient-adapted implant component having optimal feature(s) and feature measurement(s) 2650

Design patient-adapted resection cuts, e.g., corresponding to designed implant component (optional) 2670

Select implant component, e.g., from library 2640

Select patient-adapted resection cuts, e.g., corresponding to selected implant component 2660

Prepare implantation site and perform implantation 2680

*FIG. 26*

61

```
┌────────────────────────────────────────────────────────────────┐
│ Obtain patient image data and plan surgical procedure using data │──2800
└────────────────────────────────────────────────────────────────┘
                                │
                                ▼
┌────────────────────────────────────────────────────────────────┐
│ (OPTIONAL) - Determine anatomical and/or biomechanical axes      │──2801
└────────────────────────────────────────────────────────────────┘
                                │
                                ▼
┌────────────────────────────────────────────────────────────────┐
│ (OPTIONAL) - Select desired acetubular cup position/orientation  │──2802
└────────────────────────────────────────────────────────────────┘
                                │
                                ▼
┌────────────────────────────────────────────────────────────────┐
│ (OPTIONAL) - Select desired femoral position/orientation         │──2803
└────────────────────────────────────────────────────────────────┘
                                │
                                ▼
┌────────────────────────────────────────────────────────────────┐
│ (OPTIONAL) - Determine reaming depth for acetabulum              │──2804
└────────────────────────────────────────────────────────────────┘
                                │
                                ▼
┌────────────────────────────────────────────────────────────────┐
│ (OPTIONAL) - Calculate offset of acetabular bearing              │──2805
│                 surface and resulting radii                       │
└────────────────────────────────────────────────────────────────┘
```

2806
┌─────────────────────┐
│ (OPTIONAL) - select │
│ femoral head size   │
│ based on offset/radii │
└─────────────────────┘

2809
┌─────────────────────┐
│ (OPTIONAL) - design │
│ femoral head size   │
│ based on offset/radii │
└─────────────────────┘

2812
┌─────────────────────┐
│ (OPTIONAL) - adapt  │
│ femoral head size   │
│ based on offset/radii │
└─────────────────────┘

2807
┌──────────────────────┐
│ (OPTIONAL) - select  │
│ femoral neck length/ │
│ angle or combos thereof │
│ based on offset      │
└──────────────────────┘

2810
┌──────────────────────┐
│ (OPTIONAL) - design  │
│ femoral neck length/ │
│ angle or combos thereof │
│ based on offset      │
└──────────────────────┘

2813
┌──────────────────────┐
│ (OPTIONAL) - adapt   │
│ femoral neck length/ │
│ angle or combos thereof │
│ based on offset      │
└──────────────────────┘

2808
┌──────────────────────────┐
│ (OPTIONAL) - select      │
│ femoral shaft length/width/ │
│ neck angle or combos      │
│ thereof based on offset   │
└──────────────────────────┘

2811
┌──────────────────────────┐
│ (OPTIONAL) - design      │
│ femoral shaft length/width/ │
│ neck angle or combos      │
│ thereof based on offset   │
└──────────────────────────┘

2814
┌──────────────────────────┐
│ (OPTIONAL) - adapt       │
│ femoral shaft length/width/ │
│ neck angle or combos      │
│ thereof based on offset   │
└──────────────────────────┘

*FIG. 28*

Obtain patient image data and plan surgical procedure using data ──2900

(OPTIONAL) - Determine anatomical and/or biomechanical axes ──2901

(OPTIONAL) - Select desired femoral position/orientation ──2902

2903 ──
(OPTIONAL) - select femoral head size based on bone resection, component material thickness (head and cup) and acetabular offset

2906 ──
(OPTIONAL) - design femoral head size based on bone resection, component material thickness (head and cup) and acetabular offset

2909 ──
(OPTIONAL) - adapt femoral head size based on bone resection, component material thickness (head and cup) and acetabular offset

2904 ──
(OPTIONAL) - select femoral neck length/ angle or combo

2907 ──
(OPTIONAL) - design femoral neck length/ angle or combo

2910 ──
(OPTIONAL) - adapt femoral neck length/ angle or combo

2905 ──
(OPTIONAL) - select femoral shaft length/width/ neck angle or combos

2908 ──
(OPTIONAL) - design femoral shaft length/width/ neck angle or combos

2911 ──
(OPTIONAL) - adapt femoral shaft length/width/ neck angle or combos

(OPTIONAL) - Select acetabular cup position/orientation ──2912

(OPTIONAL) - Determine reaming depth for acetabulum based on component thickness/position/orientation/desired leg length ──2913

(OPTIONAL) - Adjust selection, design or adaptation of femoral component based on acetabular bone resection/optimize femoral v. acetabular bone removal for individual patient ──29

*FIG. 29*

**FIG. 30**

Obtain patient image data and plan surgical procedure using data ——3100

(OPTIONAL) - Determine anatomical and/or biomechanical axes ——3101

(OPTIONAL) - Select desired femoral position/orientation ——3102

3103——
(OPTIONAL) - select femoral headsize based on bone removal or preservation, material thickness, mill availability, head diameter or radius, neck diameter or radius

3108——
(OPTIONAL) - design femoral headsize based on bone removal or preservation, material thickness, mill availability, head diameter or radius, neck diameter or radius

3113——
(OPTIONAL) - adapt femoral headsize based on bone removal or preservation, material thickness, mill availability, head diameter or radius, neck diameter or radius

3104——
(OPTIONAL) - Determine necessary thickness of resurf femoral head

3109——
(OPTIONAL) - Determine necessary thickness of resurf femoral head

3114——
(OPTIONAL) - Determine necessary thickness of resurf femoral head

3105——
(OPTIONAL) - select milling tool for milling femoral head to accept resurfacing component

3110——
(OPTIONAL) - design milling tool for milling femoral head to accept resurfacing component

3115——
(OPTIONAL) - adapt milling tool for milling femoral head to accept resurfacing component

3106——
(OPTIONAL) - select desired central peg length

3111——
(OPTIONAL) - design desired central peg length

3116——
(OPTIONAL) - adapt desired central peg length

3107——
(OPTIONAL) - select desired central peg width/thickness

3112——
(OPTIONAL) - design desired central peg width/thickness

3117——
(OPTIONAL) - adapt desired central peg width/thickness

3118——
(OPTIONAL) - Select desired acetubular cup position/orientation

3119——
(OPTIONAL) - Determine depth for reaming

3120——
(OPTIONAL) - Calculate offset of acetabular bearing surface and resulting radii

3121——
(OPTIONAL) - Adjust selection, design or adaptation of femoral component based on acetabular bone resection/optimize femoral v. acetabular bone removal for individual patient

*FIG. 31*

321

**FIG. 32A**

322

**FIG. 32B**

321

323

## FIG. 32C

## FIG. 32D

313    312

300

314

**FIG. 34**

319    317    319    318    300    319    319

318    314    319

318

**FIG. 35a**        **FIG. 35b**

**FIG. 36**

**FIG. 37**

**FIG. 38a**

**FIG. 38b**

**FIG. 38c**

**FIG. 39**

**FIG. 40**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2847458 A1
- US 2011295378 A1
- US 20110112646
- US 20110109017 A
- US 20070004818 A
- US 8066708 B
- US 8083745 B

**Non-patent literature cited in the description**

- State of the Industry Annual Worldwide Progress Report on Additive Manufacturing. Wohlers Report 2009. Wohlers Associates, 2009
- **PHAM ; DIMOV.** Rapid manufacturing. Springer-Verlag, 2001
- Printing the Future. **GRENDA.** The 3D Printing and Rapid Prototyping Source Book. Castle Island Co, 2009
- Virtual Prototyping & Bio Manufacturing. Medical Applications. Springer, 17 December 2007, vol. 13
- Bio-Materials and Prototyping Applications. Medicine. Springer, 10 December 2007, vol. 13
- **LIOU.** Rapid Prototyping and Engineering Applications: A Toolbox for Prototype Development. CRC, 26 September 2007, vol. 13
- Advanced Manufacturing Technology for Medical Applications: Reverse Engineering. Software Conversion and Rapid Prototyping. Wiley, January 2006, vol. 13
- **BRANNER et al.** Coupled Field Simulation in Additive Layer Manufacturing. *3rd International Conference PMI,* 2008, 10